# EUROPEAN PATENT APPLICATION

(11) **EP 4 843 621 A2**
(43) Date of publication of application: **25.11.2026**
(21) Application number: 26194649.5
(22) Date of filing: 09.08.2019
(51) Int. Cl.: A61P 3/06

(54) **TRI-PEPTIDES AND TREATMENT OF METABOLIC, CARDIOVASCULAR AND INFLAMMATORY DISORDERS**

(30) Priority: 10.08.2018 US 201862717546 P
(62) Document number(s) of the earlier application(s) in accordance with Art. 76 EPC: 19847813.3 / 3 833 373
(71) Applicant: Diapin Therapeutics, LLC, Ann Arbor, MI 48109-2590 (US); The Regents of The University of Michigan, Ann Arbor, Michigan 48109-2590 (US)
(72) Inventor: ROM, Oren, Ann Arbor (US); ZHANG, Jifeng, Ann Arbor (US); ZHAO, Ying, Ann Arbor (US); CHEN, Yuqing Eugene, Ann Arbor (US)
(74) Representative: D Young & Co LLP

(57) **Abstract**

The tripeptides DT-109 (Gly-Gly-Leu ) and DT-110 (Gly-Gly-dLeu) treated NAFLD, NASH, and atherosclerosis.

## Description

### CROSS REFERENCE TO RELATED APPLICATIONS

This application claims priority to United States Serial No. 62/717,546, filed August 10, 2018. The entire contents of the aforementioned application are incorporated herein.

### BACKGROUND

"Nonalcoholic fatty liver disease (NAFLD) is increasingly common around the world, especially in western nations. In the United States, it is the most common form of chronic liver disease, affecting an estimated 80 to 100 million people. Nonalcoholic fatty liver disease is an umbrella term for a range of liver conditions affecting people who drink little to no alcohol. As the name implies, the main characteristic of nonalcoholic fatty liver disease is too much fat stored in liver cells. It is normal for the liver to contain some fat. However, if more than 5% - 10% percent of the liver's weight is fat, the condition is called a fatty liver (steatosis).

The more severe form of NAFLD is called non-alcoholic steatohepatitis (NASH). NASH causes the liver to swell and become damaged. NASH tends to develop in people who are overweight or obese, or have diabetes, high cholesterol or high triglycerides or inflammatory conditions. NASH, a potentially serious form of the disease, is marked by hepatocyte ballooning and liver inflammation, which may progress to scarring and irreversible damage.

### SUMMARY

A first aspect of the present disclosure provides methods for treating an inflammation disease, a metabolic disease and/or a cardiovascular disease, the method comprising administering a therapeutically effective amount of a glycine or a glycine-containing tripeptide molecule to a subject having one or more of an inflammation disease, a metabolic disease and/or a cardiovascular disease. In various embodiments, a glycine-containing tripeptide molecule can include one or more of DT-109 (Gly-Gly-Leu) and DT-110 (Gly-Gly-dLeu). In various embodiments, a metabolic disease refers to a group of identified disorders in which errors of metabolism, imbalances in metabolism, or sub-optimal metabolism occur. The metabolic diseases as described herein also include diseases that can be treated through the modulation of metabolism, although the disease itself may or may not be caused by a specific metabolic defect. Such metabolic diseases may involve, for example, glucose and fatty acid oxidation pathways. As used herein, "metabolic disorder" or "metabolic disease" refers to a condition characterized by an alteration or disturbance in metabolic function. "Metabolic" and "metabolism" are terms well known in the art and generally include the whole range of biochemical processes that occur within a living organism. Metabolic and cardiovascular disease includes, but is not limited to, obesity, diabetes, atherosclerosis, metabolic syndrome, dyslipidemia, coronary heart disease, coronary artery disease, arteriosclerosis, atherothrombotic stroke, non-alcoholic fatty liver disease (NAFLD), non-alcoholic steatohepatitis, (NASH), hyperfattyacidemia or metabolic syndrome, or a combination thereof. The dyslipidemia can be hyperlipidemia. The hyperlipidemia can be hypercholesterolemia, hypertriglyceridemia, or both hypercholesterolemia and hypertriglyceridemia. The NAFLD can be hepatic steatosis or steatohepatitis. The diabetes can be type 2 diabetes or type 2 diabetes with dyslipidemia. In various embodiments, methods are presented herein that are applicable to metabolic diseases related to glucose dysregulation and/or accumulation of lipids in the body, circulation or various organs, for example, the liver, and the pathological sequelae resulting therefrom, for example, (non-alcoholic fatty liver disease (NAFLD), non-alcoholic steatohepatitis (NASH), hyperglycemia, prediabetes, diabetes (type I and type II), obesity, insulin resistance, metabolic syndrome and diabetic dyslipidemia.

The metabolic disease, disorder or condition can be characterized by numerous physical symptoms. Any symptom known to one of skill in the art to be associated with the metabolic disease, disorder or condition can be prevented, treated, ameliorated or otherwise modulated with the glycine tripeptide molecules and methods described herein. In certain embodiments, the symptom can be any of, but not limited to, excessive urine production (polyuria), excessive thirst and increased fluid intake (polydipsia), blurred vision, unexplained weight loss and lethargy.

In certain embodiments, the inflammatory diseases, disorders or conditions include, but are not limited to, aortic stenosis, coronary artery disease (CAD), Alzheimer's Disease and thromboembolic diseases, disorder or conditions. Certain thromboembolic diseases, disorders or conditions include, but are not limited to, stroke, thrombosis, myocardial infarction and peripheral vascular disease.

In certain embodiments, the use of glycine or a glycine tripeptide molecule described herein modulate physiological markers or phenotypes of the inflammatory disease, disorder or condition. For example, administration of the compounds to animals can decrease inflammatory cytokine or other inflammatory markers levels in those animals compared to untreated animals. In certain embodiments, the modulation of the physiological markers or phenotypes can be associated with inhibition of liver DAG, glucose-lowering and lowering of plasma LDL levels by the compounds.

In certain embodiments, the physiological markers of the inflammatory disease, disorder or condition can be quantifiable. For example, cytokine levels can be measured and quantified by standard tests known in the art. For such markers, in certain embodiments, the marker can be decreased by at least about 5%, 10%, 15%, 20%, 25%, 30%, 35%, 40%, 45%, 50%, 55%, 60%, 65%, 70%, 75%, 80%, 85%, 90%, 95% or 99%, or a range defined by any two of these values.

Also, provided herein are methods for preventing, treating or ameliorating a symptom associated with the inflammatory disease, disorder or condition in a subject in need thereof. In certain embodiments, provided is a method for reducing the rate of onset of a symptom associated with the inflammatory disease, disorder or condition. In certain embodiments, provided is a method for reducing the severity of a symptom associated with the inflammatory disease, disorder or condition. In such embodiments, the methods comprise administering a therapeutically effective amount of glycine or a glycine tripeptide molecule, or a pharmaceutically acceptable salt thereof, to an individual in need thereof.

Certain embodiments provide compositions and methods for preventing, treating, delaying, slowing the progression and/or ameliorating metabolic, cardiovascular and inflammatory disease, particularly those disorders involving aberrant cholesterol, triglyceride and glucose related diseases, disorders, and conditions in a subject in need thereof. Certain embodiments provide compositions and methods for preventing, treating, delaying, slowing the progression and/or ameliorating triglyceride and total cholesterol related diseases, disorders, and conditions in a subject in need thereof. In certain embodiments, such diseases, disorders, and conditions include inflammatory, cardiovascular and/or metabolic diseases, disorders, and conditions. Certain such cardiovascular diseases, disorders or conditions include, but are not limited to, aortic stenosis, aneurysm (e.g., abdominal aortic aneurysm), angina, arrhythmia, atherosclerosis, cerebrovascular disease, coronary artery disease, coronary heart disease, dyslipidemia, hypercholesterolemia, hyperlipidemia, hypertension, hypertriglyceridemia, myocardial infarction, peripheral vascular disease (e.g., peripheral artery disease, peripheral artery occlusive disease), retinal vascular occlusion, or stroke. Certain such metabolic diseases, disorders or conditions include, but are not limited to, hyperglycemia, prediabetes, diabetes (type I and type II), obesity, insulin resistance, metabolic syndrome and diabetic dyslipidemia. Certain such inflammatory diseases, disorders or conditions include, but are not limited to, aortic stenosis, coronary artery disease (CAD), Alzheimer's Disease and thromboembolic diseases, disorder or conditions. Certain thromboembolic diseases, disorders or conditions include, but are not limited to, stroke, thrombosis (e.g., venous thromboembolism), myocardial infarction and peripheral vascular disease. Certain embodiments provide compositions and methods for preventing, treating, delaying, slowing the progression and/or ameliorating diet induced hyperlipidemia and steatohepatitis with suppressed mitochondrial/peroxisomal fatty acid β oxidation (FAO), in addition to preventing, treating, delaying, slowing the progression and/or ameliorating glucose sensitivity and systemic inflammation and fibrosis, in particular when associated with liver disease.

One embodiment of the first aspect of the present disclosure provides methods for treating non-alcoholic fatty liver disease (NAFLD) comprising administration of glycine or DT-109 (Gly-Gly-Leu). Another embodiment of the first aspect of the present disclosure provides methods for treating non-alcoholic steatohepatitis (NASH) comprising administration of glycine or DT-109 (Gly-Gly-Leu). Another embodiment of the first aspect of the present disclosure provides methods for treating non-alcoholic fatty liver disease (NAFLD) comprising administration of DT-110 (Gly-Gly-dLeu). One embodiment of the first aspect of the present disclosure provides methods for treating non-alcoholic steatohepatitis (NASH) comprising administration of glycine or DT-110 (Gly-Gly-dLeu). These tripeptides optionally can be administered in combination with a secondary therapeutic agent as described herein.

A second aspect of the present disclosure provides methods for reducing fibrosis in a patient comprising administering a selected tripeptide or a pharmaceutically acceptable salt thereof. One embodiment of the second aspect of the present disclosure provides methods for reducing fibrosis in a patient comprising administration of glycine or DT-109 (Gly-Gly-Leu). Another embodiment of the second aspect of the present disclosure provides methods for reducing fibrosis in a patient comprising administration glycine or DT-110 (Gly-Gly-dLeu). These tripeptides optionally can be administered in combination with a statin.

A third aspect of the present disclosure provides methods of treating a hepatic steatosis, comprising administering to a subject in need thereof, one or more tripeptide molecule or pharmaceutically acceptable salt thereof. In one embodiment of the third aspect the method decreased the triglyceride level in the hepatic lipids or decreased the total cholesterol level in the hepatic lipids.

A forth aspect of the present disclosure provides a kit for treating a subject with NAFLD or with NASH comprising a selected tripeptide, optionally a statin, and instructions for use. In one embodiment of the kit, the kit comprises an effective amount of glycine or DT-109 (Gly-Gly-Leu), optionally a statin, and instructions for use. In another embodiment the kit comprises glycine or (DT-110 (Gly-Gly-dLeu), optionally a statin, and instructions for use. In yet another kit, the kit comprises glycine or DT-109 (Gly-Gly-Leu) and/or (DT-110 (Gly-Gly-dLeu), optionally a statin, and instructions for use.

### BRIEF DESCRIPTION OF THE DRAWING(S)

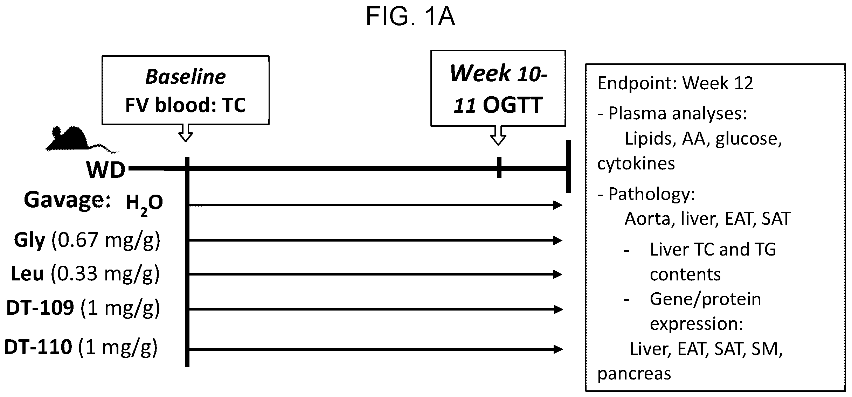

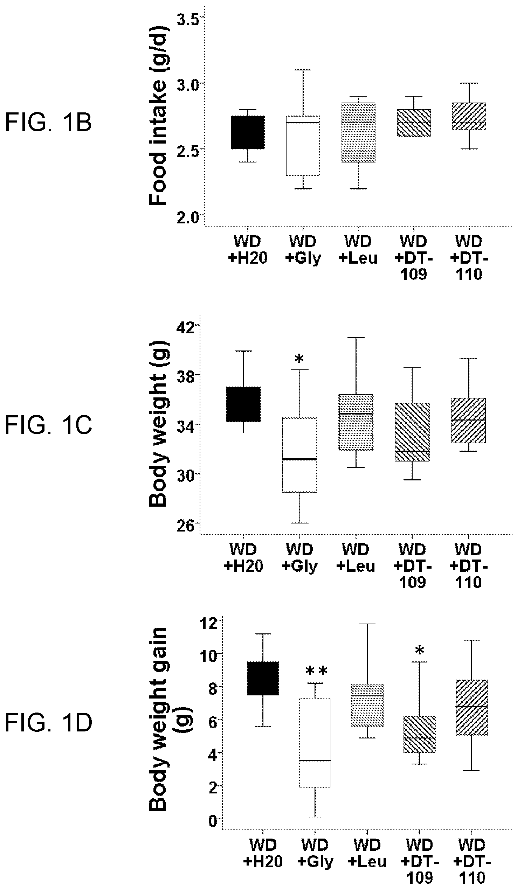

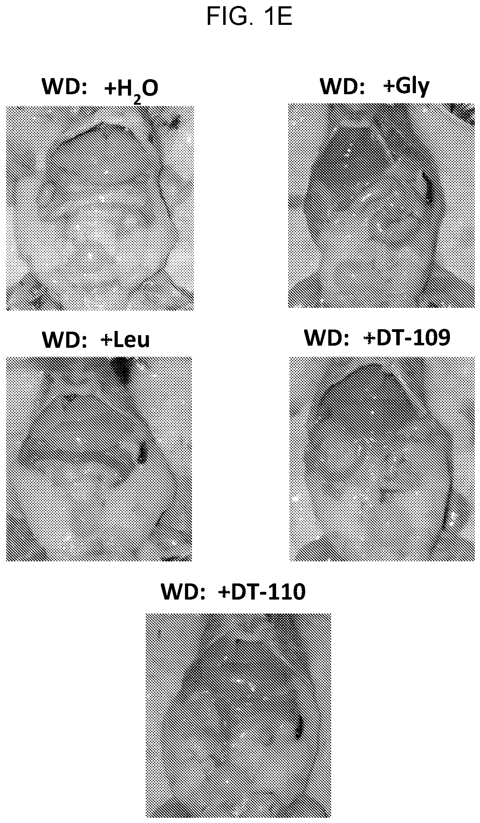

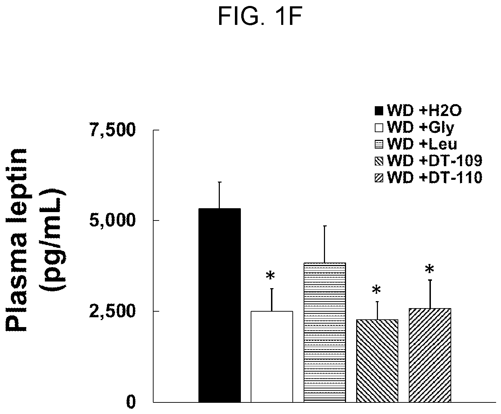

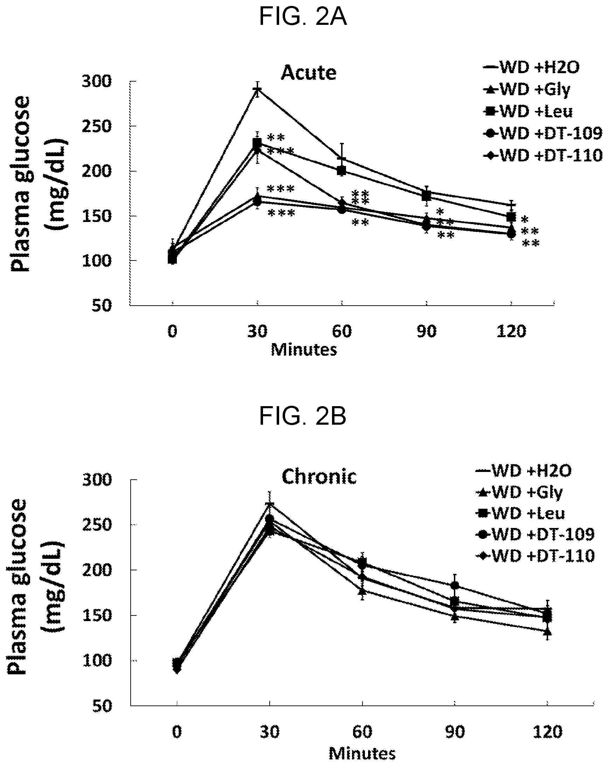

**FIGs. 1A-1F****:** Experimental design (FIG. 1A), Average food intake throughout the study (FIG. 2B), Endpoint body weight (FIG. 1C), Total gain in body weight from baseline to endpoint (FIG. 1D) (n=8-10 for B-D), Gross morphology of the peritoneal cavity at endpoint (FIG. 1E), Plasma glycine-containing tripeptide molecule levels (FIG. 1F) (n=6-8). * p<0.05; ** p<0.01 vs. WD + H₂O.

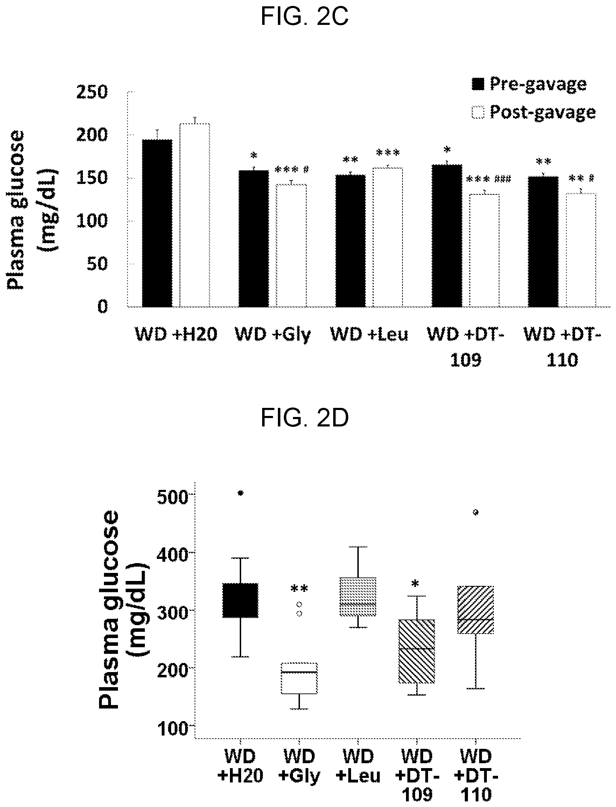

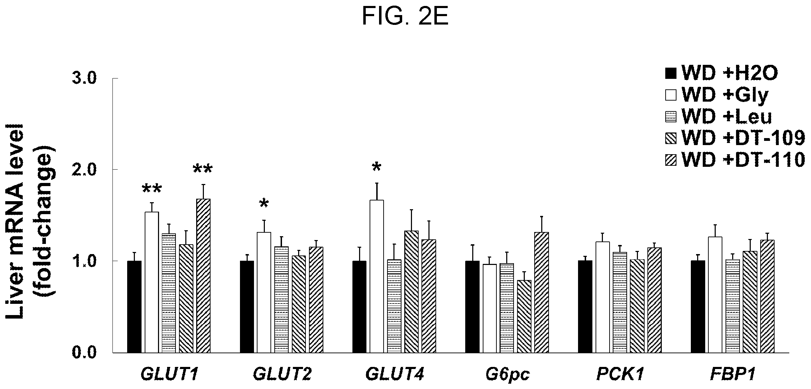

**FIGs. 2A-2E****:** Acute OGTT (mice were loaded with glucose with treatments or water as control) (FIG. 2A), Chronic OGTT (mice were loaded with glucose alone) (FIG. 2B), Non-fasting blood glucose levels measured before oral gavage with water or with the treatments (pre-gavage) and 30 min afterwards (post-gavage) (FIG. 2C), endpoint blood glucose levels (following a 6 hour fast) (FIG. 2D). qPCR analysis of hepatic gene expression regulating glucose uptake and gluconeogenesis (FIG. 2E). (n=8-10) * p<0.05; ** p<0.01, *** p<0.001 vs. WD + H₂O; # p<0.05, ### p<0.001 vs. pre-gavage.

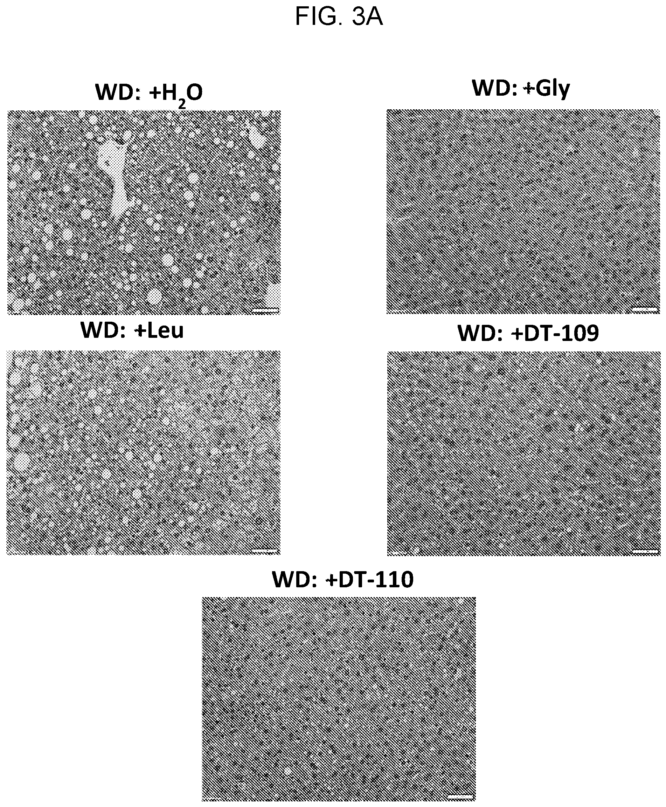

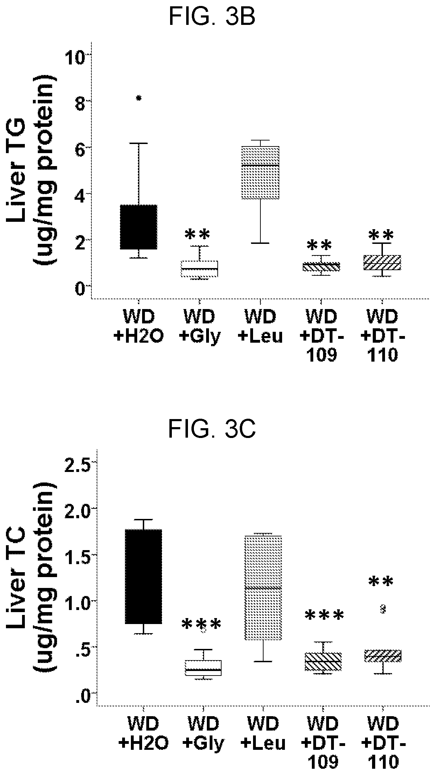

**FIGs. 3A-3C****:** Representative H&E slides of liver tissues (FIG. 3A), Microvesicular and macrovesicular steatosis are marked with red and yellow arrows, respectively. Hepatic lipids were extracted, followed by quantification of (FIG. 3B) TG, and (FIG. 3C) TC contents, ** p<0.01, *** p<0.001 vs. WD + H₂O.

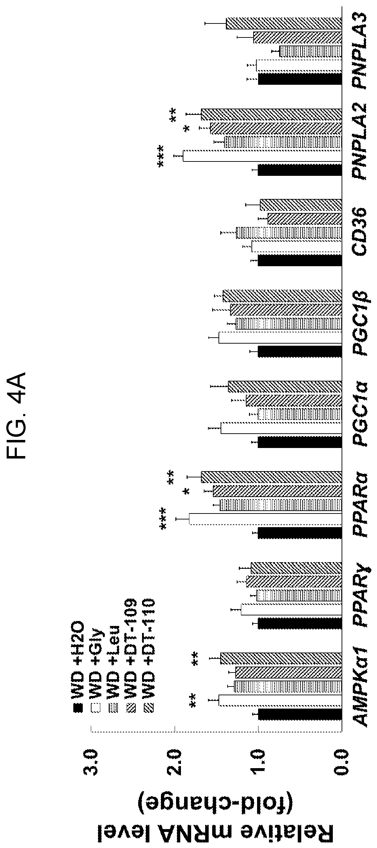

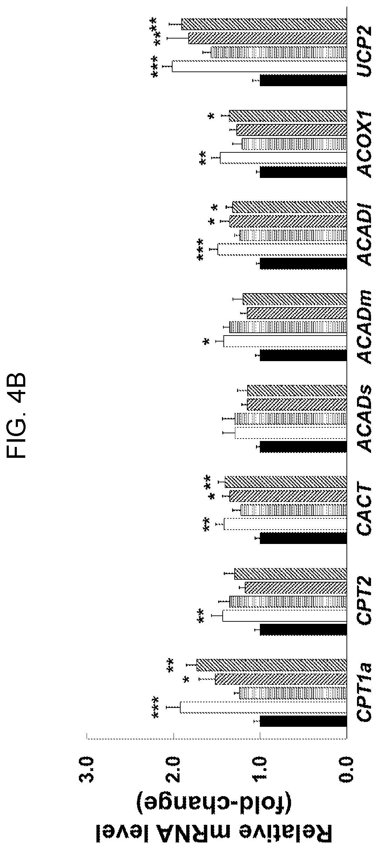

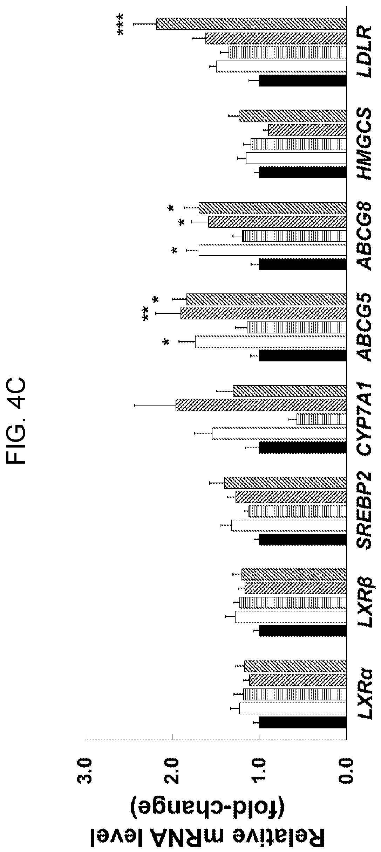

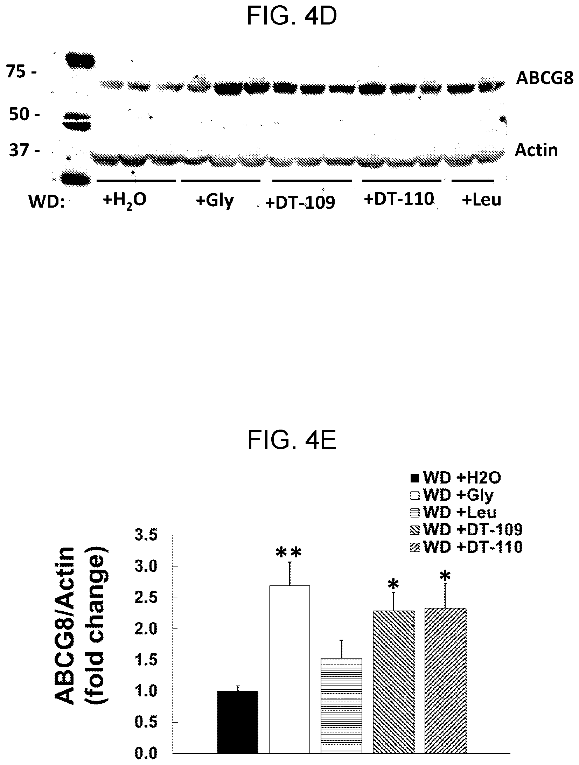

**FIGs. 4A-4E****:** qPCR analysis of hepatic gene expression regulating pathways of lipid oxidation (FIG. 4A, and FIG. 4B), qPCR analysis of hepatic gene expression regulating cholesterol homeostasis (FIG. 4C) (n=7-10). Western blot analysis and quantification of ABCG8 abundance normalized to β-Actin (FIG. 4D and FIG. 4E) (n=4-6). * p<0.05; ** p<0.01, *** p<0.001 vs. WD + H₂O.

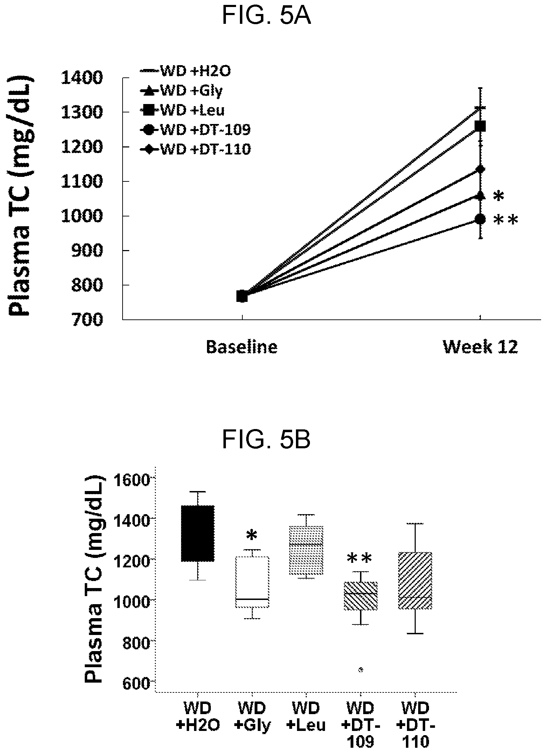

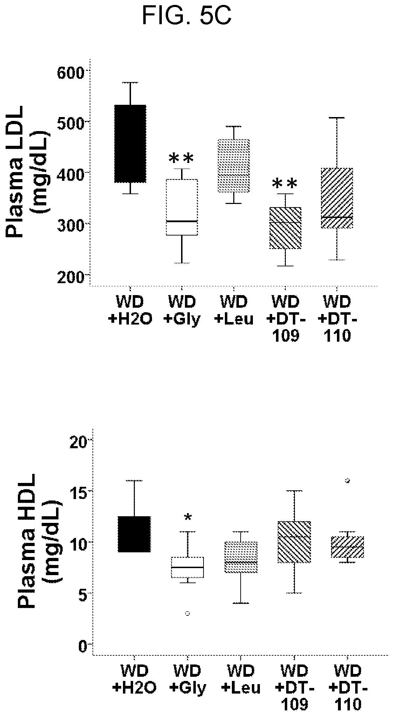

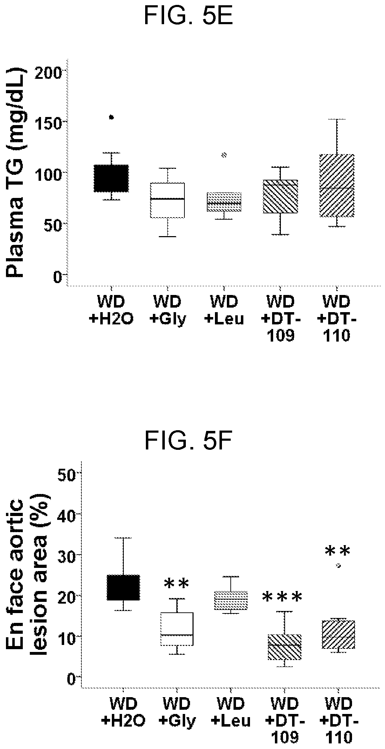

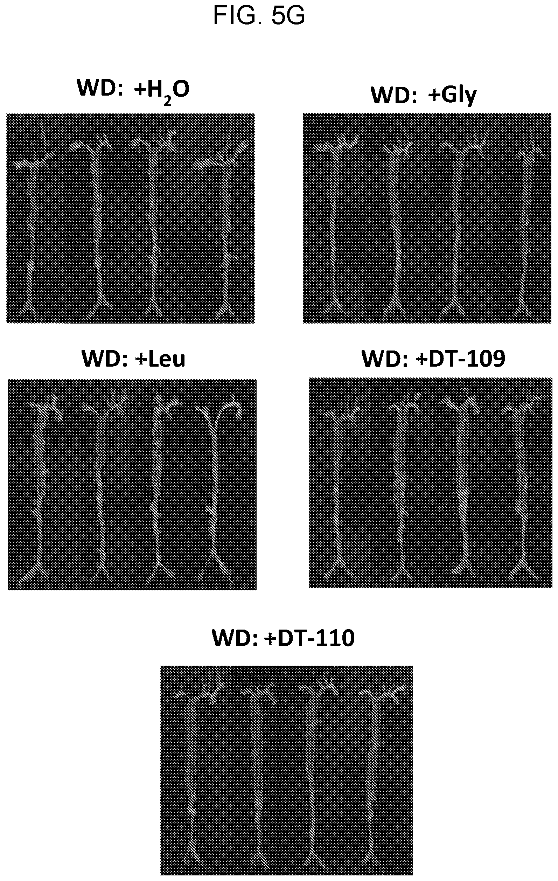

**FIGs. 5A-5G****:** Plasma TC levels at baseline (1 week of WD feeding without treatments) and at endpoint (after 12 weeks of WD feeding with treatments or water control) (FIG. 5A). Endpoint plasma levels of (FIG. 5B) TC, (FIG. 5C) LDL, (FIG. 5D) HDL, and (FIG. 5E) TG. Blood collection was performed after a 6 hour fast. (FIG. 5F) Analysis of atherosclerotic plaque visualized by Oil Red O staining, and (FIG. 5G) representative images of stained aortas. (n=6-10). * p<0.05; ** p<0.01, *** p<0.001 vs. WD + H₂O.

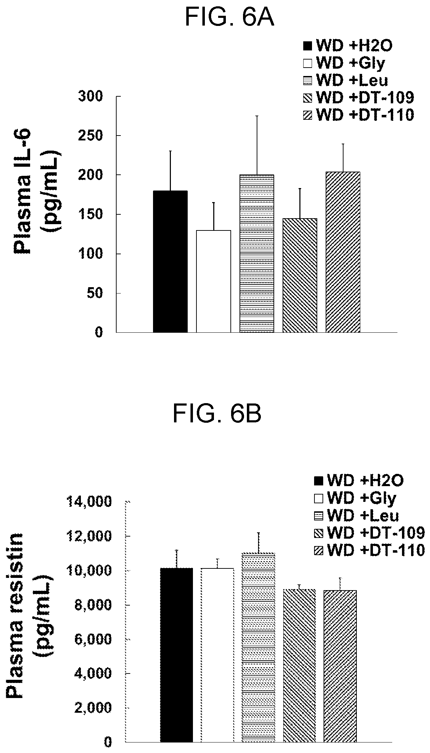

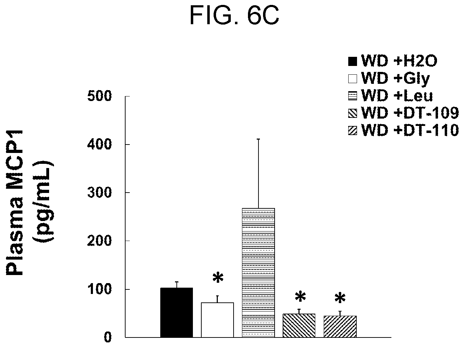

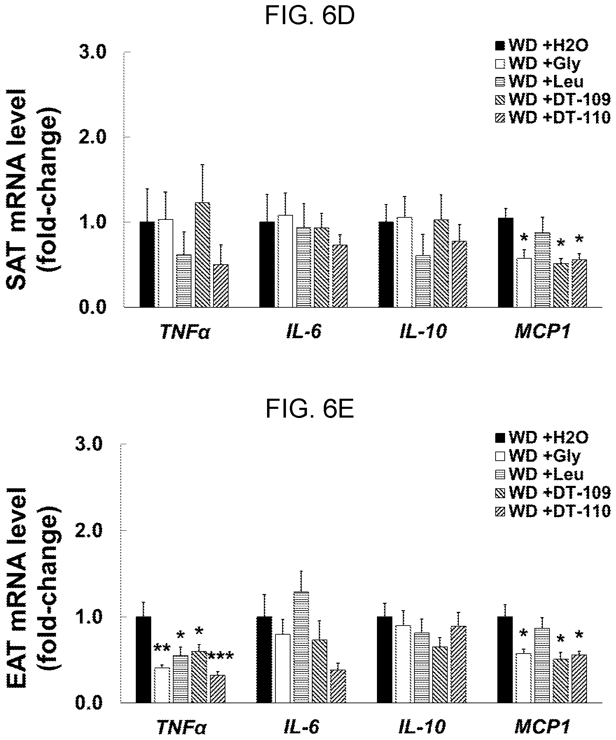

**FIGs. 6A-6E****:** Endpoint plasma levels of IL-6 (FIG. 6A), resistin (FIG. 6B), and MCP1 (FIG. 6C). qPCR analysis of inflammatory cytokines in (FIG. 6D) epididymal adipose tissue (EAT), and in (FIG. 6E) subcutaneous adipose tissue (SAT). (n=6-10). * p<0.05; ** p<0.01, *** p<0.001 vs. WD + H₂O.

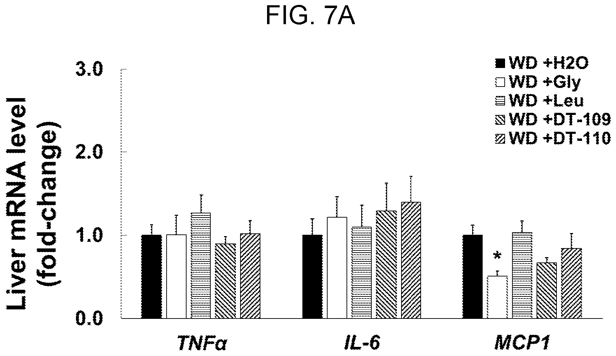

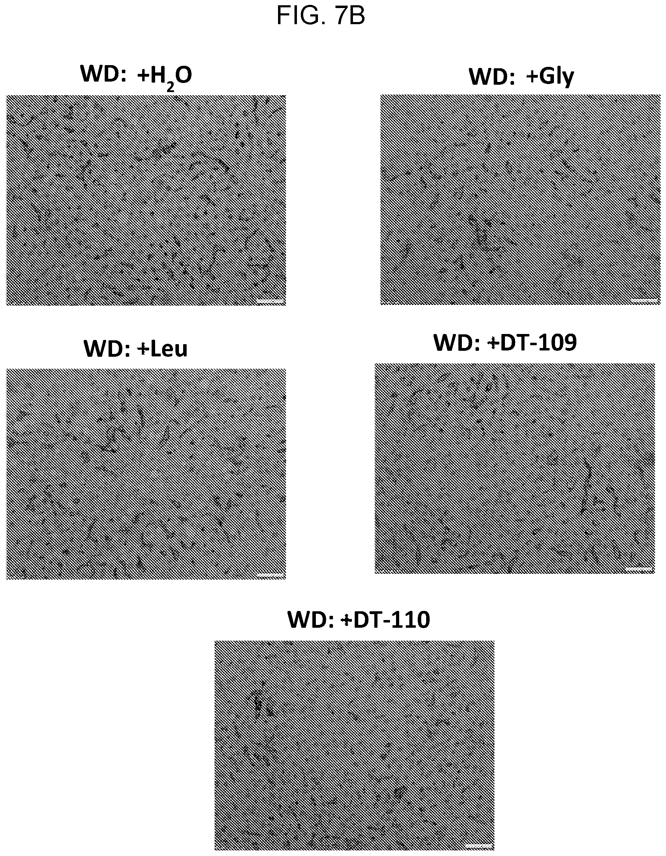

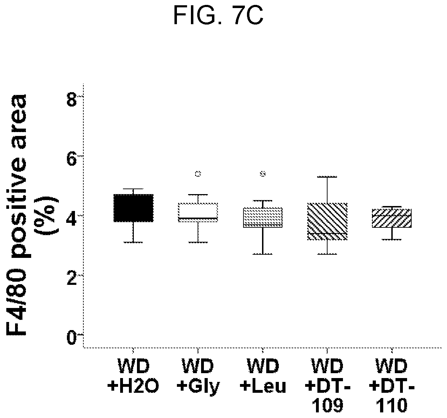

**FIGs. 7A-7C****:** qPCR analysis of inflammatory cytokines in the liver (FIG. 7A). Representative F4/80 immunohistochemistry in the liver (FIG. 7B), and quantification (FIG. 7C). (n=7-10). * p<0.05 vs. WD +H₂O.

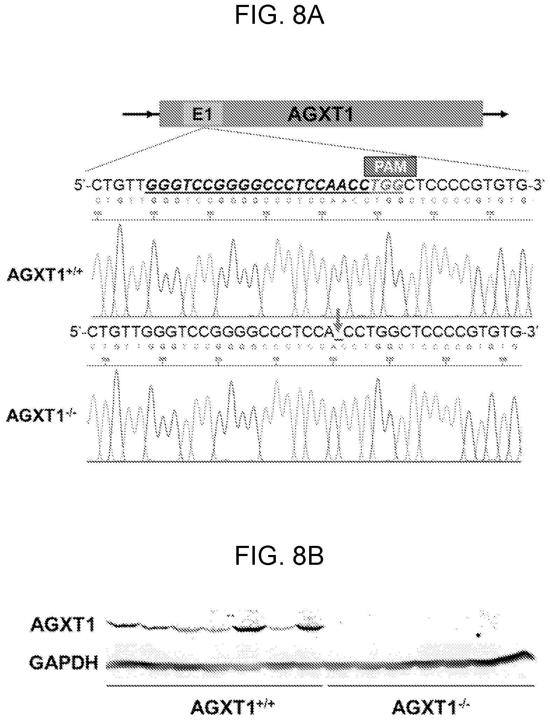

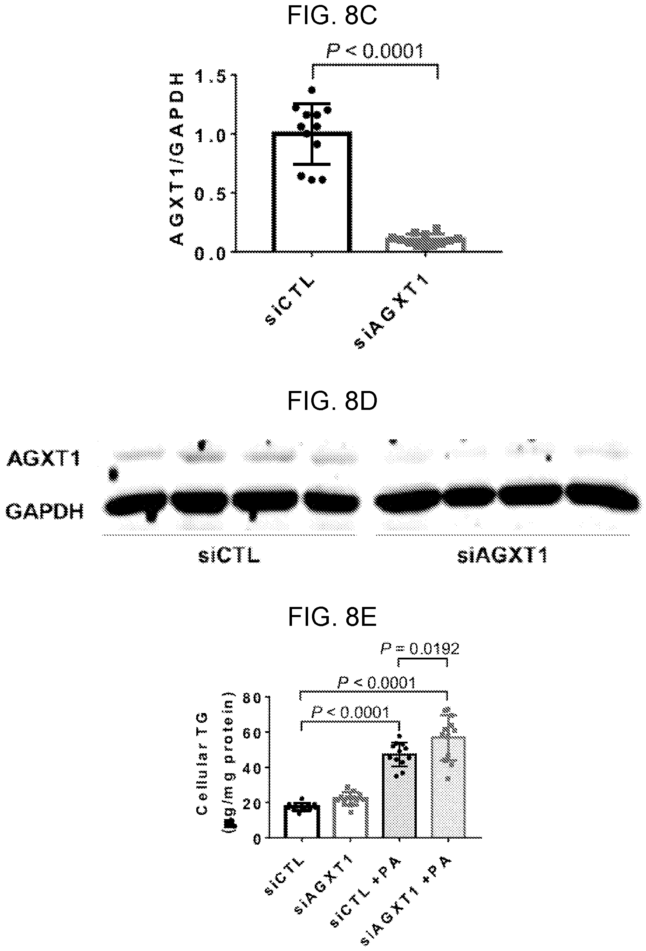

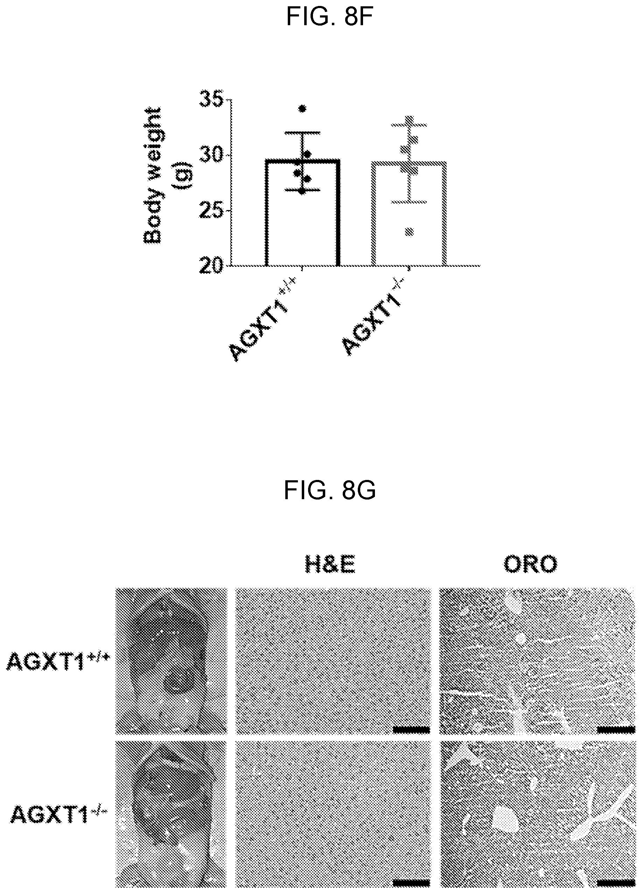

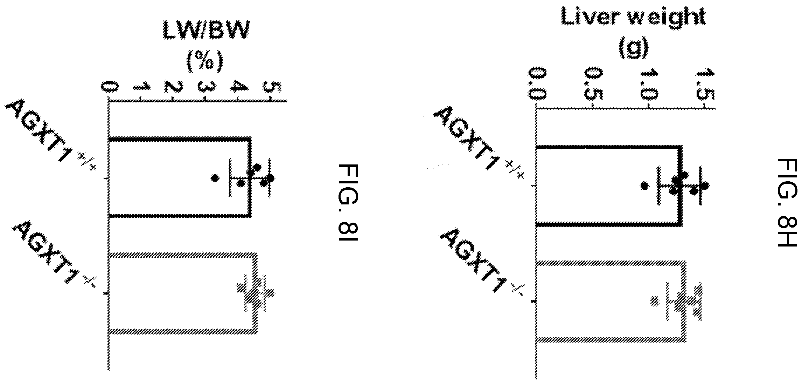

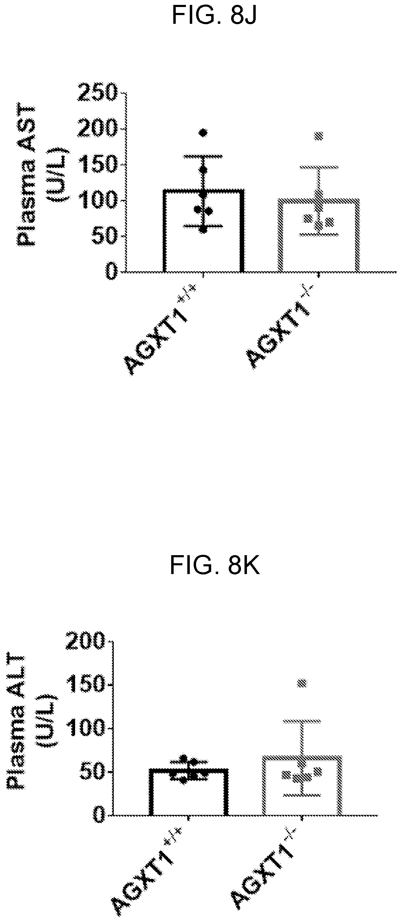

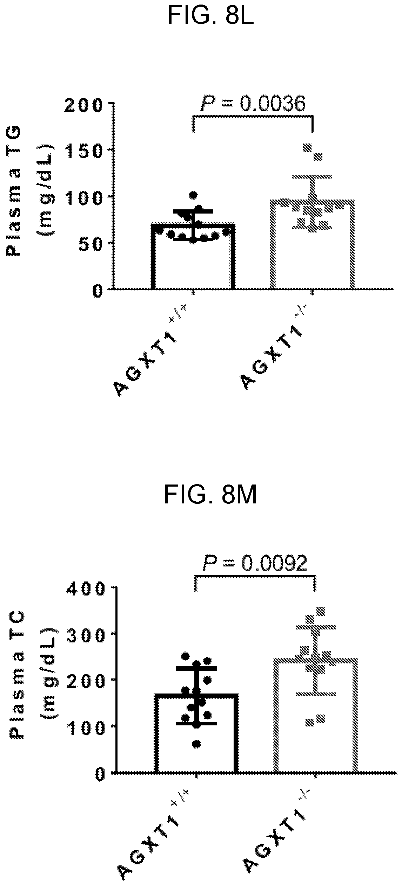

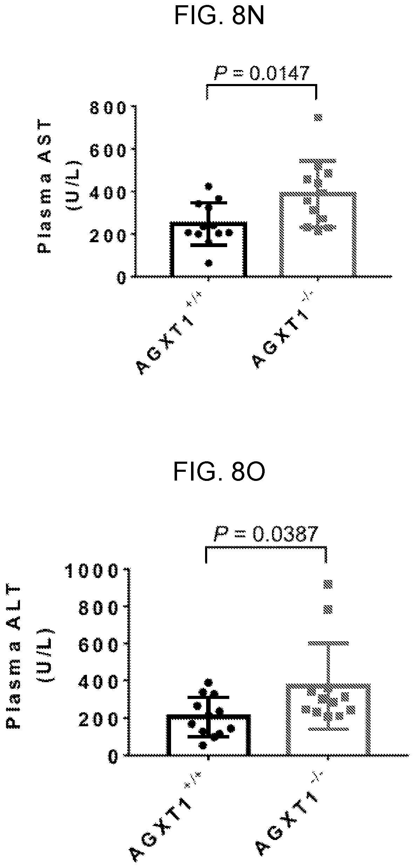

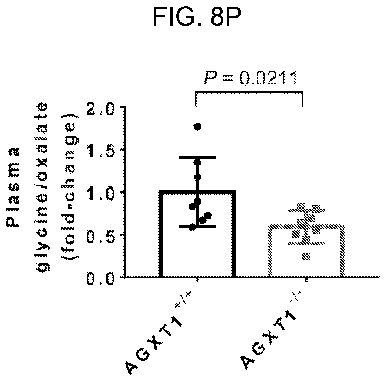

**FIGs. 8****.** Plasma alterations in AGXT1^{-/-} mice fed NASH-diet. HepG2 cells were transfected with siCTL or siAGXT1: (FIG. 8C) AGXT1 mRNA relative to GAPDH (n=12), (FIG. 8D) AGXT1 protein with GAPDH as loading control (n=4), and (FIG. 8E) Cellular TG, with or without PA loading (200µM, n=12). (FIG. 8A) The guide-RNA target site on exon 1 of the AGXT1 gene is underlined and A deletion three bases from the PAM was confirmed by Sanger sequencing. (FIG. 8B) Absence of AGXT1 confirmed by Western blot (n=7). AGXT1^{+/+} and AGXT1^{-/-} mice were fed NASH-diet for 12 weeks (n=12): Plasma (FIG. 8L) TG, (FIG. 8M) TC, (FIG. 8N) AST, (FIG. 8O) ALT, and (FIG. 8P) glycine/oxalate ratio. Data are meantSD showing all points and P values. PA, palmitic acid.
**FIGs. 8F-8K****.** AGXT1^{+/+} and AGXT1 ^{-/-} are comparable under CD feeding. AGXT1^{+/+} and AGXT1^{-/-} mice were fed standard CD for 12 weeks (n=6): (FIG. 8F) Body weight, (FIG. 8G) Gross appearance of the peritoneal cavities and histology using H&E and ORO staining (Scale bar: 50 µm for H&E, 100 µm for ORO), (FIG. 8H) Liver weight, (FIG. 8I) Ratio of liver weight (LW) to body weight (BW), (FIG. 8J) Plasma AST, and (FIG. 8K) Plasma ALT. Data are mean±SD showing all points.

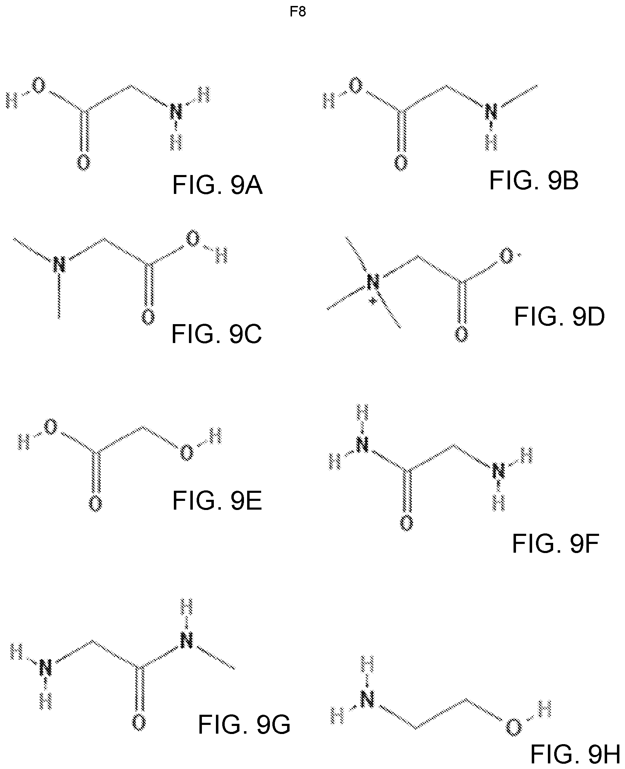

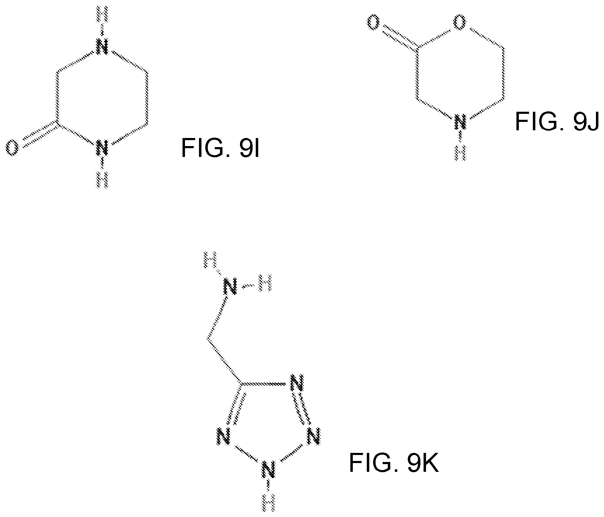

**FIGs. 9A-K**. Glycine-based compounds. Compounds structurally similar to glycine were chosen to evaluate structural, conformational, electronic and isosteric modifications to the glycine scaffold. (FIG. 9A) Glycine, (FIG. 9B) N-methylglycine, (FIG. 9C) N,N-dimethylglycine, (FIG. 9D) N,N,N-trimethylglycine, (FIG. 9E) Glycolic acid, (FIG. 9F) Glycinamide, (FIG. 9G) 2-amino-N-methylacetamide, (FIG. 9H) Ethanolamine, (FIG. 9I) 2-oxopiperazine, (FIG. 9J) Morpholin-2-one, and (FIG. 9K) (1H-tetrazol-5-yl) methanamine.

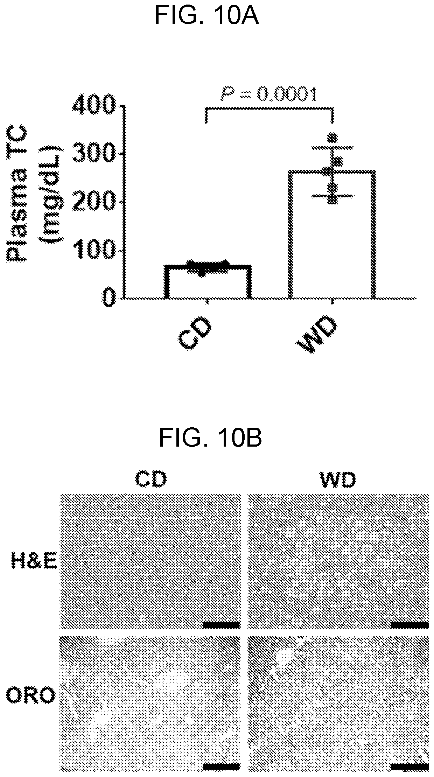

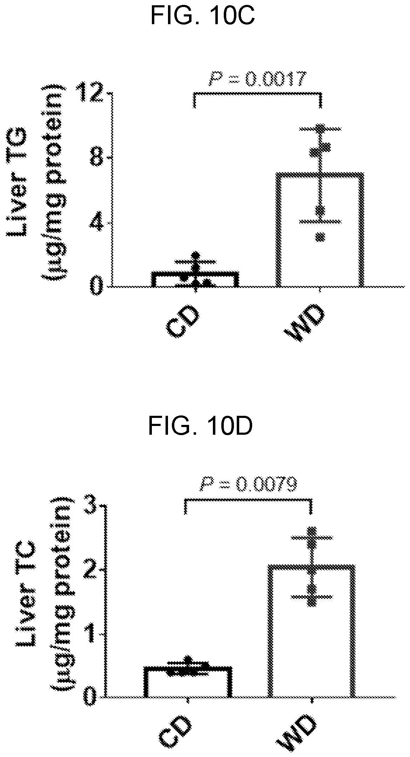

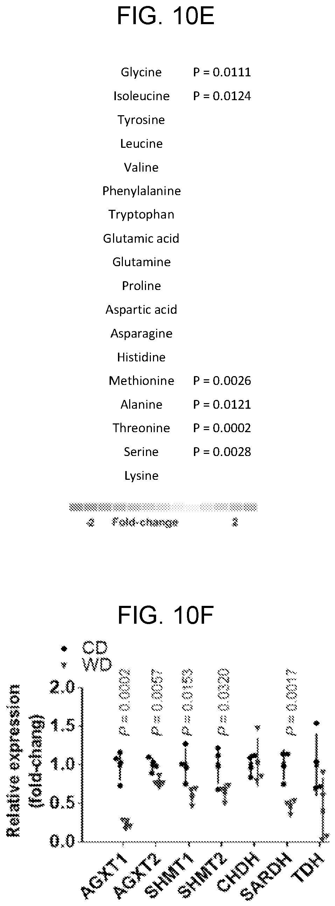

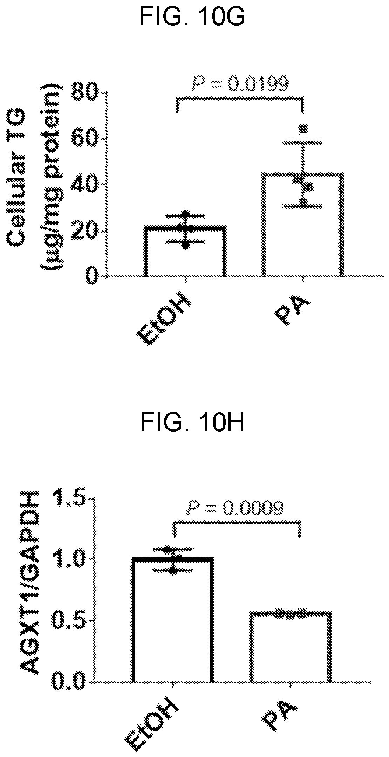

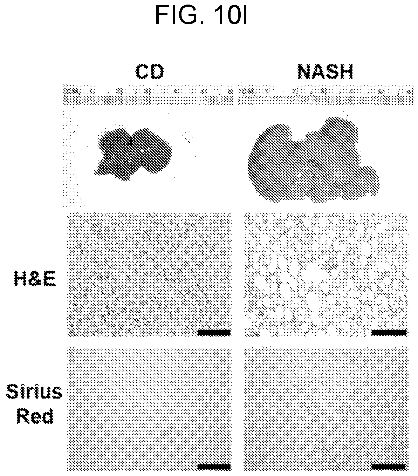

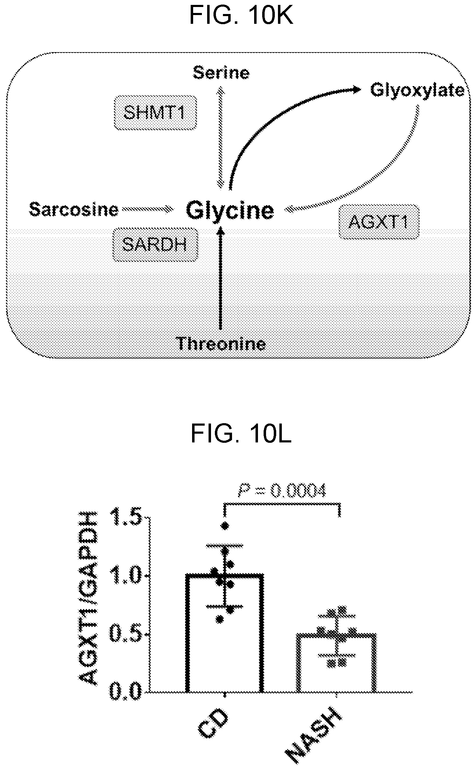

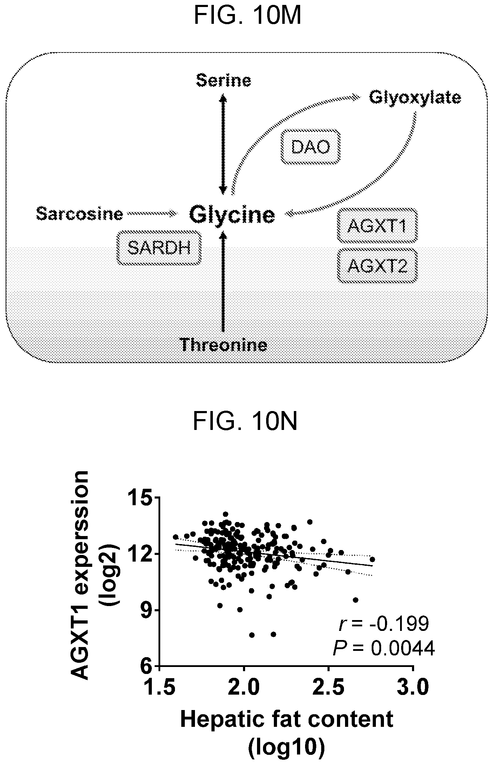

**FIGs. 10A-I****,****K-N****.** Impaired glycine biosynthesis in NAFLD. C57BL/6J mice were fed CD or WD for 12 weeks (n=4-5): (FIG. 10A) Plasma TC, (FIG. 10B) Liver histology (Scale bar: H&E 50µm, ORO 100µm), (FIG. 10C) Liver TG, (FIG. 10D) Liver TC, (FIG. 10E) Plasma AA relative to CD, (FIG. 10F) Hepatic expression of glycine biosynthetic genes relative to GAPDH. (FIG. 10G) Cellular TG, and (FIG. 10H) AGXT1 expression in HepG2 cells loaded with 200 µM PA or ethanol for 24 h (n=3-4). C57BL/6J mice were fed NASH-diet or CD for 24 weeks (n=10): (FIG. 10I) Liver morphology, H&E and Sirius Red histology (Scale bar: 50 µm). (FIG. 10K) Significant downregulation (green) of glycine biosynthetic genes/pathways by RNA-sequencing of livers from CD or NASH mice (n=3, log2FC, log2fold-change). (FIG. 10L) AGXT1 expression relative to GAPDH in mice with diet-induced NASH (n=8). (FIG. 10M) Significant downregulation (green) or upregulation (red) in glycine metabolism genes by meta-analysis of liver microarray data from healthy vs. NASH patients. (FIG. 10N) Correlation between AGXT1 expression and total hepatic fat in livers from transplantation donors (n=206). Data are mean±SD showing all points and *P* values.

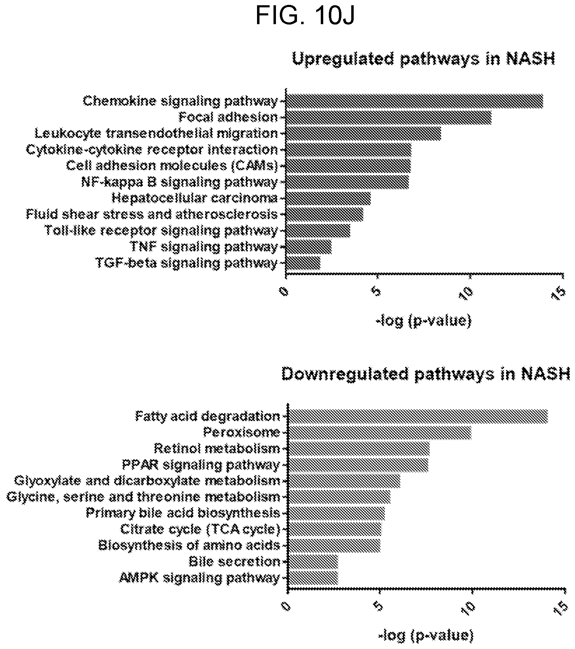

**FIG. 10J****.** Pathway analysis of livers from mice with NASH. Pathway analysis following RNA-sequencing of livers from mice fed CD or NASH-diet for 24 weeks (n=3). Pathways enriched in the upregulated differentially expressed genes (DEG) are plotted in red, while pathways enriched in the down-regulated DEG are plotted in green.

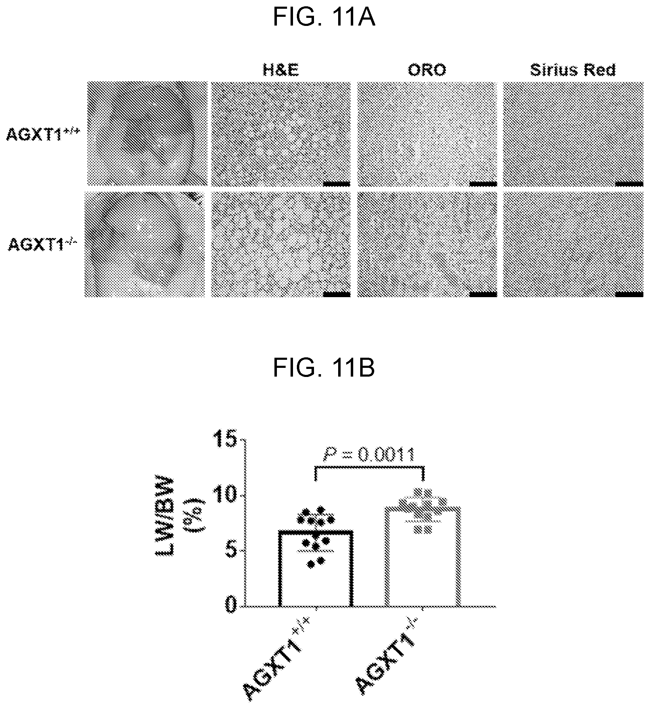

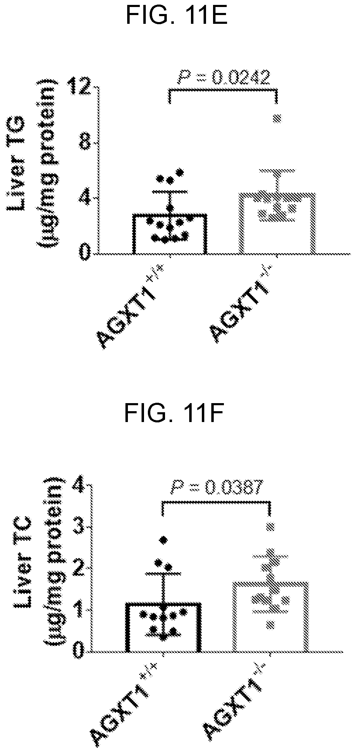

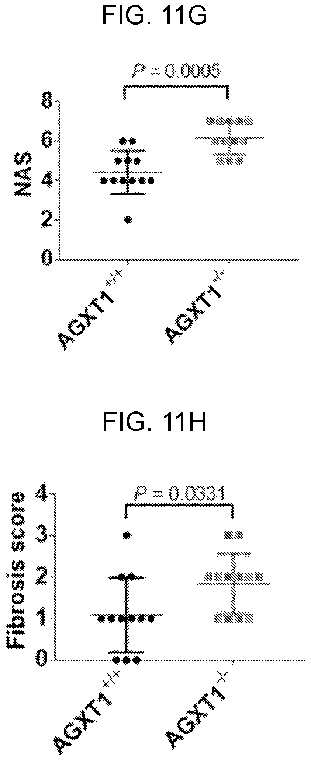

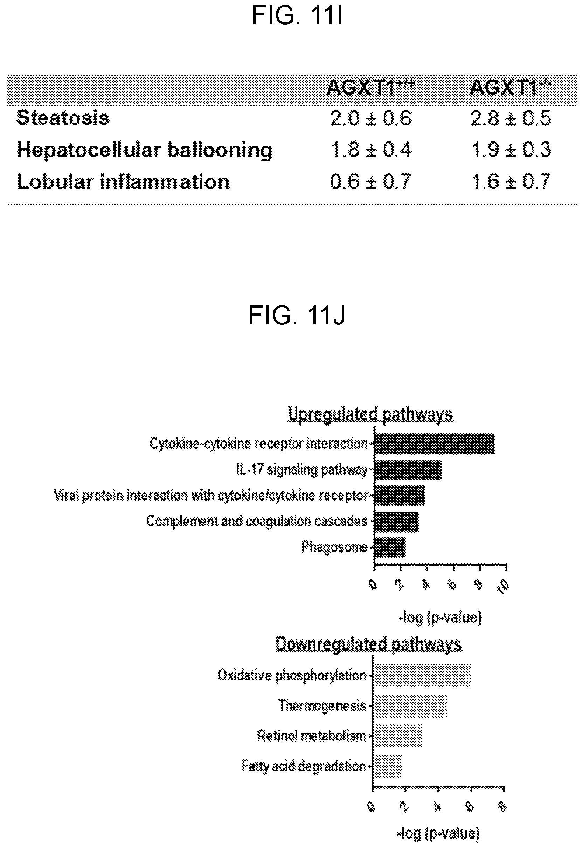

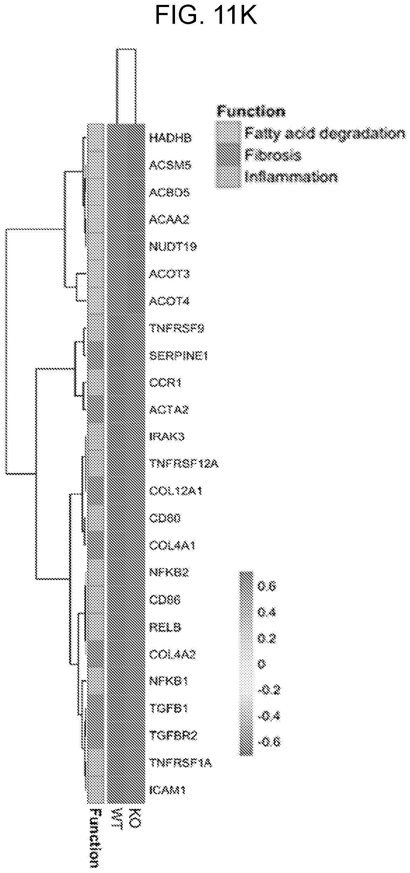

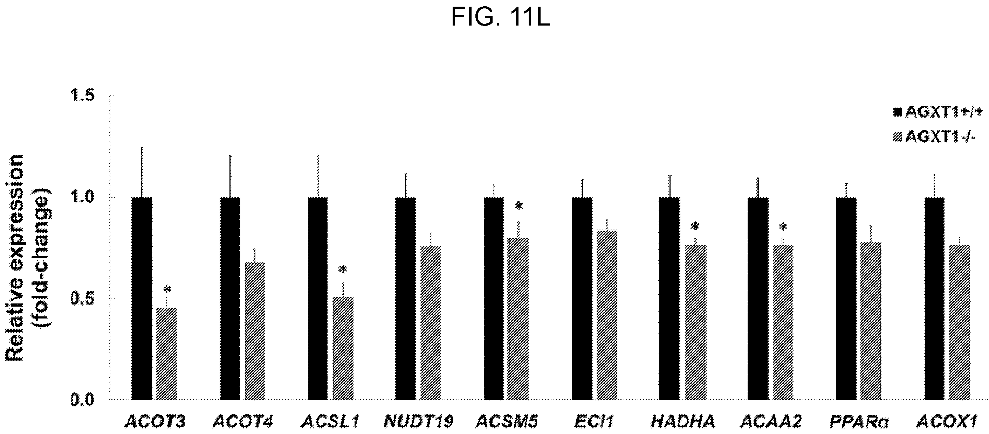

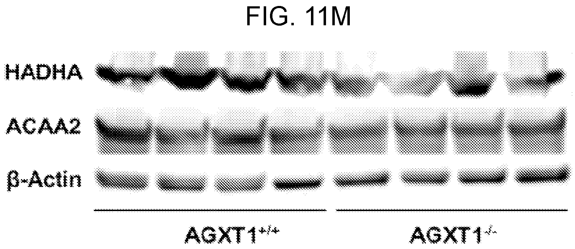

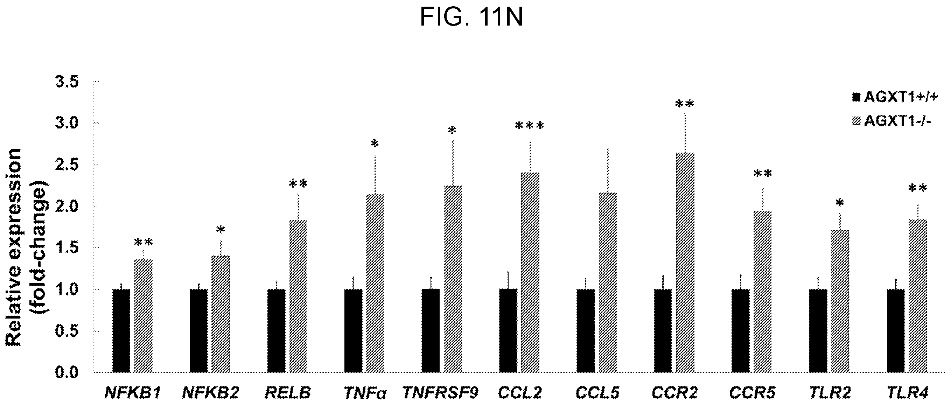

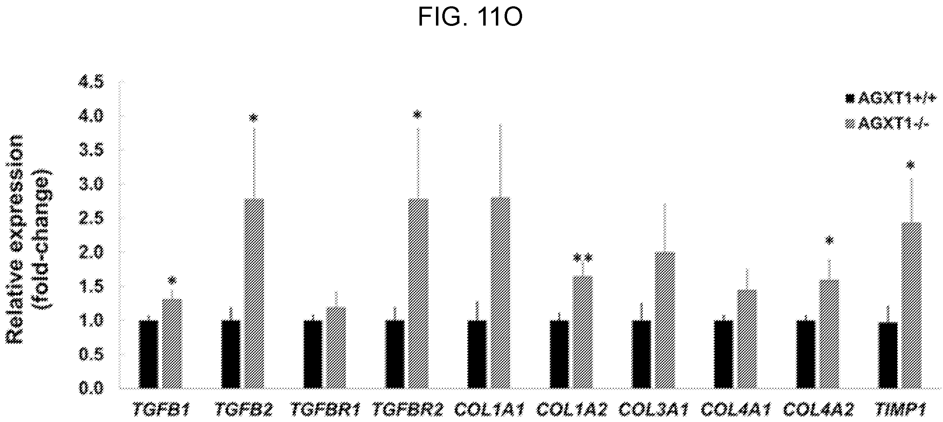

**FIGs. 11A-L** . Accelerated diet-induced NASH in AGXT1^{-/-} mice. AGXT1^{+/+} and AGXT1^{-/-}mice were fed NASH-diet for 12 weeks (n=12): (FIG. 11A) Gross appearance of the peritoneal cavities, and liver histology (Scale bar: H&E and Sirius Red 50µm, ORO 100µm), (FIG. 11B) Liver weight/body weight (LW/BW) ratio, (FIG. 11E) Liver TG, (FIG. 11F) Liver TC, (FIG. 11G) NAS, and (FIG. 11H) fibrosis score. Data are mean±SD showing all points and P values. (FIG. 11J) Pathway analysis following RNA-sequencing of livers from AGXT1^{+/+} and AGXT1^{-/-} mice (n=4). Pathways enriched in the up- or down-regulated DEG are plotted in red or green, respectively. (FIG. 11K) Heatmap of 25 NASH-related DEG. (FIG. 11L) FAO-related DEG confirmed by qPCR (n=10), and (FIG. 11M) Western bolt (n=4). (FIG. 11N) Inflammation- and (FIG. 11O) Fibrosis-related DEG confirmed by qPCR (n=10). Data are mean±SE. *P<0.05, **P<0.01, ***P<0.001 vs. AGXT1^{+/+}.

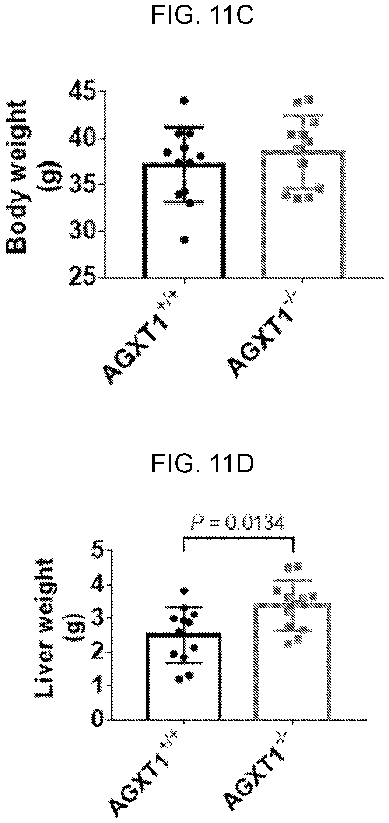

**FIGs. 11C****, D,** I. NAFLD-related parameters in AGXT1^{-/-} mice fed NASH-diet. AGXT1^{+/+} and AGXT1^{-/-} mice were fed NASH-diet for 12 weeks (n=12): (FIG. 11C) Body weight, (FIG. 11D) Liver weight, and (FIG. 11I) H&E-based scoring of steatosis, hepatocellular ballooning and lobular inflammation. Data are mean±SD.

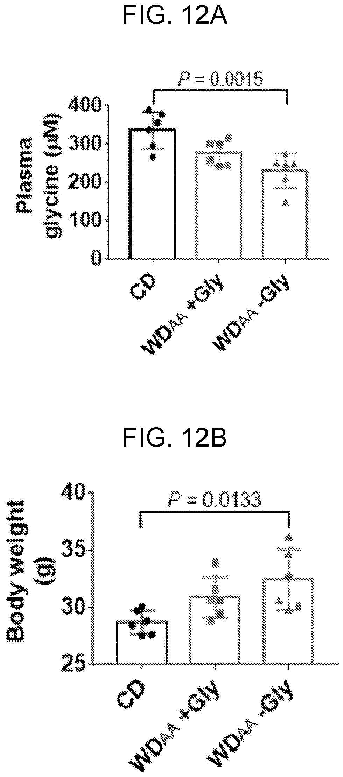

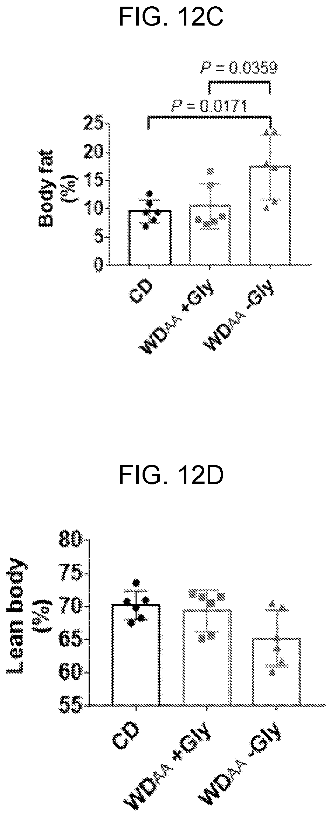

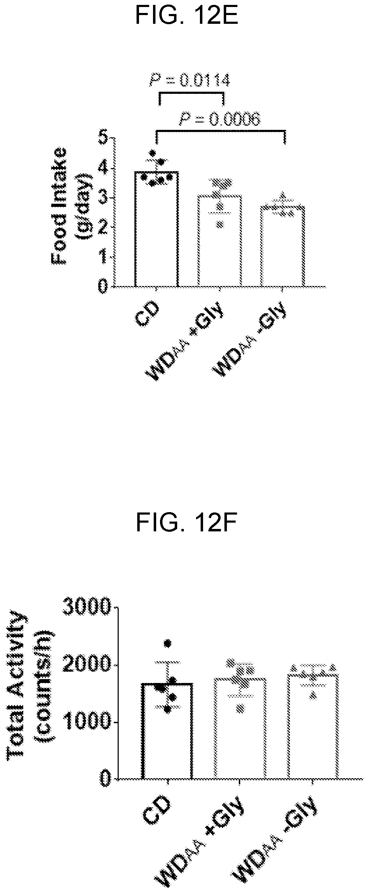

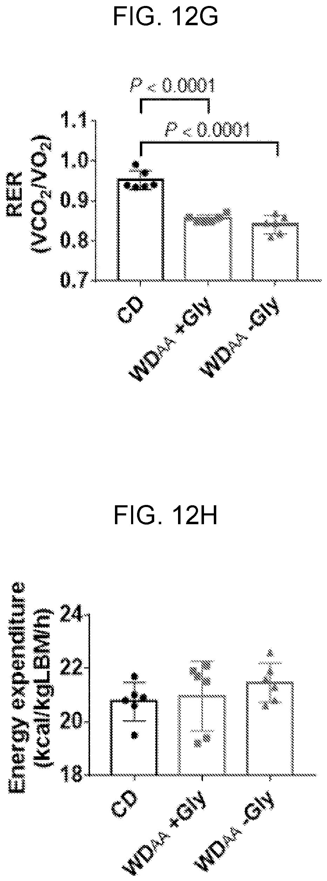

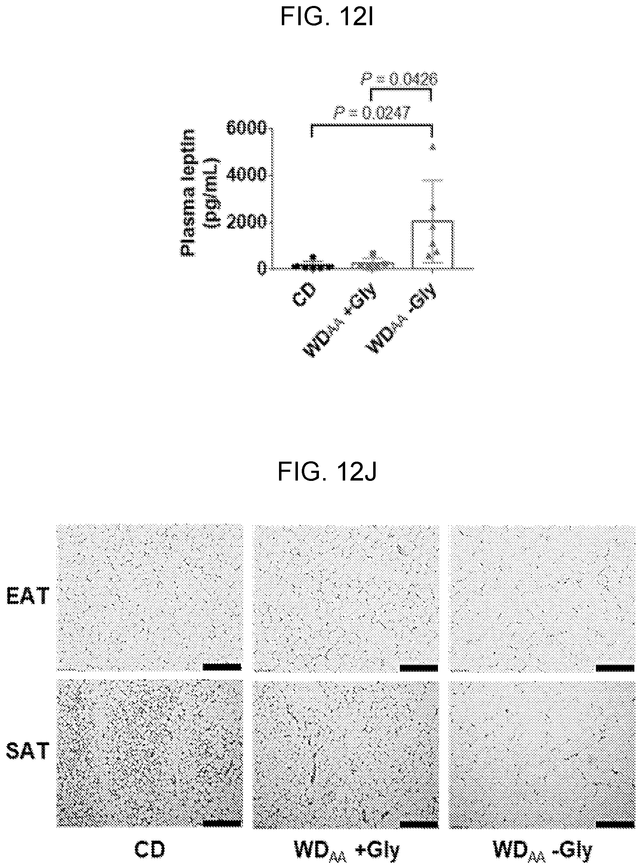

**FIGs. 12A-J** . Glycine deficiency exacerbates WD-induced obesity. ApoE^{-/-} mice were fed CD, WD_{AA} +Gly or WD_{AA} -Gly for 10 weeks (n=6): (FIG. 12A) Plasma glycine. NMR-based body composition analysis: (FIG. 12B) Body weight, (FIG. 12C) Body fat (%), and (FIG. 12D) Lean body mass (%). CLAMS analysis: (FIG. 12E) Food intake, (FIG. 12F) Total activity, (FIG. 12G) Respiratory exchange ratio (RER), and (FIG. 12H) Energy expenditure. (FIG. 12I) Plasma Glycine tripeptide molecule, and (FIG. 12J) H&E histology of epididymal and subcutaneous adipose tissues (EAT and SAT, Scale bar: 100 µm). Data are mean±SD showing all points and P values.

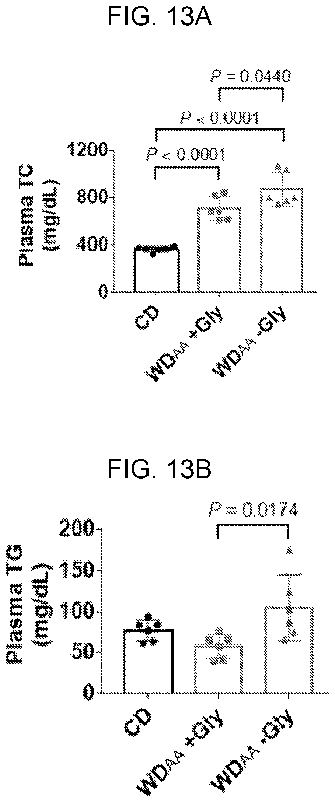

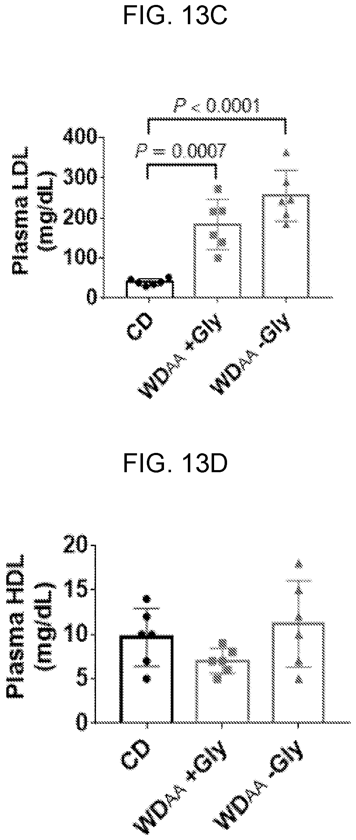

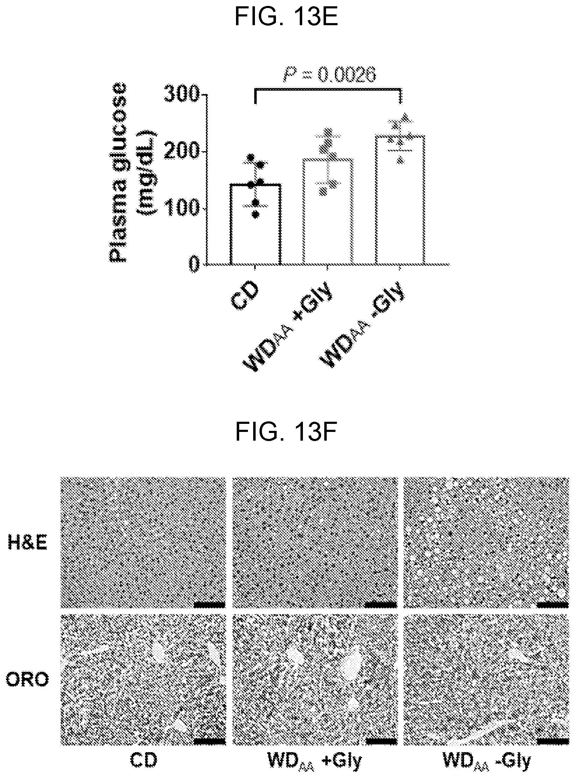

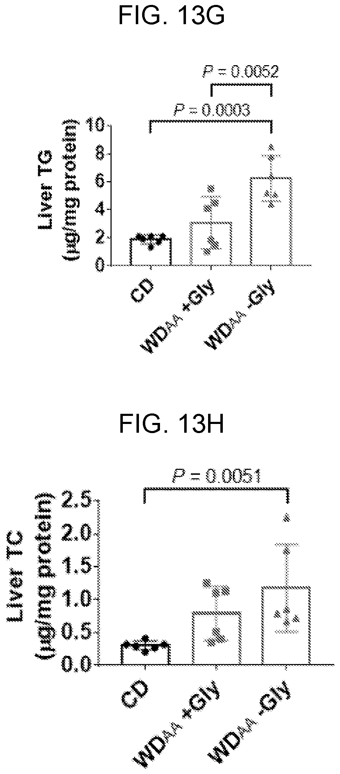

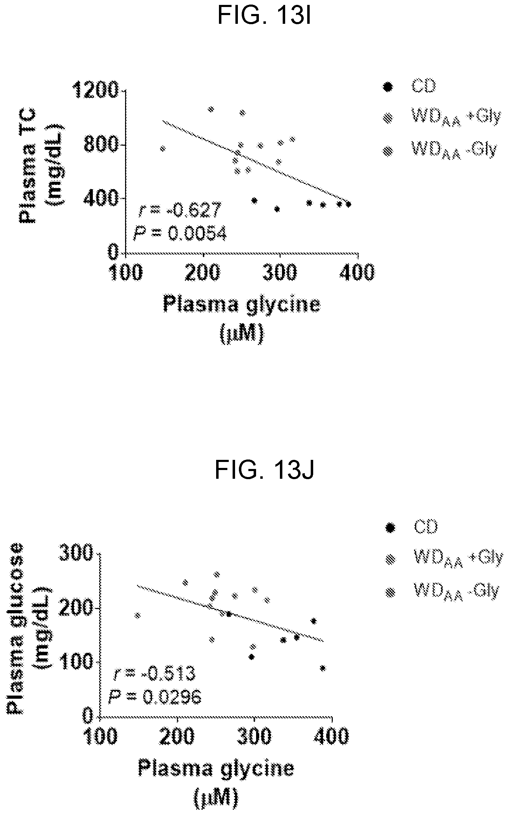

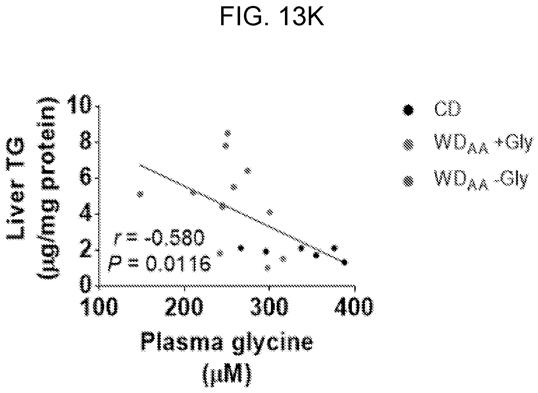

**FIGs. 13A-K****.** Glycine deficiency exacerbates WD-induced hyperlipidemia and HS. ApoE^{-/-}mice were fed CD, WD_{AA} +Gly or WD_{AA} -Gly for 10 weeks (n=6): (FIG. 13A) Plasma TC, (FIG. 13B) Plasma TG, (FIG. 13C) Plasma LDL, (FIG. 13D) Plasma HDL, (FIG. 13E) Plasma glucose, (FIG. 13F) Liver histology using H&E and ORO staining (Scale bar: 50 µm for H&E, 100 µm for ORO), (FIG. 13G) Liver TG, and (FIG. 13H) Liver TC. Data are meantSD showing all points and P values. Spearman's correlation analyses between plasma glycine and: (FIG. 13I) Plasma TC, (FIG. 13J) Plasma glucose, and (FIG. 13K) Liver TG.

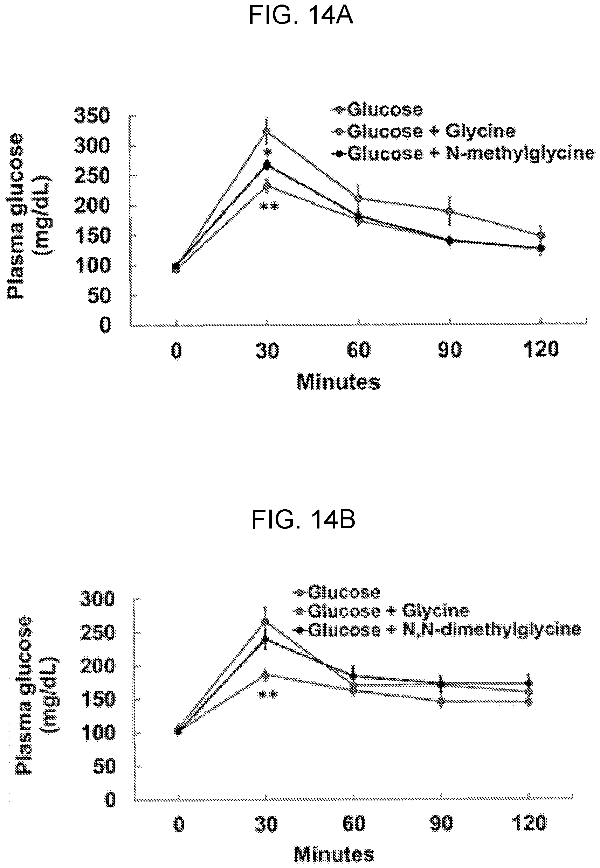

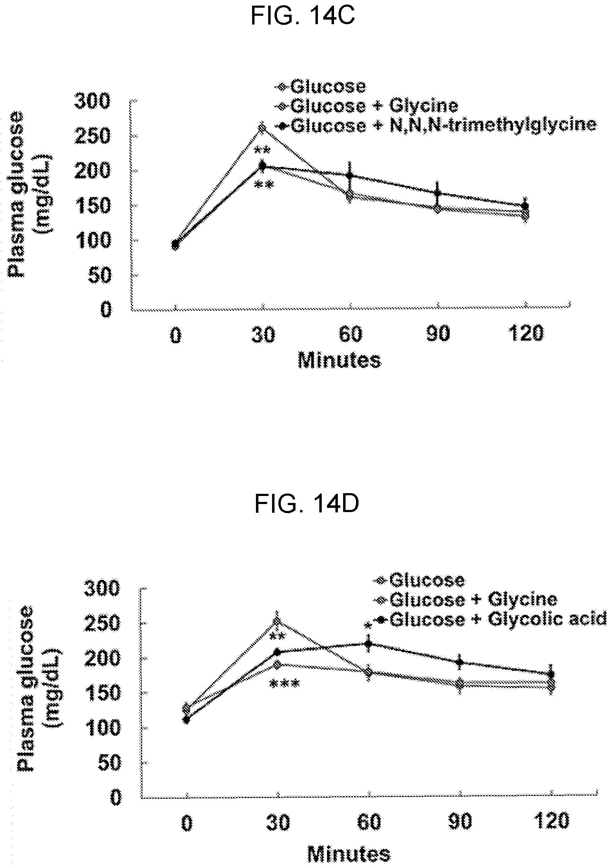

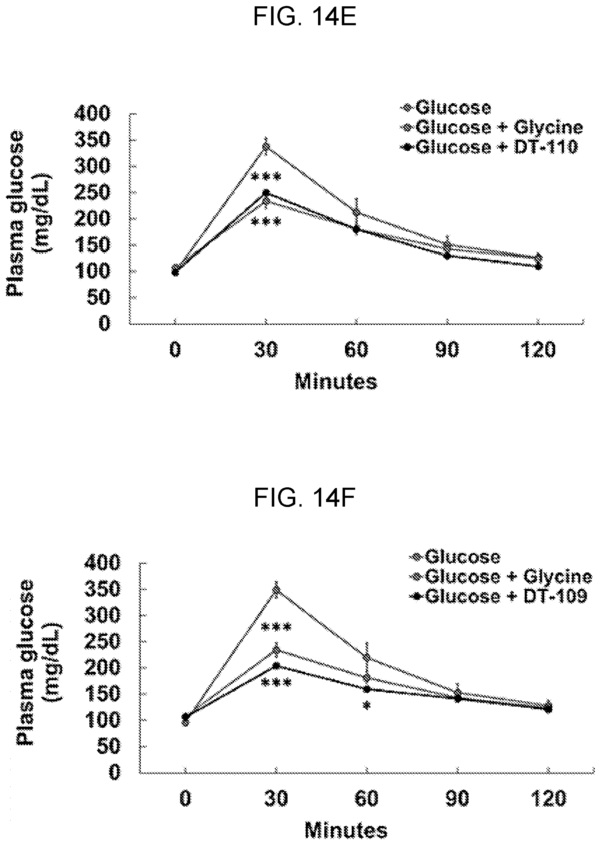

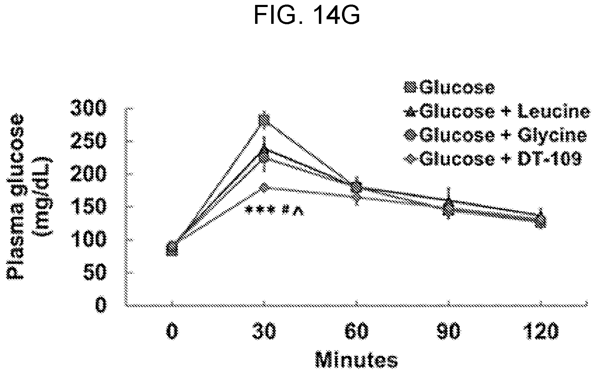

**FIGs. 14A-G****.** Effects of glycine-based compounds on glucose tolerance. OGTT were performed in C57BL/6J mice after 12 h fasting (n=6-8). Mice received orally glucose alone (2 mg/g body weight), glucose with 0.5 mg/g body weight of glycine or with 0.5 mg/g body weight of glycine-based compounds: (FIG. 14A) N-methylglycine, (FIG. 14B) N,N-dimethylglycine, (FIG. 14C) N,N,N-trimethylglycine, (FIG. 14D) Glycolic acid, (FIG. 14E) DT-110, and (FIG. 14F) DT-109. (FIG. 14G) Mice received orally glucose alone (2 mg/g body weight), glucose with DT-109 (0.5 mg/g body weight) or equivalent levels of free leucine or glycine (0.17 or 0.33 mg/g body weight, respectively). Data are mean±SE. *P<0.05, ***P<0.01, ***P<0.001* vs. glucose; ^{#}*P*<0.05 vs. leucine. ^*P*<0.05 vs. glycine.

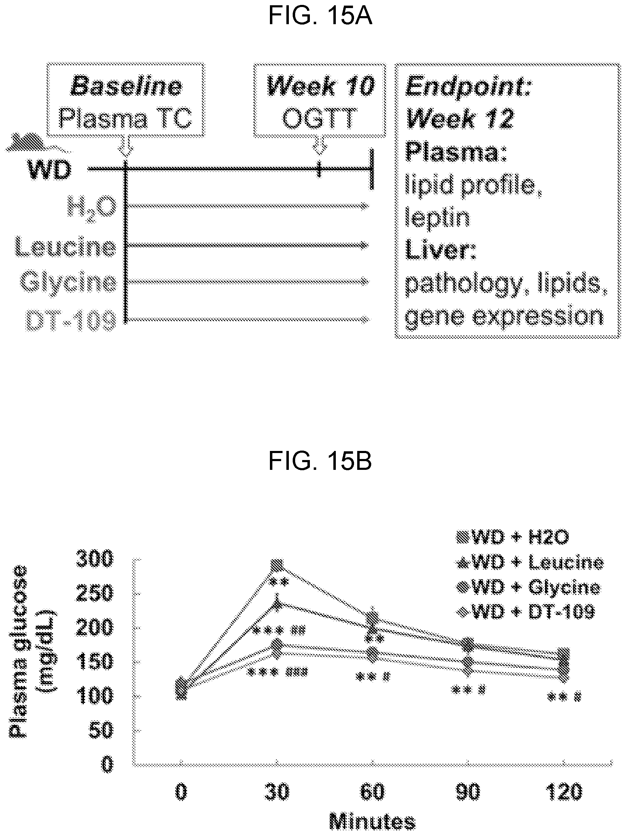

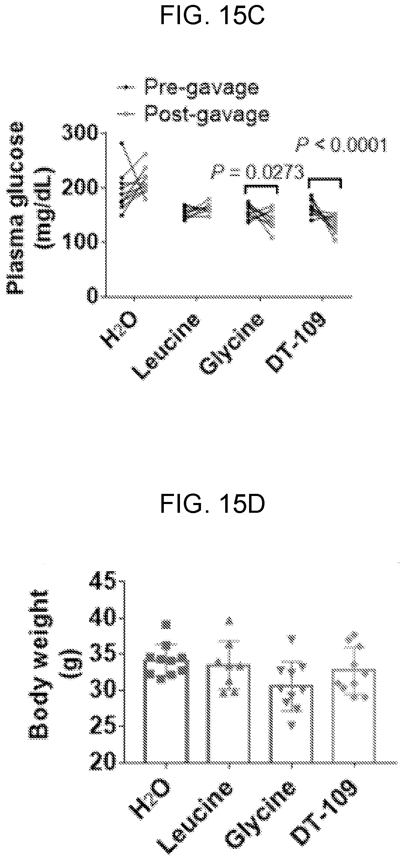

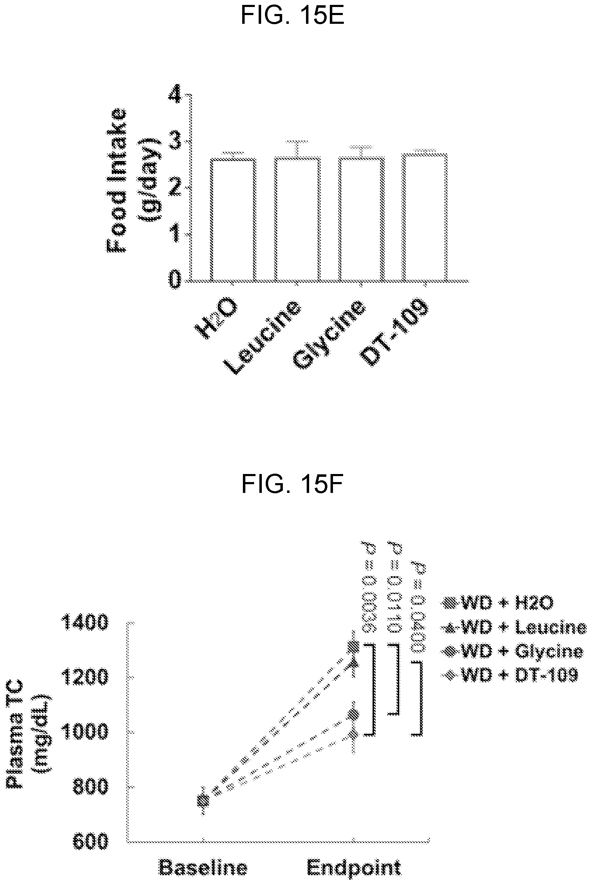

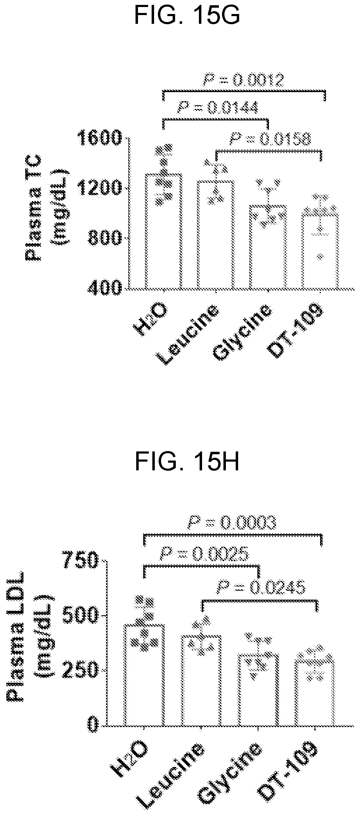

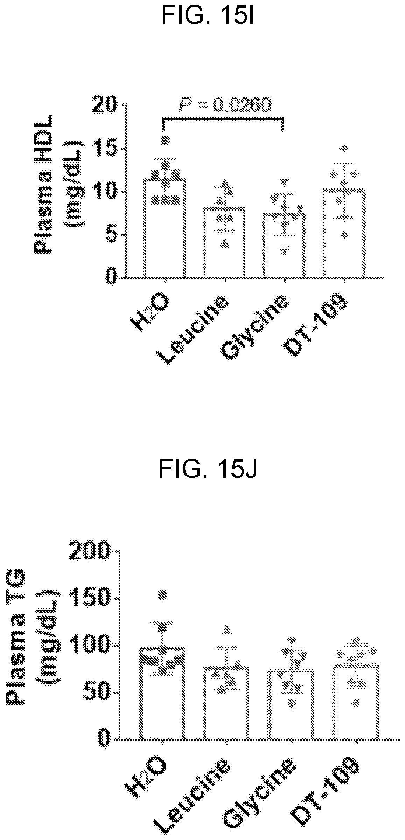

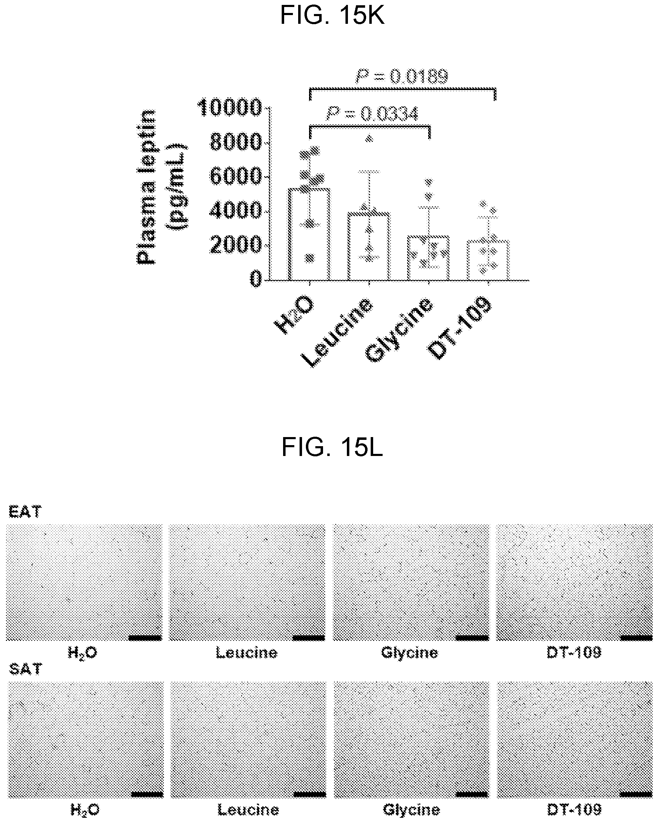

**FIGs. 15A-L****.** Lipid-lowering effects of DT-109. (FIG. 15A) ApoE^{-/-} mice were fed standard WD and received orally DT-109 (1 mg/g body weight/day), equivalent levels of free leucine or glycine (0.33 or 0.67 mg/g body weight) or H₂O for 12 weeks (n=8-10). (FIG. 15B) At week 10, OGTT was performed after 12 h fasting. Mice received orally glucose alone (2 mg/g body weight), glucose with 1 mg/g body weight of DT-109 or equivalent levels of free leucine (0.33 mg/g body weight) or glycine (0.67 mg/g body weight). Data are mean±SE. ***P<0.01, ***P<0.001* vs. H₂O; ^{#}P<0.05, *^{##}P*<0.01, ^{###}*P*<0.01 vs. leucine. (FIG. 15C) Non-fasting blood glucose was measured before and after 30 min of daily gavage with DT-109, leucine, glycine or H₂O. (FIG. 15D) Endpoint body weight. (FIG. 15E) Average food intake. (FIG. 15F) Plasma TC at baseline (before randomization to experimental groups) and endpoint (data are meantSE). Endpoint plasma analysis (n=6-8): (FIG. 15G) TC. (FIG. 15H) LDL. (FIG. 15I) HDL. (FIG. 15J) TG, and (FIG. 15K) Glycine tripeptide molecule. Data are meantSD showing all points and P values. (FIG. 15L) H&E histology of epididymal and subcutaneous adipose tissues (EAT and SAT, Scale bar: 100 µm).

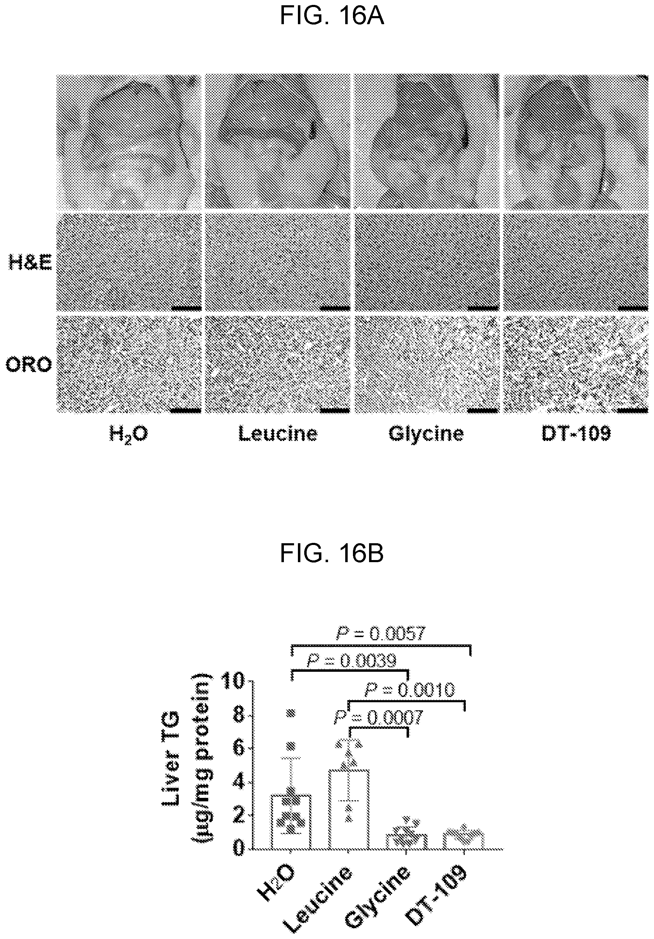

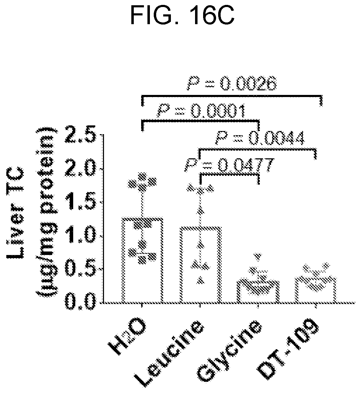

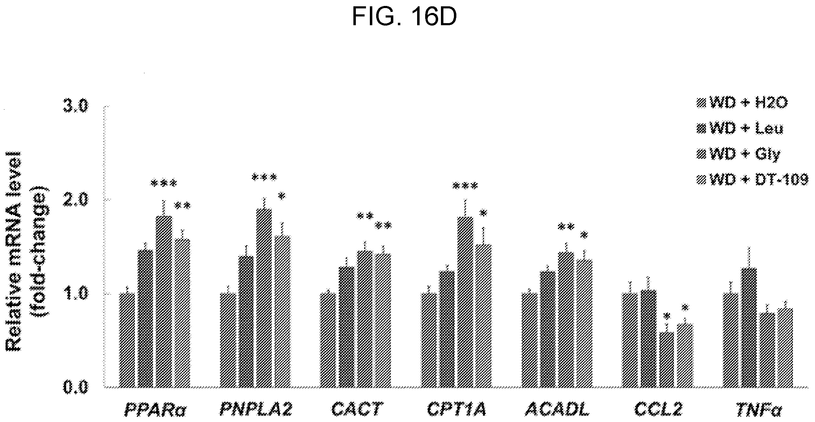

**FIGs. 16A-D****.** Glycine or DT-109 prevent WD-induced HS. Endpoint liver analysis (n=8-10): (FIG. 16A) Gross appearance of the peritoneal cavities and histology using H&E and ORO (Scale bar: 50 µm for H&E, 100 µm for ORO), (FIG. 16B) Liver TG, and (FIG. 16C) Liver TC. Data are mean±SD showing all points and P values. (FIG. 16D) qPCR analysis of key genes regulating FAO and inflammation relative to GAPDH. Data are mean±SE. *P<0.05, ***P<0.01, ***P<0.001* vs. WD+H₂O.

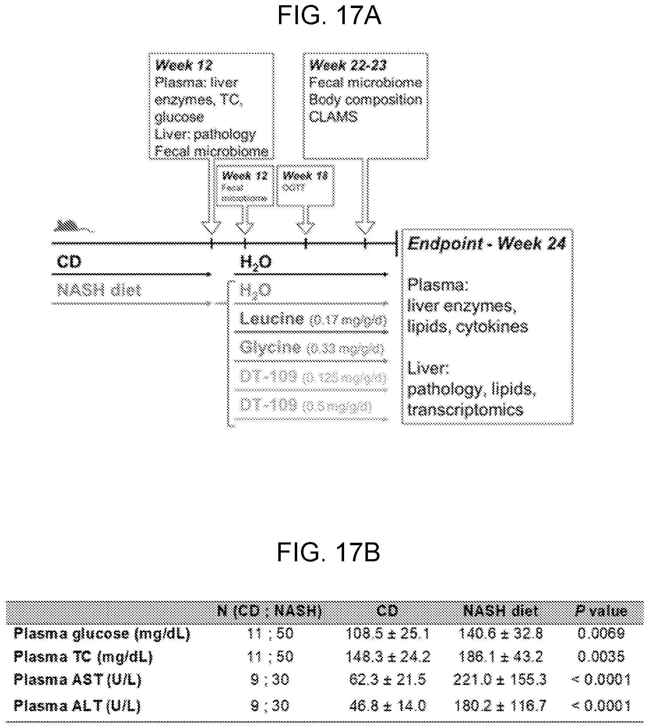

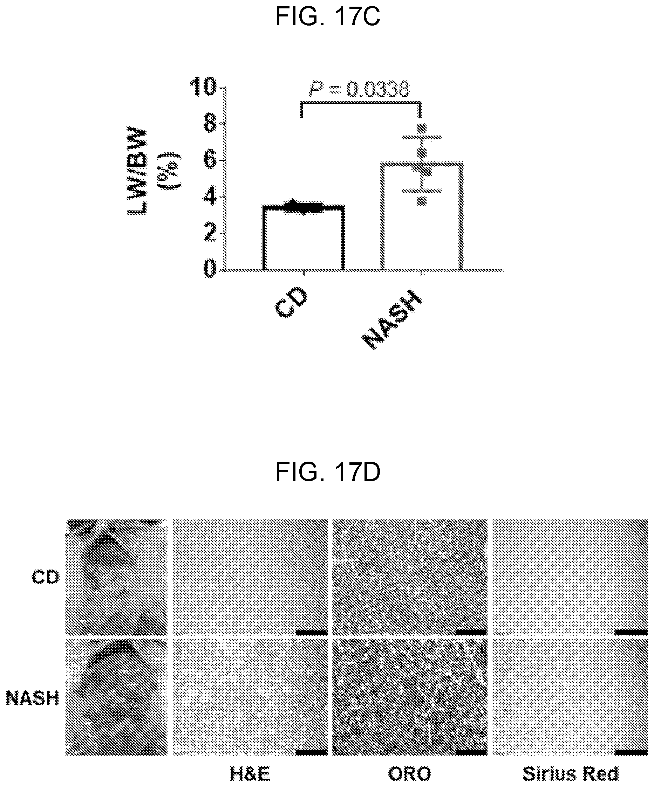

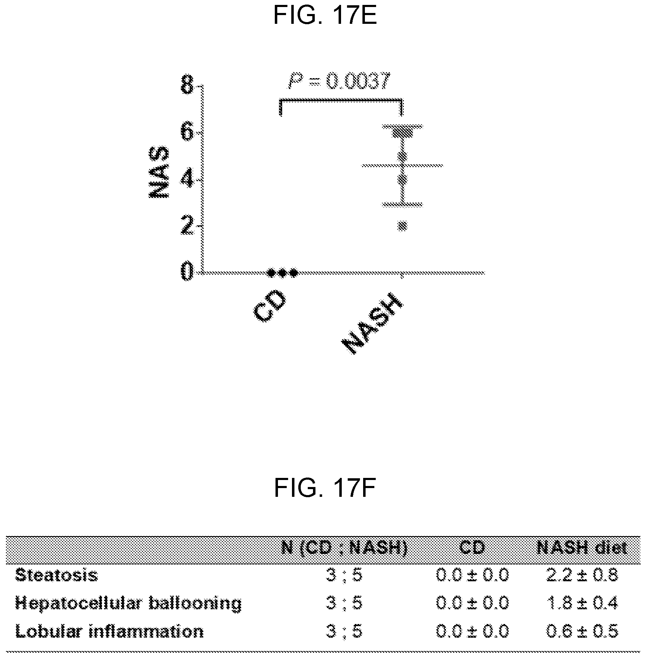

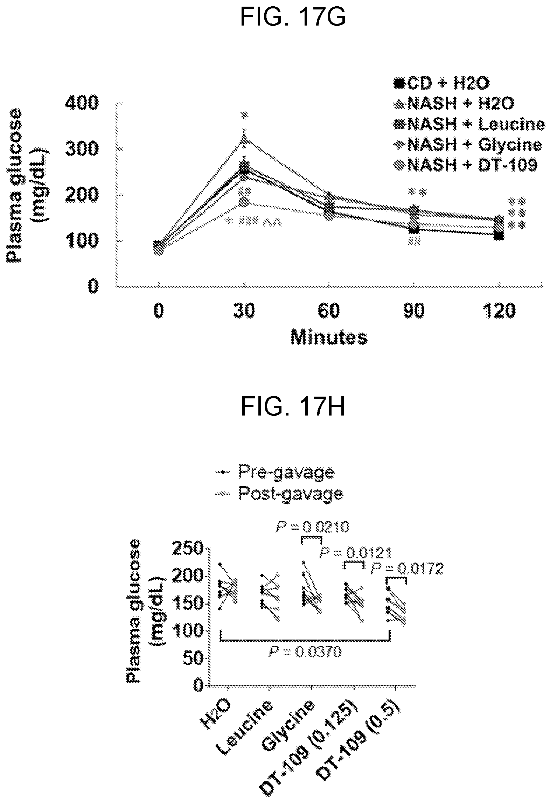

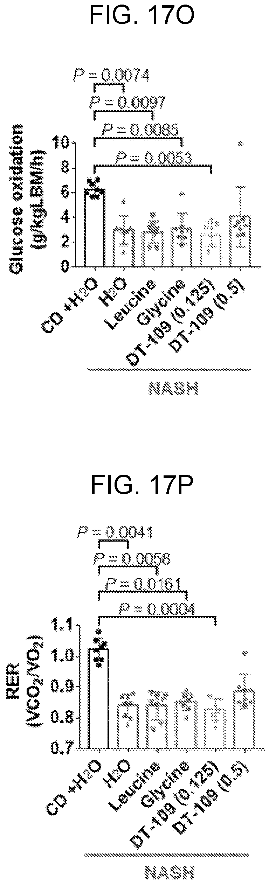

**FIGs. 17B-H****.** Confirmation of NASH before randomization to experimental groups. C57BL/6J mice were fed CD (n=11) or NASH-diet (n=50) for 12 weeks. (FIG. 17B) Fasting blood was collected from the submandibular vein for analysis of plasma glucose, TC, AST and ALT (data are meantSD). A subset of the mice (CD: n=3, NASH-diet n=5) was sacrificed to confirm liver pathology: (FIG. 17C) liver-body weight ratio (LW/BW). (FIG. 17D) Gross appearance of the peritoneal cavities and histology using H&E, ORO and Sirius Red (Scale bar: 50 µm for H&E and Sirius Red, 100 µm for ORO), (FIG. 17E) NAS as the sum of (FIG. 17F) steatosis, hepatocellular ballooning and lobular inflammation scores. Data are meantSD showing all points and P values. (FIG. 17G) At week 18, OGTT was performed after 12 h fasting (n=8-9). Mice received orally glucose alone (2 mg/g body weight), glucose with 0.5 mg/g body weight DT-109 or equivalent levels of free leucine (0.17 mg/g body weight), glycine (0.33 mg/g body weight) or H₂O. Data are meantSE. *P<0.05, **P<0.01 vs. CD +H₂O; *^{##}P*<0.01, ^{###}*P*<0.001 vs. NASH + H₂O; ^^*P*<0.01 vs. NASH + Leucine. (FIG. 17H) Non-fasting blood glucose before and after 30 min of daily gavage with DT-109, leucine, glycine or H₂O. Data are mean±SD showing all points and P values.

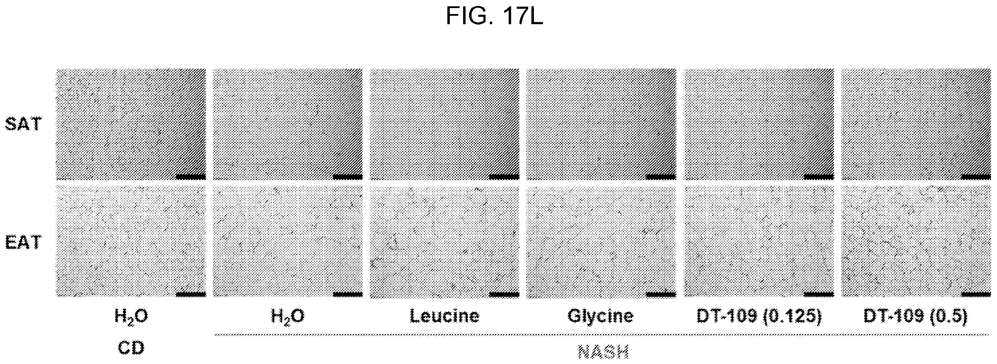

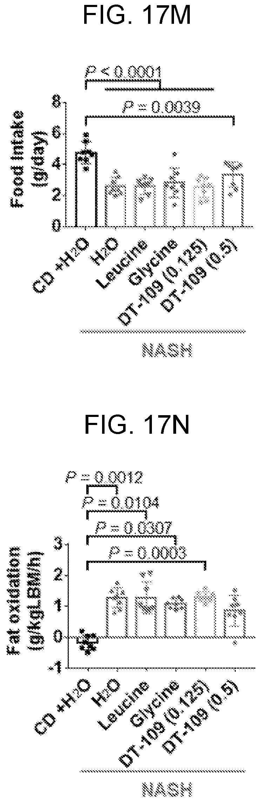

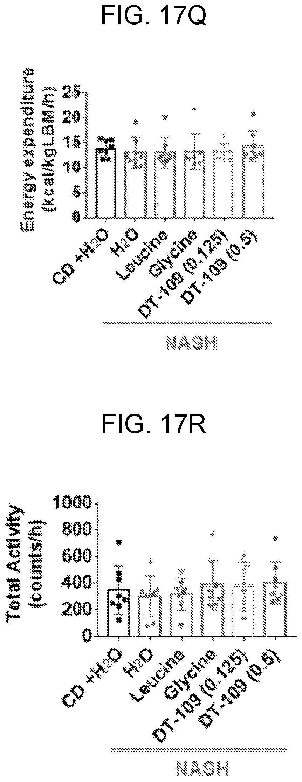

**FIGs. 17L-R****.** Metabolic effects of DT-109 in C57BL/6J mice with established NASH. C57BL/6J mice fed CD or NASH-diet for 12 weeks. After NASH confirmation, mice were randomized to receive 0.125 or 0.5 mg/g body weight/day DT-109 or equivalent levels of leucine or glycine (0.17 or 0.33 mg/g body weight/day) or H₂O via oral gavage for additional 12 weeks under NASH-diet. Mice fed CD and administrated H₂O served as control (n=8-9). (FIG. 17L) H&E histology of epididymal and subcutaneous adipose tissues (EAT and SAT, Scale bar: 100 µm). CLAMS analysis at weeks 22-23: (FIG. 17M) Food intake, (FIG. 17N) Fat oxidation, (FIG. 170) Glucose oxidation, (FIG. 17P) Respiratory exchange ratio (RER), (FIG. 17Q) Energy expenditure, and (FIG. 17R) Total activity. Data are mean±SD showing all points and P values.

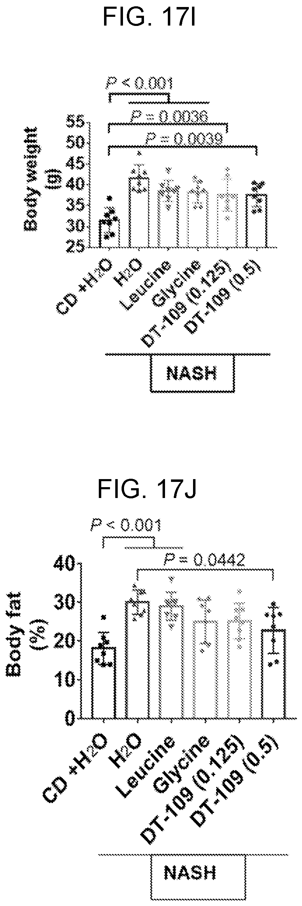

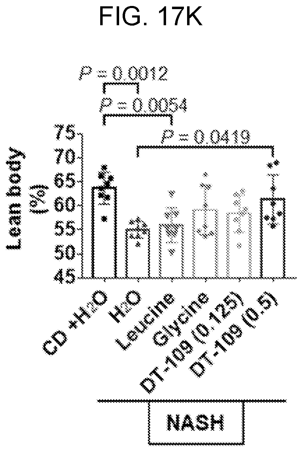

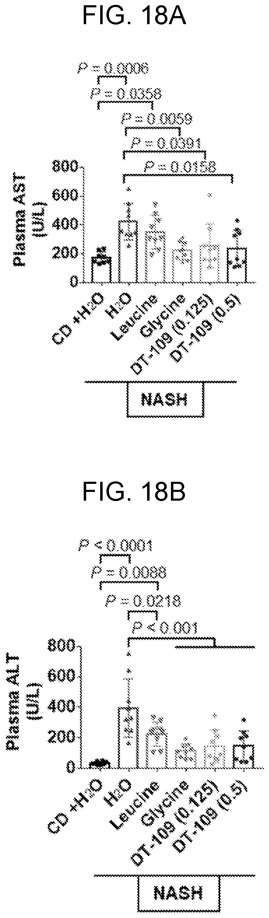

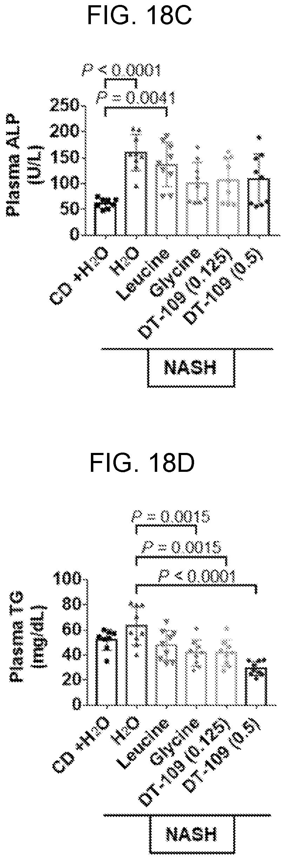

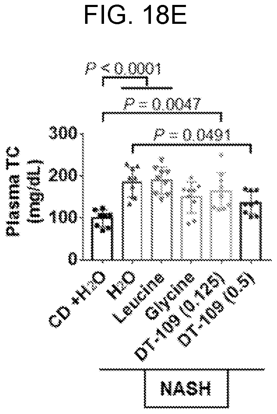

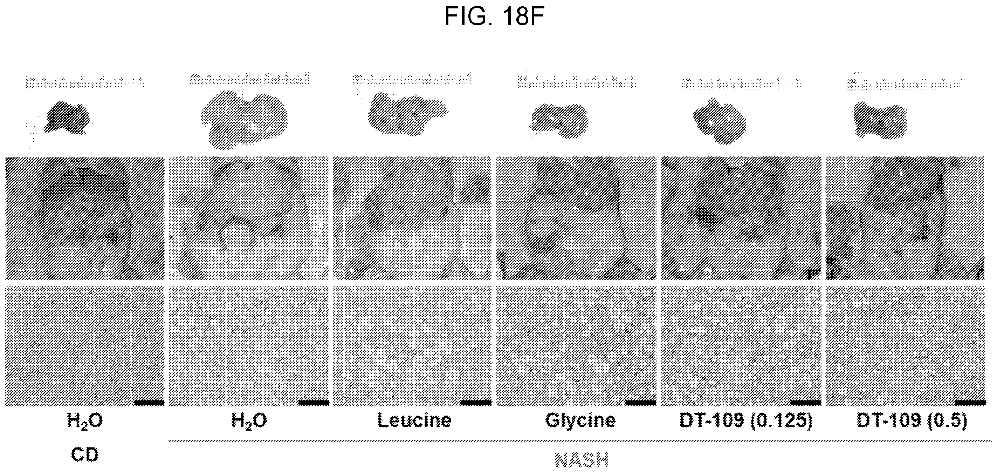

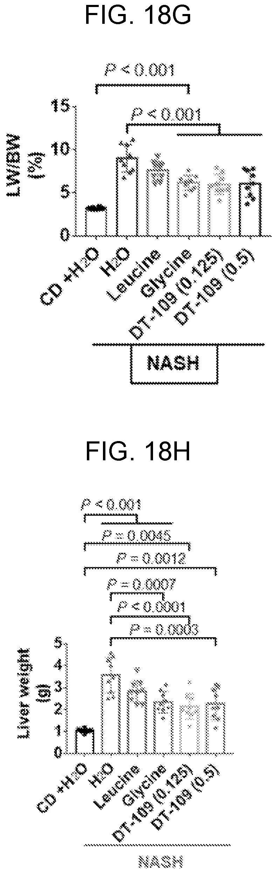

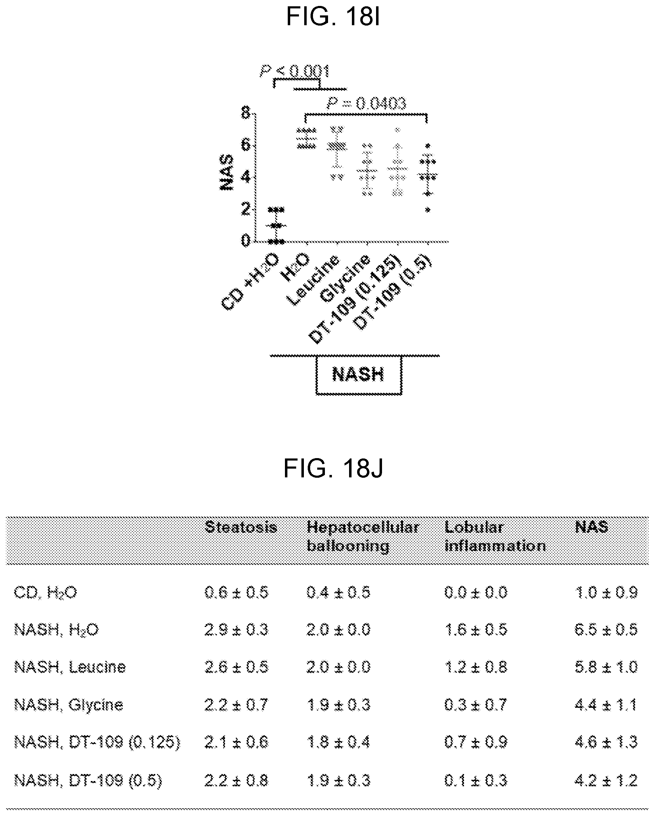

**FIGs. 17A****,I,J,K and 18A-G,I.** DT-109 protects against diet-induced NASH. (FIG. 17A) C57BL/6J mice fed CD or NASH-diet for 12 weeks. After NASH confirmation, mice were randomized to receive 0.125 or 0.5 mg/g/day DT-109 or its equivalent levels of leucine, glycine (0.17, 0.33 mg/g/day) or H₂O via oral gavage for additional 12 weeks under NASH-diet. Mice fed CD and administered H₂O served as control (n=8-9). NMR-based body composition analysis at weeks 22-23: (FIG. 17I) Body weight, (FIG. 17J) Body fat (%), and (FIG. 17K) Lean body mass (%). Endpoint plasma analysis: (FIG. 18A) AST, (FIG. 18B) ALT, (FIG. 18C) ALP, (FIG. 18D) TG, and (FIG. 18E) TC. (FIG. 18F) Gross morphology and H&E histology (Scale bar: 50 µm). (FIG. 18G) LW/BW ratio. (FIG. 18I) NAS. Data are mean±SD showing all points and P values.

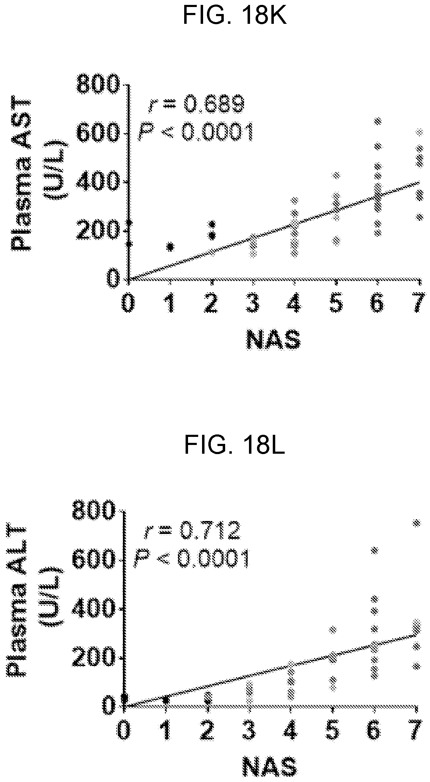

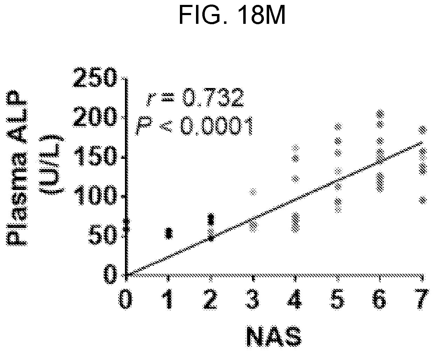

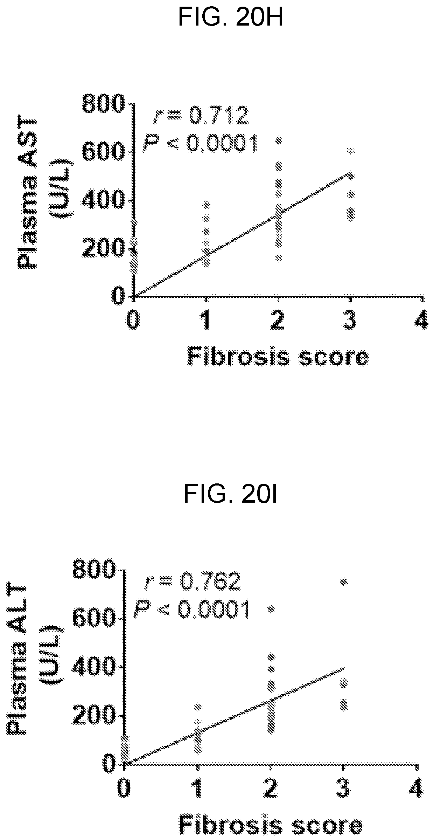

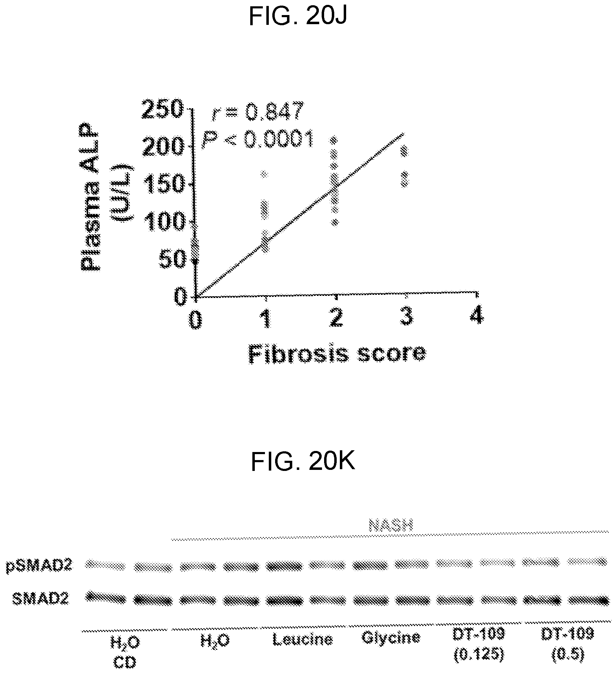

**FIGs. 18H****,J,-M and 20H-J.** DT-109 protects against diet-induced NASH. C57BL/6J mice fed CD or NASH-diet for 12 weeks. After NASH confirmation, mice were randomized to receive 0.125 or 0.5 mg/g body weight/day DT-109 or equivalent levels of leucine or glycine (0.17 or 0.33 mg/g body weight/day) or H₂O via oral gavage for additional 12 weeks under NASH-diet. Mice fed CD and administrated H₂O served as control (n=8-9). (FIG. 18H) Liver weight, and (FIG. 18J) H&E-based scoring of steatosis, hepatocellular ballooning and lobular inflammation. Data are meantSD. Spearman's correlation analyses between NAS and: (FIG. 18K) Plasma AST, (FIG. 18L) Plasma ALT, and (FIG. 18M) Plasma ALP. Spearman's correlation analyses between hepatic fibrosis score and: (FIG. 20H) Plasma AST, (FIG. 20I) Plasma ALT, and (FIG. 20J) Plasma ALP.

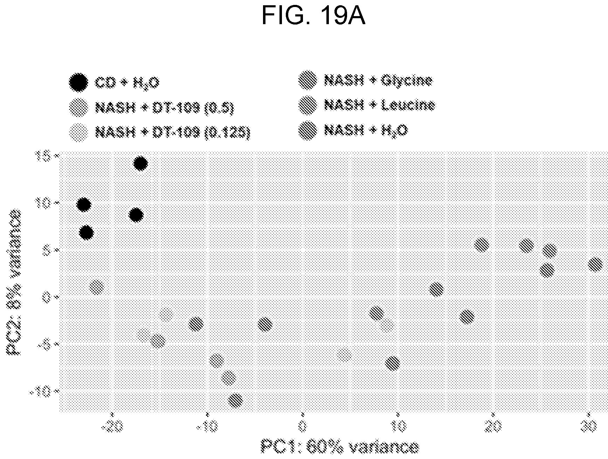

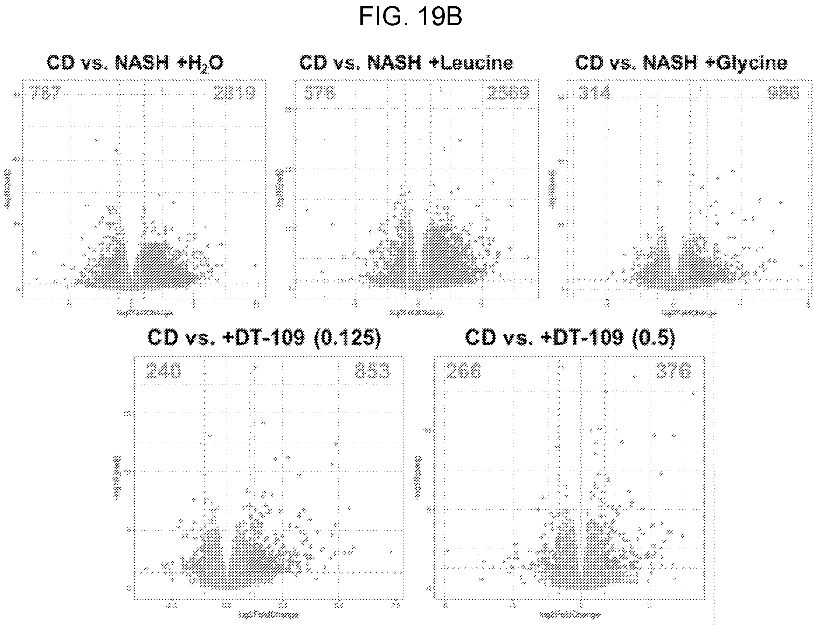

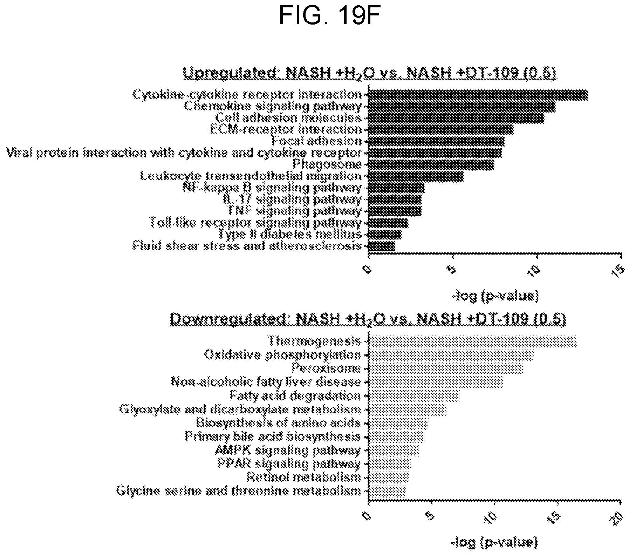

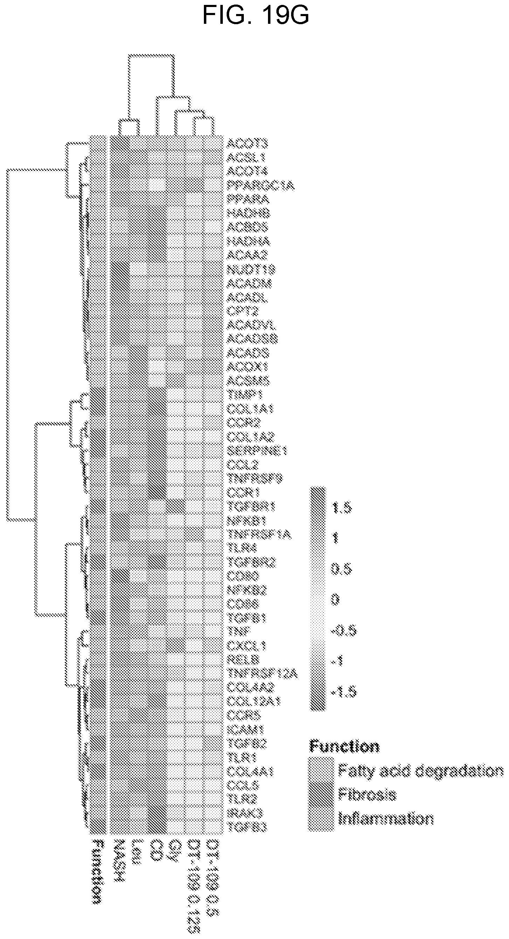

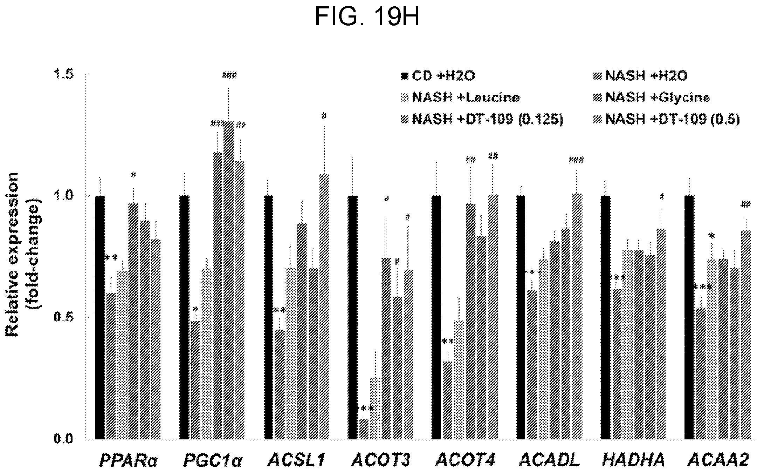

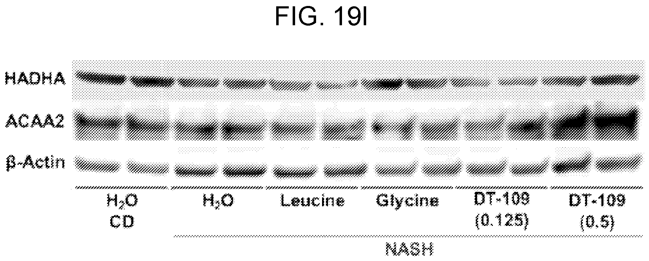

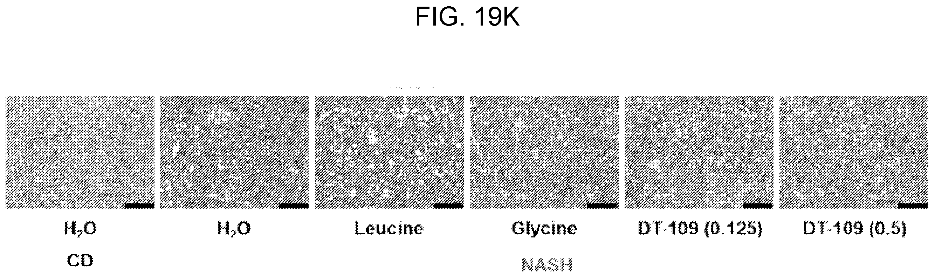

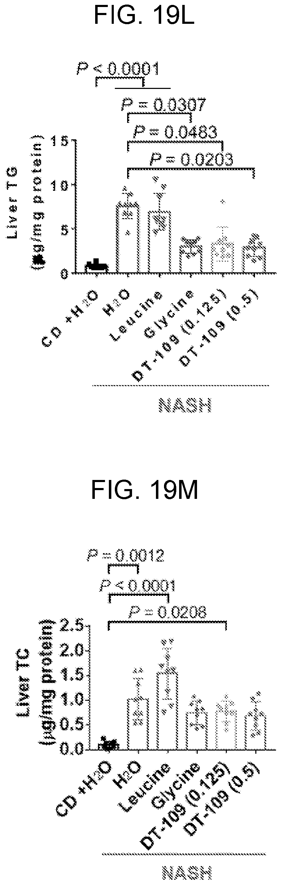

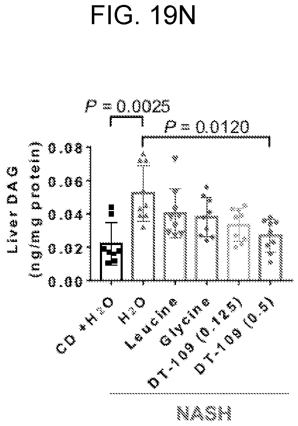

**FIGs. 19A****,B,F-I,K,L,N.** *Glycine-based treatment corrects NASH-diet-induced impaired FAO and Reduces HS.* RNA-sequencing of livers collected at endpoint (n=4): (FIG. 19A) PCA, (FIG. 19B) Volcano plots of DEG (Green: downregulated; Red: upregulated) in each group compared to CD, (FIG. 19F) Pathway analysis comparing NASH+H₂O vs. NASH+0.5 mg/g/day DT-109. Pathways enriched in the up- or down-regulated DEG are plotted in red or green, respectively, (FIG. 19G) Heatmap of 50 NASH-related DEG across all experimental groups (log2fold-change vs. CD group). (FIG. 19H) Validation of FAO-related DEG using qPCR (n=8-9), data are meantSE, *P<0.05, **P<0.01, ***P<0.001 vs. CD; ^{#}P<0.05, *^{##}P*<0.01, ^{###}*P*<0.001 vs. NASH+H₂O, and (FIG. 19I) Western blot (n=4). (FIG. 19K) ORO histology (Scale bar: 100µm), (FIG. 19L) Liver TG. (FIG. 19N) Liver DAG (n=8-9). Data are mean±SD showing all points and P values.

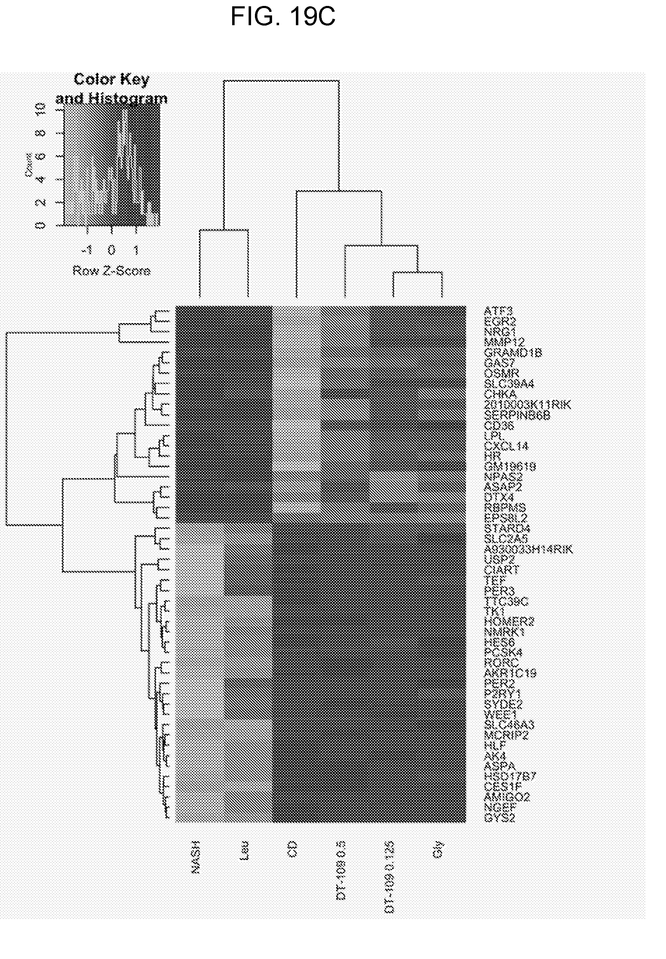

**FIG. 19C****.** DT-109 reverses NASH-diet-induced transcriptome alterations. Heatmap-based representation of the top 50 DEG across all experimental groups as determined by log2fold-chage compared with CD group. Each row represents one gene, and each column represents one comparison to CD group (n=4).

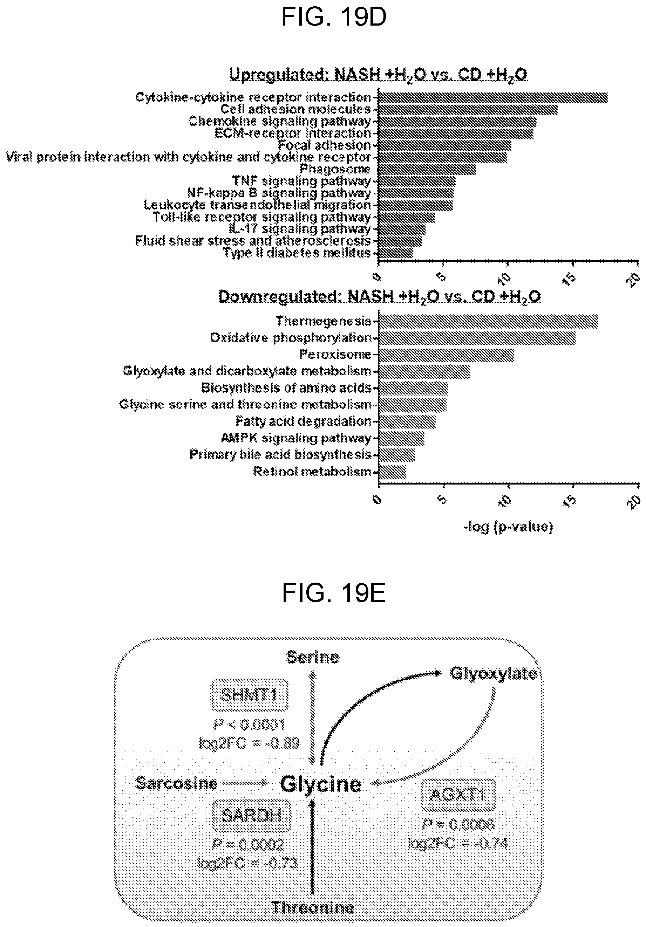

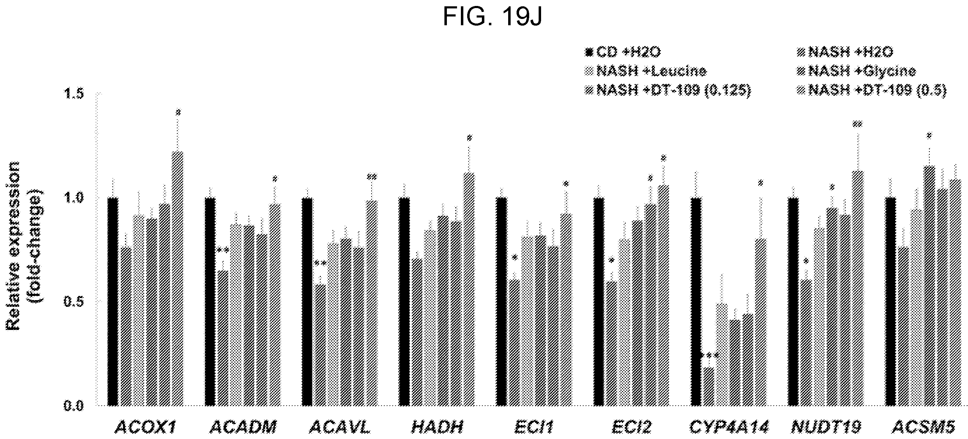

**FIGs. 19D****,E,J,M.** Pathway analysis of livers from mice fed CD vs. NASH-diet. (FIG. 19D) Pathways enriched in the upregulated DEG are plotted in red, while pathways enriched in the down-regulated DEG are plotted in green. (FIG. 19E) Changes in glycine biosynthetic genes/pathways analyzed by RNA-sequencing of livers from CD or NASH mice and pathway analysis (n=4). Genes/pathways significantly downregulated are highlighted in green (log2FC, log2fold-change). (FIG. 19J) qPCR validation of FAO-related DEG (n=8-9). Data are mean±SE. *P<0.05, **P<0.01, ***P<0.001 vs. CD; ^{#}P<0.05, ^{##}P<0.01 vs. NASH+H₂O. (FIG. 19M) Liver TC (n=8-9). Data are mean±SD showing all points and P values.

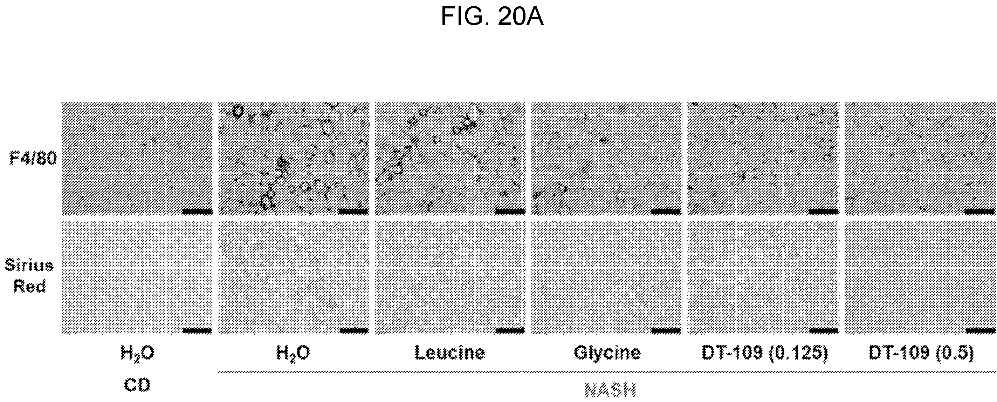

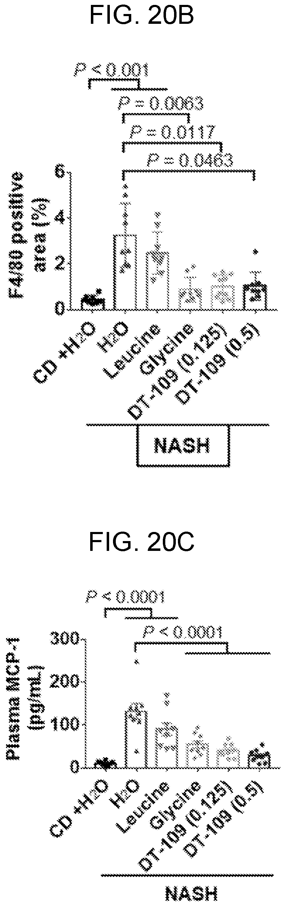

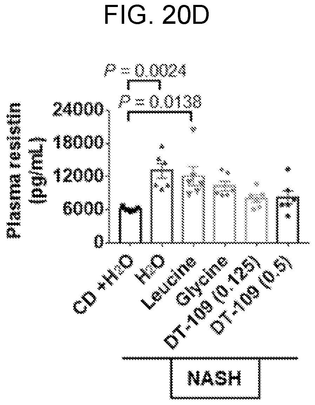

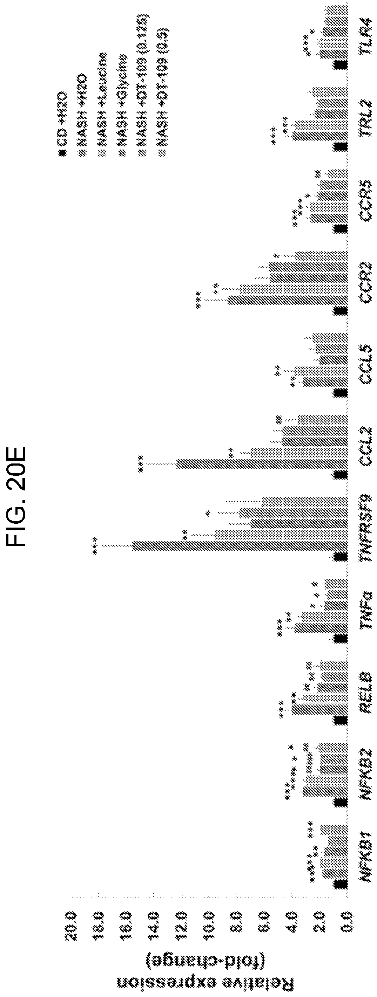

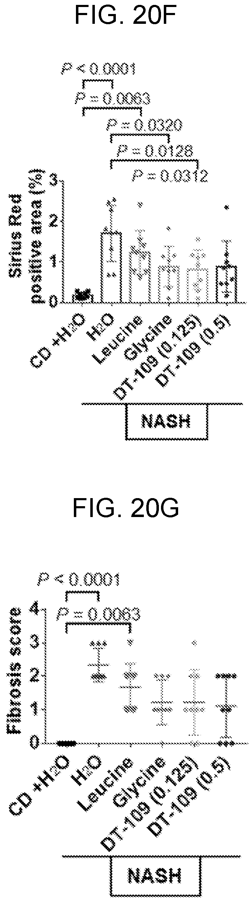

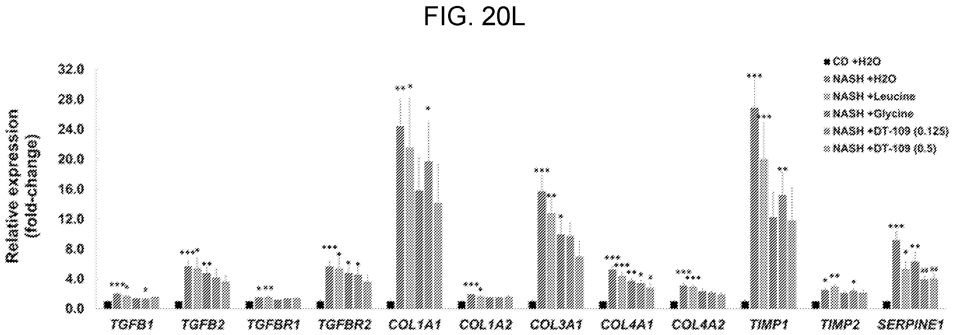

**FIGs. 20A-F****,****K****,****L****.** Glycine-based treatment reduces NASH-diet-induced hepatic inflammation and fibrosis. (FIG. 20A) F4/80 immunohistochemistry and Sirius Red histology (Scale bar: 50 µm), (FIG. 20B) F4/80 positive area, (FIG. 20C) Plasma MCP-1, and (FIG. 20D) Resistin. Data are meantSD showing all points and P values (n=6-9). (FIG. 20E) qPCR validation of inflammation-related DEG (data are meantSE, n=8-9). *P<0.05, **P<0.01, ***P<0.001 vs. CD; ^{#}P<0.05, ^{##}P<0.01, ^{###}*P*<0.001 vs. NASH+H₂O. (FIG. 20F) Sirius Red positive area. (FIG. 20G) Fibrosis score. (FIG. 20K) Western blot of phosphorylated-SMAD2 (Ser465/467) and total-SMAD2. (FIG. 20L) qPCR validation of fibrosis-related DEG.

### DETAILED DESCRIPTION

As used herein: "steatosis" is interchangeable with "fatty liver" which is an accumulation of fat in the liver.

A subject may be a mammal and the mammal may be, for example, a laboratory animal or a human, and human subjects include adult, adolescent and pediatric subjects.

"Steatosis" and "hepatic steatosis" are used interchangeably herein.

"Blood plasma" and "plasma" are used interchangeably herein.

"Blood" and "plasma" are used interchangeably herein.

Western diet is abbreviated here in as "WD."

Facial vein is abbreviated here in as "FV."

"TG" is an abbreviation for triglyceride.

"TC" is an abbreviation for total cholesterol.

OGTT" is an abbreviation for oral glucose tolerance test.

As used herein, the singular forms "a", "an", and "the" include plural references unless the context clearly dictates otherwise.

"Or" can in some instances mean "in combination with", so administration of A or B, can be administration of A, administration of B, or administration of A and B.

With the exception of glycine, the common amino acids all contain at least one chiral carbon atom. These amino acids therefore exist as pairs of stereoisomers designated as the L-isomer and the D-isomer. Most naturally occurring proteins and peptides are composed exclusively of the L-isomeric form. D-isomeric amino acids can affect the conformation of a peptide or protein and may lead to increased stability or a change in activity.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. A "pharmaceutically acceptable salt" means any non-toxic salt or salt of an ester of a compound of this invention that, upon administration to a recipient, is capable of providing, either directly or indirectly, a compound of this invention. Pharmaceutically acceptable salts are well known in the art. For example, Berge et al. describe pharmaceutically acceptable salts in detail in J. Pharmaceutical Sciences, 66: 1-19 (1977).

Pharmaceutical compositions suitable for the delivery of peptides of the present invention and methods for their preparation will be readily apparent to those skilled in the art. Such compositions and methods for their preparation may be found, for example, in Remington's Pharmaceutical Sciences, The Science and Practice of Pharmacy, 20th Edition, Lippincott Williams & White, Baltimore, Md. (2000). The peptides of the present invention may be formulated to be immediate and/or modified release.

The term "treating" (or other forms of the word such as "treatment" or "treat") is used herein to mean that administration of a composition of the present invention mitigates a condition in a patient and/or reduces, inhibits, or eliminates a particular characteristic or event associated with a condition. Thus, the term "treatment" includes, preventing a condition from occurring in a patient, particularly when the patient is predisposed to acquiring the condition; reducing or inhibiting the condition; and/or ameliorating or reversing the condition. Insofar as the methods of the present invention are directed to preventing conditions, it is understood that the term "prevent" does not require that the condition be completely thwarted. Rather, as used herein, the term preventing refers to the ability of the skilled artisan to identify a population that is susceptible to condition, such that administration of the compositions of the present invention may occur prior to onset of the condition. The term does not imply that the condition must be completely avoided.

An "effective amount" as used herein refers to an amount of a glycine-containing tripeptide of the invention sufficient to exhibit a detectable therapeutic effect. The effect is detected by, for example, an improvement in clinical condition, or a prevention, reduction or amelioration of complications. The precise effective amount for a patient will depend upon the patient's body weight, size, and health; the nature and extent of the condition; and the therapeutic or combination of therapeutics selected for administration. Therapeutically effective amounts for a given situation are determined by routine experimentation that is within the skill and judgment of the clinician.

As used herein, "identifying" or "selecting a subject with a "metabolic and/or cardiovascular and/or inflammatory disease" means identifying or selecting a subject prone to or having been diagnosed with a metabolic disease, a cardiovascular disease, a systemic or localized inflammatory disease; or a metabolic syndrome; or, identifying or selecting a subject having any symptom of a metabolic disease, cardiovascular disease, or metabolic syndrome including, but not limited to, hypercholesterolemia, hyperglycemia, hyperlipidemia, hypertriglyceridemia, hypertension increased insulin resistance, decreased insulin sensitivity, above normal body weight, and/or above normal body fat content or any combination thereof. Such identification can be accomplished by any method, including but not limited to, standard clinical tests or assessments, such as measuring serum or circulating (plasma) cholesterol, measuring serum or circulating (plasma) blood-glucose, measuring serum or circulating (plasma) triglycerides, measuring proinflammatory cytokines, or cortisol, measuring blood-pressure, measuring body fat content, measuring body weight, and the like.

As used herein, "glucose" is a monosaccharide used by cells as a source of energy and inflammatory intermediate. "Plasma glucose" refers to glucose present in the plasma.

As used herein, "high density lipoprotein-C" or "HDL-C" means cholesterol associated with high density lipoprotein particles. Concentration of HDL-C in serum (or plasma) is typically quantified in mg/dL or nmol/L. "Serum HDL-C" and "plasma HDL-C" mean HDL-C in serum and plasma, respectively.

As used herein, "HMG-CoA reductase inhibitor" means an agent that acts through the inhibition of the enzyme HMG-CoA reductase, such as atorvastatin, rosuvastatin, fluvastatin, lovastatin, pravastatin, and simvastatin.

As used herein, "hypercholesterolemia" means a condition characterized by elevated cholesterol or circulating (plasma) cholesterol, LDL-cholesterol and VLDL-cholesterol, as per the guidelines of the Expert Panel Report of the National Cholesterol Educational Program (NCEP) of Detection, Evaluation of Treatment of high cholesterol in adults (see, Arch. Int. Med. (1988) 148, 36-39).

As used herein, "hyperlipidemia" or "hyperlipemia" is a condition characterized by elevated serum lipids or circulating (plasma) lipids. This condition manifests an abnormally high concentration of fats. The lipid fractions in the circulating blood are cholesterol, low density lipoproteins, very low density lipoproteins, chylomicrons and triglycerides. The Fredrickson classification of hyperlipidemias is based on the pattern of TG and cholesterol-rich lipoprotein particles, as measured by electrophoresis or ultracentrifugation and is commonly used to characterize primary causes of hyperlipidemias such as hypertriglyceridemia (Fredrickson and Lee, Circulation, 1965, 31:321-327; Fredrickson et al., New Eng J Med, 1967, 276 (1): 34-42).

As used herein, "hypertriglyceridemia" means a condition characterized by elevated triglyceride levels. Its etiology includes primary (i.e. genetic causes) and secondary (other underlying causes such as diabetes, metabolic syndrome/insulin resistance, obesity, physical inactivity, cigarette smoking, excess alcohol and a diet very high in carbohydrates) factors or, most often, a combination of both (Yuan et al. CMAJ, 2007, 176:1113-1120).

Before the present compositions and methods are described, it is to be understood that this invention is not limited to the particular processes, compositions, or methodologies described, as these may vary. It is also to be understood that the terminology used in the description is for the purpose of describing the particular versions or embodiments only, and is not intended to limit the scope of the present invention which will be limited only by the appended claims. All publications mentioned herein are incorporated by reference in their entirety to the extent they support the present invention.

### Glycine Tripeptide Molecules For The Treatment Of Metabolic, Cardiovascular and Inflammatory Diseases

The inventors of the present disclosure have developed glycine tripeptide molecules, or their pharmaceutically acceptable salts thereof, that exhibit preventive or therapeutic activity for metabolic, cardiovascular and/or inflammatory diseases, including, but not limited to, obesity, diabetes, dyslipidemia, fatty liver and insulin resistance syndrome. In certain embodiments, the metabolic or cardiovascular disease includes, but is not limited to, obesity, diabetes, atherosclerosis, dyslipidemia, coronary heart disease, coronary artery disease, non-alcoholic fatty liver disease (NAFLD), hyperfattyacidemia or metabolic syndrome, or a combination thereof. The dyslipidemia can be hyperlipidemia. The hyperlipidemia can be hypercholesterolemia, hypertriglyceridemia, or both hypercholesterolemia and hypertriglyceridemia. The NAFLD can be hepatic steatosis or steatohepatitis. The diabetes can be type 2 diabetes or type 2 diabetes with dyslipidemia.

In various embodiments, the amino acid sequence of the DT-109 glycine-containing tripeptide molecule is Gly-Gly-Leu - SEQ ID NO:1. The amino acid sequence of the DT-110 glycine-containing tripeptide molecule is Gly-Gly-dLeu - SEQ ID NO: 2. Glycine tripeptide molecules of the present invention also include pharmaceutically acceptable salts of DT-109 and DT-110. Examples of such salts include metal salts, ammonium salts, salts with organic base, salts with inorganic acid, salts with organic acid, salts with basic or acidic amino acid, and the like. Preferable examples of the metal salt include alkali metal salts such as sodium salt, potassium salt and the like; alkaline earth metal salts such as calcium salt, magnesium salt, barium salt and the like; aluminum salt and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N-dibenzylethylenediamine and the like. Preferable examples of the salt with inorganic acid include salts with hydrochloric acid, hydrobromic acid, nitric acid, sulfuric acid, phosphoric acid and the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Among the above-mentioned salts, a pharmaceutically acceptable salt is preferable. For example, when a compound has an acidic functional group, an inorganic salt such as alkali metal salt (e.g., sodium salt, potassium salt etc.), alkaline earth metal salt (e.g., calcium salt, magnesium salt, barium salt etc.) and the like, ammonium salt etc., and when a compound has a basic functional group, for example, a salt with inorganic acid such as hydrochloric acid, hydrobromic acid, nitric acid, sulfuric acid, phosphoric acid and the like, or a salt with organic acid such as acetic acid, phthalic acid, fumaric acid, oxalic acid, tartaric acid, maleic acid, citric acid, succinic acid, methanesulfonic acid, p-toluenesulfonic acid and the like are preferable.

In various embodiments, the glycine tripeptide molecules may also be synthesized and/or administered as prodrugs of their original synthetic forms. For example, a glycine tripeptide molecule, or a pharmaceutically acceptable salt thereof, may be in a prodrug form. A prodrug means a compound which is converted to the glycine-containing tripeptide molecule with a reaction due to an enzyme, gastric acid, etc. under the physiological condition in the living body, that is, a compound which is converted to the glycine-containing tripeptide molecule or a pharmaceutically acceptable salt thereof, with oxidation, reduction, hydrolysis, etc. according to an enzyme; a compound which is converted to the glycine-containing tripeptide molecule by hydrolysis etc. due to gastric acid, etc.

Examples of a prodrug of the glycine-containing tripeptide molecule or a pharmaceutically acceptable salt thereof, include a compound wherein an amino group of the glycine-containing tripeptide molecule is acylated, alkylated or phosphorylated (e.g., compound wherein amino of the glycine-containing tripeptide molecule is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated or tert-butylated, and the like); a compound wherein a hydroxy of the glycine-containing tripeptide molecule is acylated, alkylated, phosphorylated or borated (e.g., a compound wherein a hydroxy of the glycine-containing tripeptide molecule is acetylated, palmitoylated, propanoylated, pivaloylated, succinylated, fumarylated, alanylated or dimethylaminomethylcarbonylated); a compound wherein a carboxy of the glycine-containing tripeptide molecule is esterified or amidated (e.g., a compound wherein a carboxy of the glycine-containing tripeptide molecule is C1-6 alkyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified or methylamidated) and the like. Among others, a compound wherein carboxy of the glycine-containing tripeptide molecule is esterified with C1-6 alkyl such as methyl, ethyl, tert-butyl or the like is preferably used. These compounds can be produced from the glycine-containing tripeptide molecule by a method known per se.

A prodrug of the glycine-containing tripeptide molecule or a pharmaceutically acceptable salt thereof, may also be one which is converted into the glycine-containing tripeptide molecule under a physiological condition, such as those described in IYAKUHIN no KAIHATSU (Development of Pharmaceuticals), Vol.7, Design of Molecules, p.163-198, Published by HIROKAWA SHOTEN (1990).

Exemplary glycine tripeptide molecules are three amino acid polymers which can be produced according to a peptide synthesis method described herein, and known to those of skilled in the art. The peptide synthesis method may employ currently known methods, for example, a solid phase synthesis process and a liquid phase synthesis process. That is, the object peptide for example a glycine tripeptide molecule, or a pharmaceutically acceptable salt thereof, can be produced by repeating condensation of a partial peptide or amino acid capable of constituting glycine tripeptide molecule, the peptide to be synthesized and the remaining portion (which may be constituted by two or more amino acids) according to a desired sequence. When a product having the desirable sequence has a protecting group, the object peptide can be produced by eliminating a protecting group. Examples of the condensing method and eliminating method of a protecting group to be known include methods described in the following (1)-(5).
(1) M. Bodanszky and M.A. Ondetti: Peptide Synthesis, Interscience Publishers, New York (1966)
(2) Schroeder and Luebke: The Peptide, Academic Press, New York (1965)
(3) Nobuo Izumiya, et al.: Peptide Gosei-no-Kiso to Jikken (Basics and experiments of peptide synthesis), published by Maruzen Co. (1975)
(4) Haruaki Yajima and Shunpei Sakakibara: Seikagaku Jikken Koza (Biochemical Experiment) 1, Tanpakushitsu no Kagaku (Chemistry of Proteins) IV, 205 (1977)
(5) Haruaki Yajima, ed.: Zoku lyakuhin no Kaihatsu (A sequel to Development of Pharmaceuticals), Vol. 14, Peptide Synthesis, published by Hirokawa Shoten.

### Treatment and Prevention of Metabolic, Cardiovascular and/or Systemic Inflammatory Diseases Using A Glycine Tripeptide Molecule

Glycine, or glycine containing tripeptide molecules, or their pharmaceutically acceptable salts thereof as described and exemplified herein, can be used to prevent and/or treat one or more metabolic, cardiovascular and inflammatory diseases in a subject in need thereof. For the purposes of this disclosure, "metabolic disease" refers to a wide range of diseases and disorders of the endocrine system including, for example, insulin resistance, diabetes, obesity, impaired glucose tolerance, high blood cholesterol, hyperglycemia, dyslipidemia and hyperlipidemia, and liver disease, for example, NAFLD and NASH. The metabolic diseases as described herein also include diseases that can be treated through the modulation of metabolism, although the disease itself may or may not be caused by a specific metabolic defect. Such metabolic diseases may involve, for example, glucose and fatty acid oxidation pathways.

A subject in need thereof, is a mammal, preferably a human, or a domesticated mammal or a laboratory mammal, that may be experiencing a metabolic disease, and/or a cardiovascular disease, and/or a systemic inflammatory disease, or one or more symptoms associated with these diseases.

In certain embodiments, without wishing to be bound by any particular theory, it is believed that administering glycine or the glycine tripeptide molecules, or their pharmaceutically acceptable salts thereof of the invention to a subject having a metabolic disease, cardiovascular disease and a chronic systemic inflammatory disease, or related or associated symptoms to any one of these general conditions, results in a reduction of lipid levels, including triglyceride levels, cholesterol levels, insulin resistance, glucose levels or a combination thereof. One or more of the levels can be independently reduced by 5%, 10%, 20%, 30%, 35%, or 40% or more. Administering the glycine tripeptide molecules, or their pharmaceutically acceptable salts thereof of the invention can result in improved insulin sensitivity or hepatic insulin sensitivity. Administering the glycine tripeptide molecules, or their pharmaceutically acceptable salts thereof of the invention can result in a reduction in atherosclerotic plaques, obesity, glucose, lipids, glucose resistance, cholesterol, or improvement in insulin sensitivity or any combination thereof.

Certain embodiments provide the use of glycine or a glycine tripeptide molecule, or their pharmaceutically acceptable salts thereof, as described herein in the manufacture of a medicament for treating, ameliorating, delaying or preventing one or more of a metabolic disease or a cardiovascular disease.

Certain embodiments provide a kit for treating, preventing, or ameliorating one or more of a metabolic disease or a cardiovascular disease as described herein wherein the kit comprises: a) a glycine tripeptide molecule, or a pharmaceutically acceptable salt thereof, as described herein; and optionally b) an additional second therapeutic agent or therapy as described herein. The kit can further include instructions or a label for using the kit to treat, prevent, or ameliorate one or more of a metabolic disease or a cardiovascular disease using the glycine tripeptide molecule, or a pharmaceutically acceptable salts thereof.

In some embodiments of the present invention, glycine or the glycine tripeptide molecules, or their pharmaceutically acceptable salts thereof, have potent glucose/lipid-lowering effects. In mice with established NASH, glycine tripeptide molecules, or their pharmaceutically acceptable salts thereof, for example, DT-109 is capable of reducing steatohepatitis, improve body composition, lowers circulating lipids and, normalizes or corrects liver enzymes and steatohepatitis by stimulating FAO pathways. The glycine tripeptide molecules, or their pharmaceutically acceptable salts thereof have been unexpectedly shown to lower or reduce lobular/systemic inflammation and hepatic fibrosis by inhibiting suppressing NF-κB and TGFα/SMAD pathways.

As used herein, the term "dyslipidemia" refers to abnormal lipid conditions, including hyperlipidemia, caused by aberrant lipoprotein metabolism as well as hypercholesterolemia, hypertriglyceridemia, and low HDL-cholesterolemia, due to increased levels of fat in the blood. As used herein, the term "fatty liver" refers to a condition where fat accumulates excessively in liver cells due to the disorder of lipid metabolism. It may cause various diseases such as angina, myocardial infarction, stroke, arteriosclerosis and pancreatitis. As used herein, the term "diabetes" refers to a chronic disease characterized by relative or absolute lack of insulin, leading to glucose intolerance. The term diabetes includes all kinds of diabetes, such as type 1 diabetes, type 2 diabetes and genetic diabetes. Type 1 diabetes, which is insulin-dependent diabetes, mainly results from the destruction of .beta.-cells. Type 2 diabetes, which is non-insulin-dependent diabetes, is caused by insufficient secretion of insulin after meals or insulin resistance. As used herein, the term "insulin resistance" refers to a physiological condition where insulin becomes less effective at lowering blood sugars and glucose is not effectively combusted by cells. Under high insulin resistance, the body may produce too much insulin, leading to hypertension or dyslipidemia as well as heart disease, diabetes, or the like. Especially, in type 2 diabetes, muscle and adipose tissues do not notice the increase of insulin. As used herein, the term "insulin resistance syndrome" refers to a combination of disorders caused by insulin resistance, characterized by resistance of cells against the action of insulin, hyperinsulinemia, increase of very-low-density lipoprotein (VLDL) and triglyceride, decrease of high-density lipoprotein (HDL), hypertension, or the like. It is recognized as a risk factor for cardiovascular diseases and type 2 diabetes (Reaven G M., Diabetes, 37: 1595-607 (1988)). Also, insulin resistance is known to increase oxidative stress and change the signal transduction system in cells along with other risk factors such as hypertension, diabetes, smoking, etc., thus inducing inflammatory responses and leading to atherosclerosis (Freeman B A et al., Lab. Invest. 47: 412-26 (1982), Kawamura M et al., J. Clin. Invest. 94: 771-8 (1994)).

As used herein, the term "metabolic disease" refers to a group of diseases involving disorders of metabolism which are risk factors of various cardiovascular diseases and type 2 diabetes. It includes insulin resistance and complex and diverse metabolic disorders related thereto. In 1988, Reaven proposed insulin resistance as the factor underlying these disorders and named the constellation of abnormalities insulin resistance syndrome. However, in 1998, the World Health Organization (WHO) introduced the term metabolic syndrome or metabolic disease since all the aspects of the symptoms cannot be explained by insulin resistance.

The composition of the present disclosure comprising a glycine tripeptide molecule, or their pharmaceutically acceptable salt thereof, as an active agent has the propensity to improve various metabolic diseases, and/or their symptoms, for example, obesity, diabetes, hyperlipemia, non-alcoholic fatty liver, systemic inflammation, and/or insulin resistance syndrome. The compositions of the present disclosure can prevent or treat metabolic diseases with various activities.

As used herein the term "hyperlipidemia" refers to a disease caused by higher level of blood lipids due to poor metabolism of lipids such as triglyceride and cholesterol. More specifically, hyperlipidemia is characterized by increased levels of lipids such as triglyceride, LDL cholesterol, phospholipids and free fatty acids in blood, including hypercholesterolemia and hypertriglyceridemia.

According to a preferred embodiment, the insulin resistance syndrome treated by the present invention comprises obesity, hypertension, atherosclerosis, hyperlipidemia, hyperinsulinemia, non-alcoholic fatty liver and type 2 diabetes.

According to a preferred embodiment, the composition of the present invention decreases levels of blood fat, liver fat or visceral fat. The term "liver" or "visceral" is used to encompass organ, tissue and cell.

According to the present invention, subjects fed with a diet containing glycine or a glycine tripeptide molecule, or their pharmaceutically acceptable salts thereof of the present invention significantly reduced liver weight and improved the lipid concentration of triglycerides and total cholesterol in the blood and liver tissue, and significantly reduced the total visceral fat weight.

According to a more preferred embodiment, the fat reduced by the present invention comprises triglyceride, cholesterol and free fatty acid.

According to a more preferred embodiment, the visceral fat reduced by the present invention comprises epididymal fat, perirenal fat, mesenteric fat and/or retroperitoneal fat.

According to a preferred embodiment, the composition of the present invention decreases activity of ALT (alanine aminotransferase) or AST (aspartate aminotransferase). ALT and AST as indicators for liver function are enzymes exhibiting increased levels in blood upon damage of liver.

"Nonalcoholic fatty liver disease (NAFLD) is increasingly common around the world, especially in western nations. In the United States, it is the most common form of chronic liver disease, affecting an estimated 80 to 100 million people. Nonalcoholic fatty liver disease is an umbrella term for a range of liver conditions affecting people who drink little to no alcohol. As the name implies, the main characteristic of nonalcoholic fatty liver disease is too much fat stored in liver cells. It is normal for the liver to contain some fat. However, if more than 5% - 10% percent of the liver's weight is fat, the condition is called a fatty liver (steatosis).

NAFLD is strongly associated with features of metabolic syndrome, including obesity, insulin resistance, type-2 diabetes mellitus, and dyslipidemia; it is considered the hepatic manifestation of this syndrome.

Pediatric NAFLD is currently the primary form of liver disease among children. Studies have demonstrated that abdominal obesity and insulin resistance are thought to be key contributors to the development of NAFLD. Because obesity is becoming an increasingly common problem worldwide the prevalence of NAFLD has been increasing concurrently. The only treatment shown to be truly effective in pediatric NAFLD is weight loss.

The more severe form of NAFLD is called non-alcoholic steatohepatitis (NASH). NASH causes the liver to swell and become damaged. NASH tends to develop in people who are overweight or obese, or have diabetes, high cholesterol or high triglycerides or inflammatory conditions. NASH, a potentially serious form of the disease, is marked by hepatocyte ballooning and liver inflammation, which may progress to scarring and irreversible damage. This damage is similar to the damage caused by heavy alcohol use. Macro and microscopically, NASH is characterized by lobular and/or portal inflammation, varying degrees of fibrosis, hepatocyte death and pathological angiogenesis. At its most severe, NASH can progress to cirrhosis, hepatocellular carcinoma and liver failure. Currently NAFLD and NASH are being treated e.g., by diet, treatment of insulin resistance or vitamin administration, such as vitamins E or D.

NAFLD Activity score (NAS) can be calculated according to the criteria of Kleiner (Kleiner DE. et al., Hepatology, 2005; 41:1313). NAS scores 0-2 are not considered diagnostic for NASH, NAS scores of 3-4 are considered either not diagnostic, borderline or positive for NASH, while NAS scores of 5-8 are largely considered diagnostic for NASH. A treatment effect for NASH includes the regression, stabilization or a reduction in the rate of disease progression. Sequential liver biopsies from a patient that may have NASH can be used to assess the change in the NAS score and used as an indication of the change in the disease state. A score that increases suggests progression, an unchanged score suggests stabilization, while a decreased score suggests regression of NASH. In a controlled clinical trial, the difference in NAS scores between the placebo and the test article treatment group, assessed usually over a duration of 6 months to two years, can be indicative of a treatment effect, even if both groups are progressing. A defined point spread is usually required by a regulatory authority to demonstrate a meaningful change in NASH.

The present invention also provide a method for treating a hepatic steatosis, comprising administering to a subject in need thereof, one or more tripeptide molecule or a pharmaceutically acceptable salt thereof, wherein the hepatic steatosis is treated. In one embodiment the tripeptide molecule decreased the triglyceride level in the hepatic lipids with no significant effects for the leucine negative control. In another embodiment, the tripeptide molecule decreased the total cholesterol level in the hepatic lipids with no significant effects for the leucine negative control. In any one of the above embodiments the tripeptide molecule is Gly-Gly-Leu or Gly-Gly-dLeu.

The present invention also provides a method to increase or enhance hepatic lipid oxidation, to lower triglyceride levels, or to treat cholesterol accumulation, comprising administering to a subject in need thereof, glycine or one or more glycine tripeptide molecules or a pharmaceutically acceptable salt thereof, and measuring its effect on the level of an mRNA, wherein the hepatic lipid oxidation, triglyceride level, cholesterol accumulation or any combination thereof, is enhanced or treated.

The method to enhance hepatic lipid oxidation, to lower the triglyceride level, or to treat cholesterol accumulation, comprising administering to a subject in need thereof, the glycine tripeptide Gly-Gly-Leu or Gly-Gly-dLeu or a pharmaceutically acceptable salt thereof, and measuring its effect on the level of an mRNA, wherein the hepatic lipid oxidation, triglyceride level, cholesterol accumulation or any combination thereof, is enhanced or treated. The method wherein the glycine-containing tripeptide molecule Gly-Gly-Leu or Gly-Gly-dLeu significantly induced the expression of regulators of hepatic lipid oxidation, AMPKα1 or PPARα, with no effect from the leucine negative control. The method wherein the tripeptide molecule Gly-Gly-Leu or Gly-Gly-dLeu regulated triglyceride hydrolysis by significantly upregulating CPT1a, CACT, or ACADI (mitochondrial β-oxidation) or PNPLA2. The method wherein the tripeptide molecule Gly-Gly-Leu or Gly-Gly-dLeu regulated triglyceride hydrolysis by significantly upregulating the mitochondrial anion carrier UCP2.

The method wherein the tripeptide molecule Gly-Gly-Leu or Gly-Gly-dLeu regulated cholesterol homeostasis in the liver by significantly increasing the expression of ABCG5 and ABCG8.

A method of treating the subject's plasma lipid profile, comprising administering to a subject in need thereof, one or more tripeptide molecule or a pharmaceutically acceptable salt thereof, to lower the subject's plasma triglyceride, plasma LDL level, or atherosclerotic plaque.

A method of treating the subject's plasma lipid profile, comprising administering to a subject in need thereof, the tripeptide molecule is Gly-Gly-Leu or Gly-Gly-dLeu or a pharmaceutically acceptable salt thereof, to lower the subject's plasma triglyceride, plasma LDL level, or atherosclerotic plaque. The method wherein Gly-Gly-Leu or Gly-Gly-dLeu lowers the atherosclerotic plaque. The method of treating the subject's plasma lipid profile, comprising administering to a subject in need thereof, the tripeptide molecule Gly-Gly-Leu lowers plasma total cholesterol, plasma LDL or a combination thereof.

In any of the embodiments of the methods of the invention the subject may be administered an additional lipid lowering agent. The method wherein the additional lipid lowering agent is a cholesterol absorption inhibitor, a PCSK9 inhibitor, PPAR-alpha agonists, fenofibrate, an ACC inhibitor, an ApoC-III inhibitor, an ACL-inhibitor, prescription fish oil, or a CETP inhibitor. In some embodiments method comprises administration of an additional cholesterol lowering agent is a cholesterol absorption inhibitor. The method wherein the cholesterol lowering agent is a cholesterol absorption inhibitor and the cholesterol absorption inhibitor is ezetimibe. In some embodiments the cholesterol lowering agent is a PCKS9 inhibitor.

Atherosclerosis occurs when the blood vessels that carry oxygen and nutrients from your heart to the rest of your body (arteries) become think and stiff (hardening of the arteries). Sometimes this restricts blood flow to your organs and tissues. Atherosclerosis can result in a number of complication including myocardial infarction, coronary artery disease, carotid artery disease, carotid artery disease, peripheral artery disease, aneurisms, and chronic kidney disease.

Myocardial infarction (heart attack) occurs when blood flow decreases or stops to part of the heart, causing damage to the heart muscle. Common symptoms are pain in the center or left side of the chest, shortness of breath, nausea, or it may cause heart failure, irregular heartbeat, cardiogenic shock or cardiac arrest.

Coronary artery disease is when atherosclerosis narrows the arteries close to your heart which can cause chest pain (angina), a heart attack or heart failure.

Carotid artery disease is when atherosclerosis narrows the arteries close to your brain which can cause a transient ischemic attack (TIA) or stroke. Symptoms include sudden numbness or weakness in your arms or legs, temporary loss of vision in one eye or drooping muscles in the face.

Peripheral artery disease is when atherosclerosis narrows the arteries in your arms or legs these circulation problems are call peripheral artery disease. This can make you less sensitive to heat and cold, increasing risk of burns or frostbite. In rare cases, poor circulation in arms and legs can cause tissue death (gangrene). Symptoms are leg pain when walking (claudication).

Aneurysms may occur when atherosclerosis causes a serious complication that can occur anywhere in your body. An aneurysm is a bulge in the wall of your artery which may be a medical emergency and if it bursts it can be a life-threatening event.

Chronic kidney disease can be caused by atherosclerosis when it leads to narrowing of the arteries leading to the kidneys preventing oxygenated blood from reaching them. Over time this can affect kidney function keeping waste from exiting the body. Symptoms are high blood pressure or kidney failure.

The present disclosure provides a method for treating atherosclerosis by administering to a subject in need thereof, one or more glycine tripeptide molecules, or a pharmaceutically acceptable salt thereof, wherein the administration of the glycine-containing tripeptide molecule treats the atherosclerosis. In one embodiment, the method is a method for treating atherosclerosis by administering to the subject, a glycine-containing tripeptide molecule that is Gly-Gly-Leu and/or Gly-Gly-dLeu. The present disclosure also provides for a method of treating a complication of atherosclerosis by administering to the subject with the complication, a glycine-containing tripeptide molecule to treat the myocardial infarction, coronary artery disease, carotid artery disease, carotid artery disease, peripheral artery disease, aneurisms, or chronic kidney disease. In one embodiment, the method of treating a complication of atherosclerosis by administering to the subject with the complication, a glycine-containing tripeptide molecule that is Gly-Gly-Leu or Gly-Gly-dLeu to treat the myocardial infarction, coronary artery disease, carotid artery disease, carotid artery disease, peripheral artery disease, aneurisms, or chronic kidney disease.

A method of treating inflammation in adipose tissues and in the circulation, comprising administering to a subject in need thereof, one or more glycine tripeptide molecules or a pharmaceutically acceptable salt thereof, wherein the administration of the tripeptide molecule results in less inflammation.

The method of treating inflammation in adipose tissues and in the circulation, comprising administering to a subject in need thereof, Gly-Gly-Leu and/or Gly-Gly-dLeu, or a pharmaceutically acceptable salt to the subject, wherein the inflammation in the circulation is reduced by lowering the level of plasma MCP1. A method of treating inflammation, wherein the inflammation in the adipose tissue is in the epididymal adipose tissue (EAT) or the subcutaneous adipose tissue (SAT) comprising administering to a subject in need thereof Gly-Gly-Leu and/or Gly-Gly-dLeu and the level of MCP1 mRNA is decreased.

A method of treatment of a subject for lowering plasma levels of glycine tripeptide molecule, comprising administering to a subject in need thereof, one or more tripeptide molecule or a pharmaceutically acceptable salt thereof, wherein the administration of the tripeptide molecule reduces the level of plasma glycine tripeptide molecule.

A method of treatment of a subject for lowering plasma levels of glycine tripeptide molecule, comprising administering to a subject in need thereof, the tripeptide Gly-Gly-Leu or Gly-Gly-dLeu.

A method of treating a subject to lower post-prandial glucose comprising administration to a subject in need thereof, one or more tripeptide molecule or a pharmaceutically acceptable salt thereof. The method of treating a subject to lower post-prandial glucose comprising administration to a subject in need thereof, the glycine tripeptide Gly-Gly-Leu and/or Gly-Gly-dLeu.

A method of treating a subject, comprising administering to a subject in need thereof, Gly-Gly-Leu and/or Gly-Gly-dLeu, wherein the subject has liver disease.

The method of treating a subject, comprising administering to a subject in need thereof, Gly-Gly-Leu and/or Gly-Gly-dLeu, wherein the subject has liver disease, wherein the liver disease is nonalcoholic fatty liver disease (NAFLD) or nonalcoholic steatohepatitis (NASH), or alcoholic hepatic steatosis.

A method of stabilization or reduction of the NAFDL activity score (NAS) in a subject, comprising, administering to the subject a therapeutically effective amount of a composition comprising Gly-Gly-Leu and/or Gly-Gly-dLeu, or a pharmaceutically acceptable salt thereof.

The method of stabilization or reduction of the NAFDL activity score (NAS) in a subject, comprising, administering to the subject a therapeutically effective amount of a composition comprising Gly-Gly-Leu and/or Gly-Gly-dLeu, or a pharmaceutically acceptable salt thereof, wherein the method comprises slowing the progression of, stabilizing, or reducing the steatosis component of NAS. The method that comprises slowing the progression of, stabilizing, or reducing the lobular inflammation component of NAS. The method that comprises slowing the progression of, stabilizing, or reducing the hepatocyte ballooning component of NAS.

The method according to any one of the methods of stabilization or reduction of the NAFLD activity score, wherein NAS is different by no less than 1.5 points after 6 months of treatment with a therapeutically effective amount of a composition comprising Gly-Gly-Leu and/or Gly-Gly-dLeu, or a pharmaceutically acceptable salt thereof.

A method of reducing hepatic fibrosis in a subject in need thereof, comprising administering to the subject a therapeutically effective amount of a composition comprising Gly-Gly-Leu and/or Gly-Gly-dLeu, or a pharmaceutically acceptable salt thereof.

A method for reducing plasma fibrinogen levels in a subject in need thereof, comprising administering to the subject a therapeutically effective amount of a composition comprising Gly-Gly-Leu and/or Gly-Gly-dLeu, or a pharmaceutically acceptable salt thereof.

The method for reducing plasma fibrinogen levels in a subject in need thereof, wherein the subject's fibrinogen level is greater than 300 mg/dL prior to administering to the subject a therapeutically effective amount of a composition comprising Gly-Gly-Leu and/or Gly-Gly-dLeu, or a pharmaceutically acceptable salt thereof.

One embodiment of the present invention is a kit for treating NAFLD comprising DT-109, optionally a statin, and instructions for use. Another embodiment of the present invention is a kit for treating NASH comprising DT-109, optionally a statin, and instructions for use. One embodiment of the present invention is a kit for treating NAFLD comprising DT-110, optionally a statin, and instructions for use. Another embodiment of the present invention is a kit for treating NASH comprising DT-110, optionally a statin, and instructions for use.

The present invention provides methods for reducing hepatic fibrosis in a patient comprising administering DT-109 or DT-110 or a pharmaceutically acceptable salt thereof. One embodiment is a method of reducing hepatic fibrosis in a subject in need thereof, comprising administering to the subject DT-109 or DT-110. Another embodiment is a method of reducing hepatic fibrosis in a subject in need thereof, comprising administering to the subject DT-109 or DT-110 wherein the subject has NASH.

Fibrinogen (factor I) is a mammalian glycoprotein that plays a role in the in the formation of blood clots. Fibrinogen is converted to fibrin by thrombin during blood clot formation. Fibrinogen is synthesized in liver hepatocytes. Fibrinogen therefore may be a prognostic indicator or blood marker for many disease and may also serve to effect the onset and progression of the disease state.

In various embodiments, methods for the treatment of at least one of hyperlipidemia, fatty liver, steatohepatitis, non-alcoholic fatty liver disease, non-alcoholic steatohepatitis, obesity, hyperglycemia, metabolic syndrome, cardiovascular disease, and atherosclerosis in a mammalian subject comprising administering to a subject in need thereof, a glycine tripeptide molecule, or a pharmaceutically acceptable salt thereof.

In various embodiments, the glycine-containing tripeptide molecule significantly decreases the triglyceride level in the hepatic lipids with no significant effects for the leucine negative control.

In various embodiments, the glycine-containing tripeptide molecule significantly decreases the total cholesterol level in the hepatic lipids with no significant effects for the leucine negative control.

In various embodiments, the glycine-containing tripeptide molecule is Gly-Gly-Leu, Gly-Gly-dLeu, or a pharmaceutically acceptable salt thereof.

The present disclosure provides a method to enhance hepatic lipid oxidation or utilization, to lower the triglyceride level, or treat hypercholesterolemia in a subject in need thereof. The method comprises administering to a subject in need thereof, a glycine tripeptide molecule, or a pharmaceutically acceptable salt thereof, wherein the hepatic lipid oxidation, triglyceride level, hypercholesterolemia or any combination thereof, is ameliorated as a result of treatment. In a related aspect of these embodiments, the glycine-containing tripeptide molecule is Gly-Gly-Leu, Gly-Gly-dLeu, or a pharmaceutically acceptable salt thereof.

In a related aspect of these embodiments, the glycine-containing tripeptide molecule Gly-Gly-Leu, Gly-Gly-dLeu, or a pharmaceutically acceptable salt thereof, significantly induces the expression of regulators of hepatic lipid oxidation, AMPKα1 or PPARα, with no effect from the leucine negative control.

In a related aspect of these embodiments, the glycine-containing tripeptide molecule Gly-Gly-Leu, Gly-Gly-dLeu, or a pharmaceutically acceptable salt thereof, regulates triglyceride hydrolysis by significantly upregulating CPT1a, CACT, or ACADI (mitochondrial β-oxidation) or PNPLA2.

In a related aspect of these embodiments, the glycine-containing tripeptide molecule Gly-Gly-Leu, Gly-Gly-dLeu, or a pharmaceutically acceptable salt thereof, regulates triglyceride hydrolysis by significantly upregulating the mitochondrial anion carrier UCP2.

In a related aspect of these embodiments, the glycine-containing tripeptide molecule Gly-Gly-Leu, Gly-Gly-dLeu, or a pharmaceutically acceptable salt thereof, regulates cholesterol homeostasis in the liver by significantly increasing the expression of ABCG5 and ABCG8.

In a related aspect of these embodiments, the subject's plasma lipid profile, comprising administering to a subject in need thereof, a glycine tripeptide molecule, or a pharmaceutically acceptable salt thereof, to lower the subject's plasma triglyceride, plasma LDL level, or atherosclerotic plaques. In a related aspect of these embodiments, the glycine-containing tripeptide molecule is Gly-Gly-Leu, Gly-Gly-dLeu, or a pharmaceutically acceptable salt thereof.

In a related aspect of these embodiments, the Gly-Gly-Leu, Gly-Gly-dLeu, glycine tripeptide molecules or a pharmaceutically acceptable salt thereof, lower the atherosclerotic plaques.

In a related aspect of these embodiments, the Gly-Gly-Leu, Gly-Gly-dLeu, or a pharmaceutically acceptable salt thereof, lowers plasma total cholesterol, plasma LDL or a combination thereof.

The present disclosure provides a method of treating inflammation in adipose tissues and in the circulation in a subject in need of such treatment. The method comprises administering to a subject in need thereof, a glycine tripeptide molecule, or a pharmaceutically acceptable salt thereof, wherein the administration of the glycine-containing tripeptide molecule or a pharmaceutically acceptable salt thereof results in reduced inflammation in the adipose tissues and in the circulation of the subject. In a related aspect of these embodiments, the inflammation in the circulation is reduced by lowering the level of plasma MCP1 by administration of Gly-Gly-Leu, Gly-Gly-dLeu, or a pharmaceutically acceptable salt thereof to the subject. In a related aspect of these embodiments, the inflammation in the adipose tissue is in the epididymal adipose tissue (EAT) or the subcutaneous adipose tissue (SAT) and the level of MCP1 mRNA is decreased.

The present disclosure provides a method of treating a subject to lower plasma levels of leptin in a subject in need thereof. In some embodiments, the method comprises administering to a subject in need thereof, a glycine tripeptide molecule, or a pharmaceutically acceptable salt thereof, wherein the administration of the glycine-containing tripeptide molecule reduces the level of plasma leptin. In a related aspect of these embodiments, the treatment is with Gly-Gly-Leu, Gly-Gly-dLeu, or a pharmaceutically acceptable salt thereof.

The present disclosure provides a method of treating a subject to lower post-prandial glucose in a subject in need thereof. The method comprises administering to a subject in need thereof, a glycine-containing tripeptide molecule or a pharmaceutically acceptable salt thereof.

In a related aspect of these embodiments, the treatment of the subject comprises administering a therapeutically effective amount of a glycine tripeptide molecule, e.g. Gly-Gly-Leu, Gly-Gly-dLeu, or a pharmaceutically acceptable salt thereof.

The present disclosure provides a method of treating a subject who is suffering from or likely to develop a liver disease, for example, a liver disease characterized in that the liver has excess cholesterol, triglycerides or other lipids that is illustrative of liver diseases such as NAFLD, NASH or alcoholic related steatosis of the liver, liver cirrhosis or liver inflammation. The method comprises administering to a subject in need thereof, Gly-Gly-Leu, Gly-Gly-dLeu, or a pharmaceutically acceptable salt thereof, to treat or prevent the liver disease.

In a related aspect of these embodiments, the liver disease is nonalcoholic fatty liver disease (NAFLD) or nonalcoholic steatohepatitis (NASH), or alcoholic hepatic steatosis.

In a related aspect of these embodiments, the present disclosure provides a method of stabilization or reduction of the NAFDL activity score (NAS) in a subject, wherein the method comprises administering to the subject, a therapeutically effective amount of a glycine tripeptide having an amino acid sequence of: Gly-Gly-Leu, Gly-Gly-dLeu, or a pharmaceutically acceptable salt thereof. In a related aspect of these embodiments, the the method comprises slowing the progression of, stabilizing, or reducing the steatosis component of NAS. In a related aspect of these embodiments, the method comprises slowing the progression of, stabilizing, or reducing the lobular inflammation component of NAS. In a related aspect of these embodiments, the method comprises slowing the progression of, stabilizing, or reducing the hepatocyte ballooning component of NAS. In a related aspect of these embodiments, the NAS is different by no less than 1.5 points after 6 months of treatment with Gly-Gly-Leu, Gly-Gly-dLeu, or a pharmaceutically acceptable salt thereof.

The present disclosure provides a method of reducing hepatic fibrosis in a subject in need thereof, comprising administering to the subject a therapeutically effective amount of a glycine tripeptide molecule: Gly-Gly-Leu, Gly-Gly-dLeu, or a pharmaceutically acceptable salt thereof.

The present disclosure provides a method of treating atherosclerosis, the method comprises administering to a subject in need thereof, a therapeutically effective amount of a glycine-containing tripeptide molecule or a pharmaceutically acceptable salt thereof. In a related aspect of these embodiments, the glycine-containing tripeptide molecule is Gly-Gly-Leu, Gly-Gly-dLeu, or a pharmaceutically acceptable salt thereof.

The present disclosure provides a method of treating a complication of atherosclerosis by administering to the subject with the complication, a therapeutically effective amount of a glycine-containing tripeptide molecule to treat the complication selected from the group consisting of myocardial infarction, arteriosclerosis, coronary artery disease, carotid artery disease, peripheral artery disease, atherothrombotic stroke, aneurisms, or chronic kidney disease.

The present disclosure also encompasses treatment of such exemplified metabolic diseases, cardiovascular disease and inflammatory diseases by administration of a glycine-containing tripeptide molecule in combination with a secondary therapeutic agent to treat the metabolic diseases, cardiovascular disease and inflammatory diseases. In some embodiments, the methods disclosed above further comprises administering a second therapeutic agent to the subject in need thereof selected from: a cholesterol absorption inhibitor, a PCSK9 inhibitor, PPAR-alpha agonist, an ACE inhibitor, a calcium channel blocker, an ARBs, renin, GLP-1 or a synthetic variant thereof, insulin, or a synthetic variant thereof, metformin, a sulfonyll urea compound, a thiazolidinedione (TZD), a PCSK9 inhibitor, a SGLT2 inhibitor, a DPP-IV inhibitor, an inhibitor of HMGCoA reductase, an inhibitor of proprotein convertase subtilisin/kexin type 9 (PCSK9), ezetimibe, gemfibrozil, fenofibrate, clofibrate, bezafibrate, pemafibrate, gemcabene (CI-1027), benpodoic acid (ETC-1002), an ACC inhibitor, an ApoC-III inhibitor, an ACL-inhibitor, prescription fish oil, a CETP inhibitor an anti-fibrotic agent, and combinations thereof.

Certain embodiments provide a kit for treating, preventing, or ameliorating one or more of a metabolic disease, and/or a cardiovascular disease, and/or an inflammatory disease as described herein wherein the kit comprises: a) a glycine-containing tripeptide molecule as described herein; and optionally b) an additional agent or therapy as described herein. The kit can further include instructions or a label for using the kit to treat, prevent, or ameliorate one or more of a metabolic disease, and/or a cardiovascular disease, and/or an inflammatory disease. In a related aspect of these embodiments, the kit is for treating a subject with NAFLD or with NASH comprising a selected tripeptide, optionally a statin, and instructions for use. In a related aspect of these embodiments, the kit comprises DT-109 (Gly-Gly-Leu) and/or (DT-110 (Gly-Gly-dLeu), optionally a statin, and instructions for use. In a related aspect of these embodiments, the kit further optionally comprises a cholesterol absorption inhibitor, a PCSK9 inhibitor, PPAR-alpha agonists, an ACE inhibitor, a calcium channel blocker, an ARBs, renin, GLP-1 or a synthetic variant thereof, insulin, or a synthetic variant thereof, metformin, a sulfonyll urea compound, a thiazolidinedione (TZD), a PCSK9 inhibitor, a SGLT2 inhibitor, a DPP-IV inhibitor, a statin, an inhibitor of HMGCoA reductase, (proprotein convertase subtilisin/kexin type 9), ezetimibe, gemfibrozil, fenofibrate, clofibrate, bezafibrate, pemafibrate, gemcabene, (CI-1027), benpodoic acid (ETC-1002), an ACC inhibitor, an ApoC-III inhibitor, an ACL-inhibitor, prescription fish oil, a CETP inhibitor an anti-fibrotic agent, and combinations thereof. In a related aspect of these embodiments, the kit optionally comprises ezetimibe.

### Formulations

As used herein, the term "pharmaceutically acceptable" means approved by a regulatory agent of the Federal or state government, or listed in the U.S. Pharmacopeia or other generally recognized pharmacopeia for use in animals, such as humans. The term "carrier" refers to a diluent, adjuvant, excipient, stabilizer, or vehicle with which the agent is formulated for administration. Pharmaceutical carriers can be sterile liquids, such as water and oils, including those of petroleum, animal, vegetable or synthetic origin, such as peanut oil, soybean oil, mineral oil, sesame oil, and the like. Water is a typical carrier when the pharmaceutical composition is administered intravenously. Saline solutions and aqueous dextrose and glycerol solutions can be employed as liquid carriers, particularly for injectable solutions. Suitable pharmaceutical excipients include starch, glucose, lactose, sucrose, gelatin, malt, rich, flour, chalk, silica gel, sodium stearate, glycerol monostearate, talc, sodium chloride, dried skim milk, glycerol, propylene, glycol, water, ethanol, and the like. The composition, if desired, can also contain minor amounts of wetting or emulsifying agents, or pH buffering agents. Pharmaceutical compositions can take the form of solutions, suspensions, emulsions, tablets, pills, capsules, powders, sustained-release formulations, and the like. The composition can also be formulated as a suppository, with traditional binders and carriers such as triglycerides.

The pharmaceutical composition of the present invention comprising a glycine tripeptide molecule, or a pharmaceutically acceptable salt thereof, in admixture with at least one pharmaceutically acceptable excipient, carrier, or diluent. The pharmaceutically acceptable composition contains one or more formulation materials for modifying, maintaining or preserving, for example, the pH, osmolarity, viscosity, clarity, color, isotonicity, odor, sterility, stability, rate of dissolution or release, adsorption or penetration of the composition. Suitable formulation materials include, but are not limited to, amino acids (such as glutamine, asparagine, arginine or lysine); antimicrobials; antioxidants (such as ascorbic acid, sodium sulfite or sodium hydrogen sulfite); buffers (such as borate, bicarbonate, Tris HCl, citrates, phosphates, other organic acids); bulking agents (such as mannitol or glycine), chelating agents (such as ethylenediamine tetraacetic acid (EDTA)); complexing agents (such as caffeine, polyvinylpyrrolidone, beta cyclodextrin or hydroxypropyl beta cyclodextrin); fillers; monosaccharides; disaccharides and other carbohydrates (such as glucose, mannose, or dextrins); proteins (such as serum albumin, gelatin or immunoglobulins); coloring; flavoring and diluting agents; emulsifying agents; hydrophilic polymers (such as polyvinylpyrrolidone); low molecular weight polypeptides; salt forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate 80, triton, tromethamine, lecithin, cholesterol, tyloxapal); stability enhancing agents (sucrose or sorbitol); tonicity enhancing agents (such as alkali metal halides (in one aspect, sodium or potassium chloride, mannitol sorbitol); delivery vehicles; diluents; excipients and/or pharmaceutical adjuvants. (Remington's Pharmaceutical Sciences, 18th Edition, A. R. Gennaro, ed., Mack Publishing Company, 1990).

In one aspect of the invention, glycine-containing tripeptide molecule is supplied as a single-use only glass vial containing a lyophilized cake, prepared in a formulation buffer consisting of 10 mM glutamic acid, 2% glycine, 1% sucrose, and 0.01% polysorbate 20 to pH 4.25. Upon reconstitution with a volume of sterile diluent, for example, sterile isotonic saline or water, for example, 0.5 mL to about 10 mL, for example, 2.2 mL of sterile water, the cake yields a 1 g/mL to about 100 g/mL concentration of glycine tripeptide molecule. Glycine-containing tripeptide molecule is stored in a secure location under controlled conditions. Once the single-use vial has been reconstituted, the drug is administered immediately (no more than three hours after reconstitution). Before injection, study medication is allowed to reach room temperature (15 to 30°C).

### Dosages and Administration

A "dose amount" as used herein, is generally equal to the dosage of the active ingredient which may be administered once per day, or may be administered several times a day (e.g. the unit dose is a fraction of the desired daily dose). In various embodiments, the various doses and dosage regimen apply to glycine or the glycine-containing tripeptide molecules disclosed herein. For example, dosages that are useful in the present invention are from 5 mg/Kg to 2,000 mg/Kg, or from 30 mg/Kg to 1,500 mg/Kg, or from 40 mg/Kg to 1,250 mg/Kg, or from 50 mg/Kg to 1,000 mg/Kg, or from 60 mg/Kg to 5,000 mg/Kg calculated on the subject's bodyweight. The term "unit dose" as used herein may be taken to indicate a discrete amount of the therapeutic composition which comprises a predetermined amount of the active compound. The amount of the active ingredient is generally equal to the dosage of the active ingredient which may be administered once per day, or may be administered several times a day (e.g. the unit dose is a fraction of the desired daily dose). The unit dose may also be taken to indicate the total daily dose, which may be administered once per day or may be administered as a convenient fraction of such a dose (e.g. the unit dose is the total daily dose which may be given in fractional increments, such as, for example, one-half or one-third the dosage).

As used herein, the term "starting daily dose amount" refers to the amount of glycine-containing tripeptide molecule per day that is administered or prescribed to a patient beginning glycine-containing tripeptide molecule treatment, who has not previously been subjected to a titration regimen of glycine tripeptide molecule. This amount can be administered in multiple unit doses or in a single unit dose, in a single time during the day or at multiple times during the day.

In some embodiments, the amount of glycine-containing tripeptide molecule may be from about 1 mg/kg/day to about 10,000 mg/kg/day, from about 1 mg/kg/day to about 1,000 mg/kg/day, from about 0.1 mg/kg/day to about 1,000 mg/kg/day, from about I mg/kg/day to about 1,000 mg/kg/day, from about 1,000 mg/kg/day to about 10,000 mg/kg/day, or from about 1 mg/kg/day to about 500 mg/kg/day. In some embodiments, the amount of glycine-containing tripeptide molecule may be from about 3 mg/kg/day to about 70 mg/kg/day. In some embodiments, amount of glycine-containing tripeptide molecule may be from about 7 mg/kg/day to about 40 mg/kg/day. In some embodiment, the amount of glycine-containing tripeptide molecule may be from about 3 mg/kg/day to about 50 mg/kg/day.

In some embodiments, the dosage may be 1000 mg/day to 100 g/day, more preferably 1000 mg/day to 75 g/day. The amount of glycine-containing tripeptide molecule in the compositions may preferably be about 50 mg to about 100 g, about 100 mg to about 90 g, from about 500 mg to about 75g, from about 600 mg to about 80 g, from about 700 mg to about 75 g. In some embodiments, the amount of glycine-containing tripeptide molecule in the compositions may be about from about 100 mg to about 1,000 g, from about 200 mg to about 500 g, from about 300 mg to about 100 g, from about 400 mg to about 75 g, from about 500 mg to about 50 g, from about 750 mg to about 25 g, from about 1,000 mg to about 50g, or from 1000 mg to about 25 g.

In some embodiments, the therapeutically effective dose amount of glycine-containing tripeptide molecule to be administered is from about 100 mg to about 200 g. This dose may be administered as a single daily dose, or may be divided into several doses administered throughout the day, for example, 1 to 5 doses, preferably two or three doses per day. In some embodiments, the amount of glycine-containing tripeptide molecule is from about 250 mg to about 100 g. In some embodiments, the amount of glycine-containing tripeptide molecule is from about 500 mg to about 90 g. In some embodiments, the amount of glycine-containing tripeptide molecule is from about 750 mg to about 75 g. In some embodiments, the amount of glycine-containing tripeptide molecule is from about 1000 mg to about 50 g. In some embodiments, the composition is suitable for oral administration. In some embodiments, the composition is a solid oral dosage form.

The composition may have a chiral purity for glycine-containing tripeptide molecule of at least 99.5%, preferably at least 99.6%, preferably at least 99.7%, preferably at least 99.8%, preferably at least 99.9%, preferably at least 99.95%, or more preferably at least 99.99%. In some embodiments, the chiral purity for glycine-containing tripeptide molecule is 100%. In some embodiments, the composition has a chiral purity for glycine-containing tripeptide molecule of 99.9% or greater. In some embodiments, the composition has a chiral purity for glycine-containing tripeptide molecule of 99.95% or greater. In some embodiments, the composition has a chiral purity for glycine-containing tripeptide molecule of 99.99% or greater.

In some embodiments, the composition is suitable for oral administration. In some embodiments, the composition is a solid oral dosage form. In some embodiments, the composition is a capsule. In some embodiments, the composition is a tablet. In some embodiments, the composition is formulated as an oral or parenteral solution.

The embodiments for amounts of glycine-containing tripeptide molecule in the composition, chiral purity, and dosage form, which are described herein separately for the sake of brevity, can be joined in any suitable combination.

In another aspect, the present invention relates to compositions comprising glycine-containing tripeptide molecule which is chirally pure for glycine tripeptide molecule. In some embodiments, the amount of glycine-containing tripeptide molecule may be from about 5 mg/Kg/day to 5000 mg/Kg/day, or from 5 mg/Kg/day to about 2,000 mg/Kg/day, or from 30 mg/Kg/day to 1500 mg/Kg/day, or from 40 mg/Kg/day to 1250 mg/Kg/day, or from 50 mg/Kg/day to 1000 mg/Kg/day, or from 60 mg/Kg/day to 500 mg/Kg/day calculated on the subject's bodyweight.. In some embodiments, the amount of glycine-containing tripeptide molecule may be from about 10 mg/kg/day to about 1000 mg/kg/day. In some embodiments, the amount of glycine-containing tripeptide molecule may be from about 7 mg/kg/day to about 900 mg/kg/day. In some embodiment, the amount of glycine-containing tripeptide molecule may be from about 5 mg/kg/day to about 800 mg/kg/day. In some embodiments, the dosage may be 10 mg/day to 5,000 mg/day, more preferably 100 mg/day to 2000 mg/day. In some embodiments, the compositions are administered in doses of from about 500 mg to about 100 g, from about 1,000 mg to about 75 g, from about 1500 mg to about 50 g, or from about 2000 mg to about 25 g of glycine tripeptide molecule. In some embodiments, the compositions are administered in doses of from about 250 mg to about 500 g, from about 500 mg to about 250 g, from about 750 mg to about 200 g, from about 1,000 mg to about 100 g, from about 1,250 mg to about 75g, from about 1,500 mg to about 50 g, from about 2,000 mg to about 25 g, or from 2,500 mg to about 20g. In some embodiments, the effective amount of a glycine-containing tripeptide molecule dosed to a subject in need thereof, to prevent or treat a metabolic disease, and/or a cardiovascular disease, and/or an inflammatory disease may range from about 500 mg to about 200 g. This dose may be administered as a single daily dose, or may be divided into several doses administered throughout the day, for example, 1 to 5 doses per day, preferably two to three doses per day. These doses of glycine-containing tripeptide molecule preferably are in preparations which have a chemical purity of 97% or greater and a chiral purity for glycine tripeptide molecule, of 99.6% or greater, 99.7% or greater, 99.8% or greater, 99.9% or greater, preferably 99.95% or greater and more preferably 99.99% or greater. In a preferred embodiment, the compositions comprising glycine-containing tripeptide molecule may have a chiral purity for glycine-containing tripeptide molecule of 100%. The compositions may further comprise a carrier. The compositions of the present invention may be administered orally, preferably as a solid oral dose, and more preferably as a solid oral dose that may be a capsule or tablet. In preferred embodiments, the compositions of the present invention may be formulated as tablets for oral administration.

In another aspect, the present invention further provides a composition comprising a therapeutically effective amount of glycine tripeptide molecule. The composition may further comprise a pharmaceutically acceptable carrier.

In some embodiments, the therapeutically effective amount of glycine-containing tripeptide molecule may be from about 1 mg/kg/day to about 10,000 mg/kg/day, from about 5 mg/kg/day to about 5,000 mg/kg/day, from about 20 mg/kg/day to about 1,000 mg/kg/day, from about 30 mg/kg/day to about 1,000 mg/kg/day, from about 50 mg/kg/day to about 10,000 mg/kg/day, or from about 100 mg/kg/day to about 5,000 mg/kg/day.

In some embodiments, the therapeutically effective amount of glycine-containing tripeptide molecule may be from about 5 mg/kg/day to about 5,000 mg/kg/day. In some embodiments, the therapeutically effective amount of glycine-containing tripeptide molecule may be from about 10 mg/kg/day to about 4,000 mg/kg/day. In some embodiment, the therapeutically effective amount of glycine-containing tripeptide molecule may be from about 25 mg/kg/day to about 2,000 mg/kg/day. In some embodiments, the dosage may be 10 mg/day to 1,000 g/day, more preferably 500 mg/day to 100 g/day. The therapeutically effective amount of glycine-containing tripeptide molecule in the compositions may preferably be about 250 mg to about 500 g, from about 500 mg to about 400g, from about 750 mg to about 200g, from about 1,000 mg to about 100g. In some embodiments, the therapeutically effective amount of glycine-containing tripeptide molecule in the compositions may be about from about 300 mg to about 1,000g, from about 500 mg to about 500g, from about 600 mg to about 400g, from about 700 mg to about 300g, from about 800 mg to about 200g, from about 900 mg to about 150g, or from about 1,000 mg to about 100g. In some embodiments, the amount of glycine-containing tripeptide molecule is from about 600 mg to about 300g. This dose may be administered as a single daily dose, or may be divided into several doses administered throughout the day, for example, 1 to 5 doses per day, preferably two to three doses per day. In some embodiments, the therapeutically effective amount of glycine-containing tripeptide molecule is from about 500 mg to about 350g. In some embodiments, the therapeutically effective amount of glycine-containing tripeptide molecule is from about 750 mg to about 250g. In some embodiments, the therapeutically effective amount of glycine-containing tripeptide molecule is from about 1,000 mg to about 150g. In some embodiments, the therapeutically effective amount of glycine-containing tripeptide molecule is from about 1,500 mg to about 100g. In some embodiments, the composition is suitable for oral administration. In some embodiments, the composition is a solid oral dosage form. In some embodiments, the composition is a liquid oral dosage form. In some embodiments, the composition is a liquid parenteral dosage form.

In some embodiments, the composition is suitable for oral administration. In some embodiments, the composition is a solid oral dosage form. In some embodiments, the composition is a capsule. In some embodiments, the composition is a tablet.

In another aspect, the beneficial effect of the glycine tripeptide molecule, or a pharmaceutically acceptable salt thereof, on a subject's liver function is, in one aspect, seen by favorable changes in messenger RNA or protein expression of hepatic lipid oxidation, AMPKα1, glucokinase, peroxisomal proliferator activated receptor-a, peroxisomal proliferator activated receptor-γ, PPARγ coactivator 1, pyruvate kinase, sterol regulatory element binding protein-1c, long chain and very long chain acyl-CoA dehydrogenase or stearoyl-CoA desaturase. In a further aspect, the beneficial effect of glycine tripeptide molecule, or a pharmaceutically acceptable salt thereof, on a subject's liver function is seen by an improvement in messenger RNA or protein expression of phosphoenoyl pyruvate kinase, microsomal transfer protein, arylacetaminde deacetylase, apolipoprotein C2, carnitine palmitoyl transferase II, or phospholipase D1.

The glycine tripeptide molecule, or a pharmaceutically acceptable salt thereof, of the present invention may be administered by any suitable route. For example, compositions of the invention can be administered by the oral, ocular, intradermal, intraperitoneal, intranasal, subcutaneous, intramuscular or intravenous route.

Formulations suitable for oral administration include, for example, solid, semi-solid and liquid systems such as, tablets; soft or hard capsules containing multi- or nano-particulates, liquids, or powders; lozenges (including liquid-filled); chews; gels; fast dispersing dosage forms; films; ovules; sprays. In some embodiments the peptides of the present invention are formulated for oral administration using delivery vehicles known in the art, including but not limited to, microspheres, liposomes, enteric coated dry emulsions or nanoparticles.

Liquid dosage forms for oral administration include, but are not limited to, pharmaceutically acceptable emulsions, microemulsions, solutions, suspensions, syrups and elixirs. In addition to the active peptides, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Solid dosage forms for oral administration include capsules, tablets, pills, powders, and granules. In such solid dosage forms, the active peptide is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. The active compounds can also be in microencapsulated form with one or more excipients as noted above. Solid compositions of a similar type may also be employed as fillers in soft and hard-filled gelatin capsules using such excipients as lactose or milk sugar as well as high molecular weight polyethylene glycols, poloxamers and the like. The solid dosage forms of tablets, dragees, capsules, pills, and granules can be prepared with coatings and shells such as enteric coatings and other coatings well known in the pharmaceutical formulating art. Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

Those of skill in the art will understand that the amounts of glycine-containing tripeptide molecule polypeptides administered for therapeutic use vary. In one aspect, the glycine tripeptide molecule, or a pharmaceutically acceptable salt thereof, containing composition is substantially free of contaminating factors, contamination level of less than 0.02% (w/w). Glycine-containing tripeptide molecule compositions, suitable for injection into a patient, are prepared, for example, by reconstitution with a pharmacologically acceptable diluent of a lyophilized sample comprising purified glycine-containing tripeptide molecule and stabilizing salts. Administration of the glycine-containing tripeptide molecule composition is alternatively systemic or local as discussed herein below in detail, and comprise administration of a therapeutically-effective amount of the glycine-containing tripeptide molecule protein composition.

### Combination Treatments and Compositions

In addition to therapies based solely on the delivery of the glycine tripeptide molecule, or a pharmaceutically acceptable salt thereof, containing composition, a combination therapy is specifically contemplated. In the context of the invention, it is contemplated that the glycine-containing tripeptide molecule therapy is used similarly in conjunction with other agents commonly used for the treatment of a metabolic disease, for example, a fatty liver disease.

In various embodiments, the second therapeutic agent(s) can be combined with the glycine tripeptide molecule, in particular for a synergistic enhancement of activity. Administration of the active ingredient combination can take place either by separate administration of the active ingredients to the patient or in the form of combination products in which a plurality of active ingredients are present in one pharmaceutical preparation. It is expected that the secondary therapeutic agent can be dosed at its approved dosage when used as a single agent, or initially, the secondary therapeutic agent may be dosed at therapeutically effective concentrations or at sub-therapeutically effective concentrations, such that the combination of the secondary therapeutic agent with the glycine tripeptide molecule, or pharmaceutically acceptable salt thereof, when administered in combination, provides a therapeutically effective result in the patient being treated.

To achieve the appropriate therapeutic outcome, using the methods and compositions of the present invention, one may provide a composition comprising glycine-containing tripeptide molecule and at least one other therapeutic agent or agents (second therapeutic agent(s)). In the invention, it is contemplated that the second therapeutic agent may be selected from pramlinitide, peptide YY (PYY), exanatide, or an insulin sensitizer including, but not limited to, a thiazolidinedione or metformin, or glimepiride, and analogues of any of these compounds. Other secondary therapeutic agents used for the control of dyslipidemia, diabetes and cardiovascular diseases associated with atherosclerosis are well known in the art and may be used in combination with a glycine-containing tripeptide molecule of the present invention.

The combination therapy compositions is provided in a combined amount effective to produce the desired therapeutic outcome in the treatment of a metabolic disease, for example, a metabolic disease associated with one or more of: aberrant glucose metabolism, dyslipidemia, for example, dyslipidemia associated with increases in cholesterol (pure or isolated hypercholesterolemia), and/or increases in triglycerides (TGs) only (pure or isolated hypertriglyceridemia), and/or increases in both cholesterol and TGs (mixed or combined hyperlipidemias), fatty liver disease, including NAFLD, NASH, steatosis, liver inflammation, peripheral artery disease, coronary artery disease, atherosclerosis, systemic inflammation, and ischemic stroke associated with plaque related thrombosis. This process involves administering to the subject in need thereof, a therapeutically effective amount of a first therapeutic agent of the present invention (i.e. a composition comprising a therapeutically effective amount of a glycine tripeptide molecule, or a pharmaceutically acceptable salt thereof), and optionally, a second therapeutic agent(s) or factor(s) at the same time. This is achieved by administering a single composition or pharmacological formulation that includes both therapeutic agents, or by administering two distinct compositions or formulations, at the same time, wherein one composition includes the glycine-containing tripeptide molecule therapeutic composition and the other includes the second therapeutic agent.

Antidiabetics useful as a secondary therapeutic agent used in combination with a glycine-containing tripeptide molecule may include insulin and insulin derivatives such as, for example, Lantus^{®} or HMR 1964 or Levemir^{®} (insulin detemir) or those described in WO2005005477 (Novo Nordisk), fast-acting insulins (see U.S. Pat. No. 6,221,633), inhalable insulins such as, for example, Exubera^{®} or oral insulins such as, for example, IN-105 (Nobex) or Oral-lyn ^{™} (Generex Biotechnology), GLP-1 derivatives and GLP-1 agonists such as, for example, exenatide, liraglutide or those which have been disclosed in WO98/08871, WO2005027978, WO2006037811 or WO2006037810 of Novo Nordisk A/S, in WO01/04156 of Zealand or in WO00/34331 of Beaufour-Ipsen, pramlintide acetate (Symlin; Amylin Pharmaceuticals), BIM-51077, PC-DAC-exendin-4 (an exendin-4 analog covalently bonded to recombinant human albumin), agonists like those described for example in D. Chen et al., Proc. Natl. Acad. Sci. USA 104 (2007) 943, those as are described in WO2006124529, and orally effective hypoglycemic active ingredients.

Antidiabetics also include agonists of the glucose-dependent insulinotropic polypeptide (GIP) receptor as are described for example in WO2006121860 and GLP-1 and GLP-2 and their synthetic variants and mutants used to control blood glucose and treat diabetes, including type II diabetes mellitus.

Exemplary secondary therapeutic agents useful in combination with a glycine-containing tripeptide molecule disclosed herein may include orally effective hypoglycemic active ingredients, preferably sulfonylureas, biguanidines, meglitinides, oxadiazolidinediones, thiazolidinediones, glucosidase inhibitors, inhibitors of glycogen phosphorylase, glucagon antagonists, glucokinase activators, inhibitors of fructose-1,6-bisphosphatase, modulators of glucose transporter 4 (GLUT4), inhibitors of glutamine-fructose-6-phosphate amidotransferase (GFAT), GLP-1 agonists, potassium channel openers such as, for example, pinacidil, cromakalim, diazoxide or those described in R. D. Carr et al., Diabetes 52, 2003, 2513.2518, in J. B. Hansen et al., Current Medicinal Chemistry 11, 2004, 1595-1615, in T. M. Tagmose et al., J. Med. Chem. 47, 2004, 3202-3211 or in M. J. Coghlan et al., J. Med. Chem. 44, 2001, 1627-1653, or those which have been disclosed in WO 97/26265 and WO 99/03861 of Novo Nordisk A/S, inhibitors of dipeptidylpeptidase IV (DPP-IV), insulin sensitizers, inhibitors of liver enzymes involved in stimulating gluconeogenesis and/or glycogenolysis, modulators of glucose uptake, of glucose transport and of glucose reabsorption, inhibitors of 11.beta.-HSD1, inhibitors of protein tyrosine phosphatase 1B (PTP1B), modulators of the sodium-dependent glucose transporter 1 or 2 (SGLT1, SGLT2), compounds which alter lipid metabolism such as anti hyperlipidemic active ingredients and antilipidemic active ingredients, compounds which reduce food intake, compounds which increase thermogenesis, PPAR and RXR modulators and active ingredients which act on the ATP-dependent potassium channel of the beta cells.

In one embodiment of the invention, glycine-containing tripeptide molecule is administered in combination with an HMGCoA reductase inhibitor such as simvastatin, fluvastatin, pravastatin, lovastatin, atorvastatin, cerivastatin, rosuvastatin or L-659699.

In one embodiment of the invention, a glycine-containing tripeptide molecule is administered in combination with a cholesterol absorption inhibitor such as, for example, ezetimibe, tiqueside, pamaqueside, FM-VP4 (sitostanol/campesterol ascorbyl phosphate; Forbes Medi-Tech, WO2005042692, WO2005005453), MD-0727 (Microbia Inc., WO2005021497, WO2005021495) or with compounds as described in WO2002066464, WO2005000353 (Kotobuki Pharmaceutical Co. Ltd.), or WO2005044256 or WO2005062824 (Merck & Co.) or WO2005061451 and WO2005061452 (AstraZeneca AB), and WO2006017257 (Phenomix) or WO2005033100 (Lipideon Biotechnology AG), or as described in WO2004097655, WO2004000805, WO2004000804, WO2004000803, WO2002050068, WO2002050060, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163.

In one embodiment of the invention, a glycine-containing tripeptide molecule is administered in combination with Vytorin^{™}, a fixed combination of ezetimibe and simvastatin.

In one embodiment of the invention, a glycine-containing tripeptide molecule is administered in combination with a fixed combination of ezetimibe with atorvastatin.

In one embodiment of the invention, a glycine-containing tripeptide molecule is administered in combination with a fixed combination of ezetimibe with fenofibrate.

In a further embodiment of the invention, a glycine-containing tripeptide molecule is administered in combination with a fixed combination of fenofibrate and rosuvastatin.

In one embodiment of the invention, a glycine-containing tripeptide molecule is administered in combination with Synordia^{®}, a fixed combination of fenofibrate with metformin.

In one embodiment of the invention, a glycine-containing tripeptide molecule is administered in combination with ISIS-301012, an antisense oligonucleotide able to regulate the apolipoprotein B gene.

In one embodiment of the invention, a glycine-containing tripeptide molecule is administered in combination with a PPAR gamma agonist such as, for example, rosiglitazone, pioglitazone, JTT-501, GI 262570, R-483, CS-011 (rivoglitazone).

In one embodiment of the invention, a glycine-containing tripeptide molecule is administered in combination with Competact^{™}, a fixed combination of pioglitazone hydrochloride with metformin hydrochloride.

In one embodiment of the invention, a glycine-containing tripeptide molecule is administered in combination with Tandemact^{™}, a fixed combination of pioglitazone with glimepiride.

In one embodiment of the invention, a glycine-containing tripeptide molecule is administered in combination with a fixed combination of pioglitazone hydrochloride with an angiotensin II receptor antagonist such as, for example, TAK-536.

In one embodiment of the invention, a glycine-containing tripeptide molecule is administered in combination with a PPAR alpha agonist such as, for example, GW9578, GW-590735, K-111, LY-674, KRP-101, DRF-10945, LY-518674 or those as are described in WO2001040207, WO2002096894, WO2005097076.

In one embodiment of the invention, a glycine-containing tripeptide molecule is administered in combination with a mixed PPAR alpha/gamma agonist such as, for example, naveglitazar, LY-510929, ONO-5129, E-3030, AVE 8042, AVE 8134, AVE 0847, CKD-501 (lobeglitazone sulfate) or as described in WO 00/64888, WO 00/64876, WO03/020269 or in J. P. Berger et al., TRENDS in Pharmacological Sciences 28(5), 244-251, 2005.

In one embodiment of the invention, a glycine-containing tripeptide molecule is administered in combination with a PPAR delta agonist such as, for example, GW-501516 or as described in WO2006059744, WO2006084176, WO2006029699, WO2007039172-WO2007039178.

In one embodiment, a glycine-containing tripeptide molecule is administered in combination with metaglidasen or with MBX-2044 or other partial PPAR gamma agonists/antagonists.

In one embodiment of the invention, a glycine-containing tripeptide molecule is administered in combination with a fibrate such as, for example, fenofibrate, clofibrate or bezafibrate.

In one embodiment of the invention, a glycine-containing tripeptide molecule is administered in combination with an MTP inhibitor such as, for example, implitapide, BMS-201038, R-103757, AS-1552133 or those described in WO2005085226, WO2005121091, WO2006010423.

In one embodiment of the invention, a glycine-containing tripeptide molecule is administered in combination with a CETP inhibitor such as, for example, torcetrapib or JTT-705 or those described in WO2006002342, WO2006010422, WO2006012093, WO2006073973, WO2006072362, WO2006097169, WO2007041494.

In one embodiment of the invention, a glycine-containing tripeptide molecule is administered in combination with a bile acid absorption inhibitor (see, for example, U.S. Pat. No. 6,245,744, U.S. Pat. No. 6,221,897 or WO00/61568), such as, for example, HMR 1741 or those as described in DE 10 2005 033099.1 and DE 10 2005 033100.9, WO2007009655-56.

In one embodiment of the invention, a glycine-containing tripeptide molecule is administered in combination with a polymeric bile acid adsorbent such as, for example, cholestyramine or colesevelam.

In one embodiment of the invention, a glycine-containing tripeptide molecule is administered in combination with an LDL receptor inducer (see U.S. Pat. No. 6,342,512), such as, for example, HMR1171, HMR1586 or those as described in WO2005097738.

In one embodiment of the invention, a glycine-containing tripeptide molecule is administered in combination with an ABCA1 expression enhancer as described for example in WO2006072393.

In a further embodiment of the invention, a glycine-containing tripeptide molecule is administered in combination with an and inhibitor of PCSK9 (proprotein convertase subtilisin/kexin type 9), for example, Evolocumab, inclisiran, or a RNAi therapeutic directed against PCSK9. In a further embodiment of the invention, glycine-containing tripeptide molecule is administered in combination with an antibody directed against PCSK9, for example, Alirocumab (Praluent), which may be dosed 75-150 mg every two weeks; and Evolocumab (Repatha), dosed 140 mg every two weeks or 420 mg monthly.

In one embodiment, a glycine-containing tripeptide molecule is administered in combination with Omacor^{®} (omega-3 fatty acids; highly concentrated ethyl esters of eicosapentaenoic acid and of docosahexaenoic acid).

In one embodiment of the invention, a glycine-containing tripeptide molecule is administered in combination with an ACAT inhibitor such as, for example, avasimibe or SMP-797.

In one embodiment of the invention, glycine-containing tripeptide molecule is administered in combination with an antioxidant such as, for example, OPC-14117, probucol, tocopherol, ascorbic acid, .beta.-carotene or selenium.

In one embodiment of the invention, a glycine-containing tripeptide molecule is administered in combination with a vitamin such as, for example, vitamin B6 or vitamin B12.

In one embodiment of the invention, a glycine-containing tripeptide molecule is administered in combination with a lipoprotein lipase modulator such as, for example, ibrolipim (NO-1886).

In one embodiment of the invention, a glycine-containing tripeptide molecule is administered in combination with an ATP citrate lyase inhibitor such as, for example, SB-204990.

In one embodiment of the invention, a glycine-containing tripeptide molecule is administered in combination with a squalene synthetase inhibitor such as, for example, BMS-188494, TAK-475 or as described in WO2005077907, JP2007022943.

In one embodiment of the invention, a glycine-containing tripeptide molecule is administered in combination with a lipoprotein(a) antagonist such as, for example, gemcabene (CI-1027).

In one embodiment of the invention, glycine-containing tripeptide molecule is administered in combination with an agonist of GPR109A (HM74A receptor agonist; NAR agonist (nicotinic acid receptor agonist) such as, for example, nicotinic acid or extended release niacin in conjunction with MK-0524A or those compounds described in WO2006045565, WO2006045564, WO2006069242, WO2006124490, WO2006113150, WO2007017261, WO2007017262, WO2007017265, WO2007015744, WO2007027532.

In another embodiment of the invention, a glycine-containing tripeptide molecule is administered in combination with an agonist of GPR116 as are described for example in WO2006067531, WO2006067532.

In one embodiment of the invention, a glycine-containing tripeptide molecule is administered in combination with a lipase inhibitor such as, for example, orlistat or cetilistat (ATL-962).

In one embodiment of the invention, a glycine-containing tripeptide molecule is administered in combination with insulin.

In one embodiment, a glycine-containing tripeptide molecule is administered in combination with a sulfonylurea such as, for example, tolbutamide, glibenclamide, glipizide, gliclazide or glimepiride.

In one embodiment, glycine-containing tripeptide molecule is administered in combination with a substance which enhances insulin secretion, such as, for example, KCP-265 (WO2003097064) or those described in WO2007026761.

In one embodiment, a glycine-containing tripeptide molecule is administered in combination with agonists of the glucose-dependent insulinotropic receptor (GDIR) such as, for example, APD-668.

In one embodiment, a glycine-containing tripeptide molecule is administered in combination with a biguanide such as, for example, metformin.

In yet another embodiment, a glycine-containing tripeptide molecule is administered in combination with a meglitinide such as, for example, repaglinide, nateglinide or mitiglinide
In a further embodiment, a glycine-containing tripeptide molecule is administered with a combination of mitiglinide with a glitazone, e.g. pioglitazone hydrochloride, or rosiglitazone maleate, or combinations of a glitazone with glimepiride, or metformin, or combinations thereof.

In a further embodiment, a glycine-containing tripeptide molecule is administered with a combination of mitiglinide with an alpha-glucosidase inhibitor.

In one embodiment, a glycine-containing tripeptide molecule is administered in combination with a thiazolidinedione such as, for example, troglitazone, ciglitazone, pioglitazone, rosiglitazone or the compounds disclosed in WO 97/41097 of Dr. Reddy's Research Foundation, in particular 5-[[4-[(3,4-dihydro-3-methyl-4-oxo-2-quinazolinylmethoxy]-phenyl]methyl]-- 2,4-thiazolidinedione.

In one embodiment, a glycine-containing tripeptide molecule is administered in combination with an .alpha.-glucosidase inhibitor such as, for example, miglitol or acarbose.

In one embodiment, a glycine-containing tripeptide molecule is administered in combination with an active ingredient which acts on the ATP-dependent potassium channel of the beta cells, such as, for example, tolbutamide, glibenclamide, glipizide, glimepiride or repaglinide.

In one embodiment, a glycine-containing tripeptide molecule is administered in combination with more than one of the aforementioned compounds, e.g. in combination with a sulfonylurea and metformin, a sulfonylurea and acarbose, repaglinide and metformin, insulin and a sulfonylurea, insulin and metformin, insulin and troglitazone, insulin and lovastatin, etc.

In one embodiment, a glycine-containing tripeptide molecule is administered in combination with an inhibitor of glycogen phosphorylase, such as, for example, PSN-357 or FR-258900 or those as described in WO2003084922, WO2004007455, WO2005073229-31 or WO2005067932.

In one embodiment, a glycine-containing tripeptide molecule is administered in combination with glucagon receptor antagonists such as, for example, A-770077, NNC-25-2504 or as described in WO2004100875 or WO2005065680.

In one embodiment, a glycine-containing tripeptide molecule is administered in combination with activators of glucokinase, such as, for example, LY-2121260 (WO2004063179), PSN-105, PSN-110, GKA-50 or those as are described for example in WO2004072031, WO2004072066, WO2005080360, WO2005044801, WO2006016194, WO2006058923, WO2006112549, WO2006125972, WO2007017549, WO2007017649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847.

In one embodiment, a glycine-containing tripeptide molecule is administered in combination with an inhibitor of gluconeogenesis, such as, for example, FR-225654.

In one embodiment, a glycine-containing tripeptide molecule is administered in combination with inhibitors of fructose-1,6-bisphosphatase (FBPase), such as, for example, CS-917 (MB-06322) or MB-07803 or those described in WO2006023515, WO2006104030, WO2007014619.

In one embodiment, glycine-containing tripeptide molecule is administered in combination with modulators of glucose transporter 4 (GLUT4), such as, for example, KST-48 (D.-O. Lee et al.: Arzneim.-Forsch. Drug Res. 54 (12), 835 (2004)).

In one embodiment, a glycine-containing tripeptide molecule is administered in combination with inhibitors of glutamine-fructose-6-phosphate amidotransferase (GFAT), as are described for example in WO2004101528.

In one embodiment, a glycine-containing tripeptide molecule is administered in combination with inhibitors of dipeptidylpeptidase IV (DPP-IV), such as, for example, vildagliptin (LAF-237), sitagliptin (MK-0431), sitagliptin phosphate, saxagliptin ((BMS-477118), GSK-823093, PSN-9301, SYR-322, SYR-619, TA-6666, TS-021, GRC-8200, GW-825964X, KRP-104, DP-893, ABT-341, ABT-279 or another salt thereof or those compounds as are described in WO2003074500, WO2003106456, WO2004037169, WO200450658, WO2005058901, WO2005012312, WO2005/012308, WO2006039325, WO2006058064, WO2006015691, WO2006015701, WO2006015699, WO2006015700, WO2006018117, WO2006099943, WO2006099941, JP2006160733, WO2006071752, WO2006065826, WO2006078676, WO2006073167, WO2006068163, WO2006090915, WO2006104356, WO2006127530, WO2006111261, WO2007015767, WO2007024993, WO2007029086.

In one embodiment, a glycine-containing tripeptide molecule is administered in combination with Janumet^{™}, a fixed combination of sitagliptin phosphate with metformin hydrochloride.

In one embodiment, a glycine-containing tripeptide molecule is administered in combination with inhibitors of 11-beta-hydroxysteroid dehydrogenase 1 (11.beta.-HSD1), such as, for example, BVT-2733, JNJ-25918646, INCB-13739 or those as are described for example in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138695, WO2006133926, WO2007003521, WO2007007688, US2007066584, WO2007047625, WO2007051811, WO2007051810.

In one embodiment, a glycine-containing tripeptide molecule is administered in combination with inhibitors of protein tyrosine phosphatase 1B (PTP1B), as are described for example in WO200119830-31, WO200117516, WO2004506446, WO2005012295, WO2005116003, WO2005116003, WO2006007959, DE 10 2004 060542.4, WO2007009911, WO2007028145, WO2007081755.

In one embodiment, a glycine-containing tripeptide molecule is administered in combination with modulators of the sodium-dependent glucose transporter 1 or 2 (SGLT1, SGLT2), such as, for example, KGA-2727, T-1095, SGL-0010, AVE 2268, SAR 7226 and sergliflozin or as described for example in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895, WO2007080170 or by A. L. Handlon in Expert Opin. Ther. Patents (2005) 15(11), 1531-1540.

In one embodiment, a glycine-containing tripeptide molecule is administered in combination with modulators of GPR40 as are described for example in WO2007013689, WO2007033002.

In one embodiment, a glycine-containing tripeptide molecule is administered in combination with modulators of GPR119b as are described for example in WO2004041274.

In one embodiment, glycine-containing tripeptide molecule is administered in combination with modulators of GPR119 as are described for example in WO2005061489 (PSN-632408), WO2004065380, WO2007003960-62 and WO2007003964.

In a further embodiment, a glycine-containing tripeptide molecule is administered in combination with modulators of GPR120.

In one embodiment, a glycine-containing tripeptide molecule is administered in combination with inhibitors of hormone-sensitive lipase (HSL) and/or phospholipases as described for example in WO2005073199, WO2006074957, WO2006087309, WO2006111321, WO2007042178.

In one embodiment, a glycine-containing tripeptide molecule is administered in combination with inhibitors of acetyl-CoA carboxylase (ACC), such as, for example, those as described in WO199946262, WO200372197, WO2003072197, WO2005044814, WO2005108370, JP2006131559, WO2007011809, WO2007011811, WO2007013691.

In a further embodiment, a glycine-containing tripeptide molecule is administered in combination with modulators of xanthine oxidoreductase (XOR).

In one embodiment, a glycine-containing tripeptide molecule is administered in combination with an inhibitor of phosphoenolpyruvate carboxykinase (PEPCK), such as, for example, those as described in WO2004074288.

In one embodiment, a glycine-containing tripeptide molecule is administered in combination with an inhibitor of glycogen synthase kinase 3 beta (GSK-3 beta), as described for example in US2005222220, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727 or WO2004046117.

In one embodiment, a glycine-containing tripeptide molecule is administered in combination with an inhibitor of the serum/glucocorticoid-regulated kinase (SGK) as described for example in WO2006072354.

In one embodiment, a glycine-containing tripeptide molecule is administered in combination with an agonist of the RUP3 receptor as described for example in WO2007035355.

In one embodiment, a glycine-containing tripeptide molecule is administered in combination with an inhibitor of protein kinase C beta (PKC beta), such as, for example, ruboxistaurin.

In another embodiment, glycine-containing tripeptide molecule is administered in combination with an activator of the gene which codes for the ataxia telangiectasia mutated (ATM) protein kinase, such as, for example, chloroquine.

In one embodiment, a glycine-containing tripeptide molecule is administered in combination with an endothelin A receptor antagonist such as, for example, avosentan (SPP-301).

In one embodiment, a glycine-containing tripeptide molecule is administered in combination with inhibitors of "I-kappaB kinase" (IKK inhibitors), as are described for example in WO02001000610, WO2001030774, WO2004022553 or WO2005097129.

In one embodiment, a glycine-containing tripeptide molecule is administered in combination with modulators of the glucocorticoid receptor (GR), as are described for example in WO2005090336, WO2006071609, WO2006135826.

In a further embodiment, a glycine-containing tripeptide molecule is administered in combination with CART modulators (see "Cocaine-amphetamine-regulated transcript influences energy metabolism, anxiety and gastric emptying in mice" Asakawa, A. et al.: Hormone and Metabolic Research (2001), 33(9), 554-558);

In a further embodiment, a glycine-containing tripeptide molecule is administered in combination with a NPY antagonist, such as, for example, naphthalene-1-sulfonic acid {4-[(4-aminoquinazolin-2-ylamino)methyl]cyclohexylmethyl}amide hydrochloride (CGP 71683A); NPY-5 receptor antagonists such as L-152804, or as are described for example in WO2006001318; NPY-4 receptor antagonists as are for example described in WO2007038942; NPY-2 receptor antagonists as are for example described in WO2007038943; Peptide YY 3-36 (PYY3-36) or analogous compounds, such as, for example, CJC-1682 (PYY3-36 conjugated with human serum albumin via Cys34), CJC-1643 (derivative of PYY3-36 which conjugates in vivo to serum albumin) or those as are described in WO2005080424, WO2006095166; derivatives of the peptide obestatin as are described in WO2006096847; CB1R (cannabinoid receptor 1) antagonists (such as, for example, rimonabant, SR147778, SLV-319, AVE-1625, MK-0364 or salts thereof or those compounds as are described for example in EP 0656354, WO00/15609, WO2001/64632-64634, WO 02/076949, WO2005080345, WO2005080328, WO2005080343, WO2005075450, WO2005080357, WO200170700, WO2003026647-48, WO200302776, WO2003040107, WO2003007887, WO2003027069, U.S. Pat. No. 6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007016460, WO2007020502, WO2007026215, WO2007028849, WO2007031720, WO2007031721, WO2007036945, WO2007038045, WO2007039740, US20070015810, WO2007046548, WO2007047737, WO2007084319, WO2007084450); cannabinoid receptor 1/cannabinoid receptor 2 (CB1/CB2) modulating compounds as described for example in WO2007001939, WO2007044215, WO2007047737; MC4 agonists (e.g. 1-amino-1,2,3,4-tetrahydronaphthalene-2-carboxylic acid [2-(3a-benzyl-2-methyl-3-oxo-2,3,3a,4,6,7-hexahydropyrazolo[4,3-c]pyridin- -5-yl)-1-(4-chlorophenyl)-2-oxoethyl]amide; (WO 01/91752)) or LB53280, LB53279, LB53278 or THIQ, MB243, RY764, CHIR-785, PT-141 or those that are described in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, WO2005118573, EP1538159, WO2004072076, WO2004072077, WO2006021655-57, WO2007009894, WO2007015162, WO2007041061, WO2007041052; orexin receptor antagonists (e.g. 1-(2-methylbenzoxazol-6-yl)-3-[1,5]naphthyridin-4-ylurea hydrochloride (SB-334867-A) or those as are described for example in WO200196302, WO200185693, WO2004085403, WO2005075458 or WO2006067224); histamine H3 receptor agonists (e.g. 3-cyclohexyl-1-(4,4-dimethyl-1,4,6,7-tetrahydroimidazo[4,5-c]pyridin-5-yl- )propan-1-one oxalic acid salt (WO 00/63208) or those as are described in WO200064884, WO2005082893, WO2006107661, WO2007003804, WO2007016496, WO2007020213); histamine H1/histamine H3 modulators such as for example betahistine and its dihydrochloride; CRF antagonists (e.g. [2-methyl-9-(2,4,6-trimethylphenyl)-9H-1,3,9-triazafluoren-4-yl]dipropyla- mine (WO 00/66585)); CRF BP antagonists (e.g. urocortin); urocortin agonists; agonists of the beta-3 adrenoceptor such as, for example, 1-(4-chloro-3-methanesulfonylmethylphenyl)-2-[2-(2,3-dimethyl-1H-indol-6-- yloxy)ethylamino]ethanol hydrochloride (WO 01/83451); or Solabegron (GW-427353) or N-5984 (KRP-204) or those described in JP2006111553, WO2002038543, WO2007048840-843; MSH (melanocyte-stimulating hormone) agonists; MCH (melanin-concentrating hormone) receptor antagonists (such as, for example, NBI-845, A-761, A-665798, A-798, ATC-0175, T-226296, T-71, GW-803430 or compounds such as are described in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649); CCK-A agonists (such as, for example, {2-[4-(4-chloro-2,5-dimethoxyphenyl)-5-(2-cyclohexylethyl)thiazol-2-ylcar- bamoyl]-5,7-dimethylindol-1-yl}acetic acid trifluoroacetic acid salt (WO 99/15525), SR-146131 (WO 0244150) or SSR-125180 or those as are described in WO2005116034); mixed sertoninergic and noradrenergic compounds (e.g. WO 00/71549); 5-HT receptor agonists, e.g. 1-(3-ethylbenzofuran-7-yl)piperazine oxalic acid salt (WO 01/09111); mixed dopamine/norepinephrine/acetylcholine reuptake inhibitors (e.g. tesofensine); 5-HT2C receptor agonists (such as, for example, lorcaserin hydrochloride (APD-356), BVT-933 or those as are described in WO200077010, WO20077001-02, WO2005019180, WO2003064423, WO200242304, WO2005035533, WO2005082859, WO2006077025, WO2006103511); 5-HT6 receptor antagonists such as for example E-6837 or BVT-74316 or those as are described for example in WO2005058858, WO2007054257; bombesin receptor agonists (BRS-3 agonists); galanin receptor antagonists; growth hormone (e.g. human growth hormone or AOD-9604); growth hormone-releasing compounds (tertiary butyl 6-benzyloxy-1-(2-diisopropyl-aminoethylcarbamoyl)-3,4-dihydro-1H-isoquino- line-2-carboxylate (WO 01/85695)); growth hormone secretagogue receptor antagonists (ghrelin antagonists) such as, for example, A-778193 or those as are described in WO2005030734; TRH agonists (see, for example, EP 0 462 884); uncoupling protein 2 or 3 modulators; leptin agonists (see, for example, Lee, Daniel W.; Leinung, Matthew C.; Rozhayskaya-Arena, Marina; Grasso, Patricia. Leptin agonists as a potential approach to the treatment of obesity. Drugs of the Future (2001), 26(9), 873-881); DA agonists (bromocriptine or Doprexin); lipase/amylase inhibitors (for example WO 00/40569); inhibitors of diacylglycerol O-acyltransferases (DGATs) such as, for example, BAY-74-4113 or as described for example in US2004/0224997, WO2004094618, WO200058491, WO2005044250, WO2005072740, JP2005206492, WO2005013907, WO2006004200, WO2006019020, WO2006064189, WO2006082952, WO2006120125, WO2006113919, WO2006134317, WO2007016538; inhibitors of fatty acid synthase (FAS) such as, for example, C75 or those as described in WO2004005277; inhibitors of stearoyl-CoA delta9 desaturase (SCD1) as described for example in WO2007009236, WO2007044085, WO2007046867, WO2007046868, WO20070501124; oxyntomodulin; oleoyl-estrone or thyroid hormone receptor agonists or partial agonists such as, for example: KB-2115 or those as described in WO20058279, WO200172692, WO200194293, WO2003084915, WO2004018421, WO2005092316, WO2007003419, WO2007009913, WO2007039125.

In a further embodiment, a glycine-containing tripeptide molecule is administered in combination with a varenicline tartrate, a partial agonist of the alpha 4-beta 2 nicotinic acetylcholine receptor.

In a further embodiment, glycine-containing tripeptide molecule is administered in combination with trodusquemine.

In a further embodiment, a glycine-containing tripeptide molecule is administered in combination with a modulator of the SIRT1 enzyme.

In a further embodiment, a glycine-containing tripeptide molecule is administered in combination with dexamphetamine or amphetamine.

In a further embodiment, a glycine-containing tripeptide molecule is administered in combination with fenfluramine or dexfenfluramine.

In a further embodiment, a glycine-containing tripeptide molecule is administered in combination with a sibutramine.

In a further embodiment, a glycine-containing tripeptide molecule is administered in combination with mazindole or phentermine.

Alternatively, the glycine-containing tripeptide molecule polypeptide treatment precedes or follows the secondary agent treatment by intervals ranging from minutes to weeks. In embodiments where the second therapeutic agent and the glycine-containing tripeptide molecule are administered separately, one would generally ensure that a significant period of time did not expire between the times of each delivery, such that the second agent and the glycine-containing tripeptide molecule would still be able to exert an advantageously combined effect. In such instances, it is contemplated that one administers both modalities within about 12-24 hours of each other and, in one aspect, within about 6-12 hours of each other, with a delay time of only about 12 hours being most preferred. In some situations, it is desirable to extend the time period for treatment significantly, however, where several days (2, 3, 4, 5, 6 or 7) to several weeks (1, 2, 3, 4, 5, 6, 7 or 8) lapse between the respective administrations.

In some embodiments, methods for the treatment of a metabolic disease, for example, a fatty liver disease may comprise administering a glycine-containing tripeptide molecule and a second therapeutic agent, for example, an ACC inhibitor, an ApoC-III inhibitor, an ACL-inhibitor, prescription fish oil, or a CETP inhibitor.

Systemic delivery of glycine-containing tripeptide molecule expression constructs or peptides to patients is a very efficient method for delivering a therapeutically effective composition to counteract the immediate clinical manifestations of a disease. Alternatively, local delivery of the glycine-containing tripeptide molecule and/or the second therapeutic agent is appropriate in certain circumstances.

The invention further provides methods of administering the compositions of the invention to a mammal. In one aspect, the mammal is human. An effective amount of a composition to be employed therapeutically will depend, for example, upon the therapeutic context and objectives. One skilled in the art will appreciate that the appropriate dosage levels for treatment will thus vary depending, in part, upon the molecule delivered, the indication for which the composition is being used, the route of administration, and the size (body weight, body surface or organ size) and condition (the age and general health) of the patient. Accordingly, the clinician may titer the dosage and modify the route of administration to obtain the optimal therapeutic effect.

In one aspect, a regimen for delivering the composition comprising a glycine-containing tripeptide molecule , or a pharmaceutically acceptable salt thereof to a mammal would include administration of from 5 mg/Kg to 2000 mg/Kg, or from 30 mg/Kg to 1500 mg/Kg, or from 40 mg/Kg to 1250 mg/Kg, or from 50 mg/Kg to 1000 mg/Kg, or from 60 mg/Kg to 5000 mg/Kg, given in daily doses or in equivalent doses at longer or shorter intervals, e.g., every other day, twice weekly, weekly, monthly, semi-annually, or even twice or three times daily. Administration may be oral, intravenous, subcutaneous, intranasal, inhalation, transdermal, transmucosal, or by any other route discussed herein.

In a further aspect, glycine-containing tripeptide molecule is administered by subcutaneous injection at the doses specified herein. However, glycine-containing tripeptide molecule may be administered by any of the methods discussed herein and as known in the art. In a further aspect, injections at a single site have a maximum allowable volume of 2.0 ml. It is contemplated that glycine-containing tripeptide molecule may be injected at multiple sites or at a greater dosage if a greater concentration of glycine-containing tripeptide molecule is needed. In another aspect, glycine-containing tripeptide molecule is administered at approximately the same time each day. However, alternative times for delivery are included in the invention.

The pharmaceutical compositions can also be selected for parenteral delivery. Alternatively, the compositions may be selected for inhalation or for delivery through the digestive tract, such as orally. The preparation of such pharmaceutically acceptable compositions is within the skill of the art.

In one embodiment, a pharmaceutical composition may be formulated for inhalation. For example, a glycine-containing tripeptide molecule composition may be formulated as a dry powder for inhalation. Pharmaceutical composition inhalation solutions may also be formulated with a propellant for aerosol delivery. In yet another embodiment, solutions may be nebulized. Pulmonary administration is further described in PCT Application No. PCT/US94/001875, which describes pulmonary delivery of chemically modified proteins.

It is also contemplated that certain formulations may be administered orally. In one embodiment of the present invention, glycine-containing tripeptide molecule compositions which are administered in this fashion can be formulated with or without those carriers customarily used in the compounding of solid dosage forms such as tablets and capsules. For example, a capsule may be designed to release the active portion of the formulation at the point in the gastrointestinal tract when bioavailability is maximized and pre systemic degradation is minimized. Additional agents can be included to facilitate absorption of the composition. Diluents, flavorings, low melting point waxes, vegetable oils, lubricants, suspending agents, tablet disintegrating agents, and binders may also be employed.

Another glycine-containing tripeptide molecule composition may involve an effective quantity of glycine-containing tripeptide molecule in a mixture with non-toxic excipients which are suitable for the manufacture of tablets. By dissolving the tablets in sterile water, or other appropriate vehicle, solutions can be prepared in unit dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional glycine-containing tripeptide molecule compositions will be evident to those skilled in the art, including formulations involving compositions in sustained or controlled delivery formulations. Techniques for formulating a variety of other sustained or controlled delivery means, such as liposome or micelle carriers, bioerodible microparticles or porous beads and depot injections, are also known to those skilled in the art.

The frequency of dosing will depend upon the pharmacokinetic parameters of the glycine-containing tripeptide molecule composition in the formulation used. Typically, a clinician will administer the composition until a dosage is reached that achieves the desired effect. The composition may therefore be administered as a single dose, or as two or more doses (which may or may not contain the same amount of the desired molecule) over time, or as a continuous infusion via implantation device or catheter. Further refinement of the appropriate dosage is routinely made by those of ordinary skill in the art and is within the ambit of tasks routinely performed by them. Appropriate dosages may be ascertained through use of appropriate dose response data.

In addition to the routes of administration disclosed herein, compositions of the invention can be introduced for treatment into a mammal by any mode, such as but not limited to, intravenous, intraperitoneal, intracerebral (intraparenchymal), intracerebroventricular, intramuscular, intraocular, intraarterial, intraportal, intralesional routes, intraarticular, intratumor, cerebrospinal, intrarectal and colon, topical, subconjunctival, intrabladder, intravaginal, epidural, intracostal, intradermal, inhalation, transdermal, transserosal, intrabuccal, oral, intranasal, dissolution in the mouth or other body cavities, instillation to the airway, insufflation through the airway, injection into vessels, tumors, organ and the like, and injection or deposition into cavities in the body of a mammal.

Alternatively or additionally, the composition may be administered locally via implantation of a membrane, sponge, or another appropriate material on to which the desired molecule has been absorbed or encapsulated. Where an implantation device is used, the device may be implanted into any suitable tissue or organ, and delivery of the desired molecule may be via diffusion, timed release bolus, or continuous administration.

In some cases, it may be desirable to use compositions in an ex vivo manner. In such instances, cells, tissues, or organs that have been removed from the patient are exposed to compositions after which the cells, tissues and/or organs are subsequently implanted back into the patient.

Administration of the compositions of the invention can be carried out using any of several standard methods including, for example, continuous infusion, bolus injection, intermittent infusion, inhalation, or combinations of these methods. For example, one mode of administration that can be used involves continuous intravenous infusion. In such an approach, the infusion dosage rate of the compositions of the invention can be, for example, 0.001-0.5 mg/kg body weight/hour, more preferably 0.01-0.2 mg/kg/hour, and most preferably 0.03-0.1 mg/kg/hour, with the drug being infused over the course of, for example, 1-100, 10-100, or about 12, 24, 48, 72, 84 or 96 hours. The infusion of the compositions of the invention can, if desired, be preceded by a bolus injection. Such a bolus injection is given at a dosage ranging from about 0.001 to about 10 mg/kg. Variations in the dose and in the time period of infusion of the compositions of the invention may occur and are also included in the invention.

A single bolus injection may be given by intravenous infusion through, for example, a central access line or a peripheral venous line, or by direct injection, using a syringe. Such administration may be desirable if a patient is only at short-term risk for exposure to endotoxin, and thus does not need prolonged persistence of the drug. For example, this mode of administration may be desirable in surgical patients, if appropriate, such as patients having cardiac surgery, e.g., coronary artery bypass graft surgery and/or valve replacement surgery. In these patients, a single bolus infusion of drug can be administered over a period of four hours prior to and/or during surgery. (Note that the amount of drug administered is based on the weight and condition of the patient and is determined by the skilled practitioner.) Shorter or longer time periods of administration can be used, as determined to be appropriate by one of skill in this art.

In cases in which longer-term delivery of a glycine tripeptide molecule, or a pharmaceutically acceptable salt thereof, of the invention is desirable, intermittent administration can be carried out. In these methods, a loading dose is administered, followed by either (i) a second loading dose and a maintenance dose (or doses), or (ii) a maintenance dose or doses, without a second loading dose, as determined to be appropriate by one of skill in this art.

To achieve further delivery of the glycine tripeptide molecule, or a pharmaceutically acceptable salt thereof in a patient, a maintenance dose (or doses) of the compound can be administered, so that levels of the compound are maintained in the blood of a patient. Maintenance doses can be administered at levels that are less than the loading dose(s), for example, at a level that is about 1/6 of the loading dose. Specific amounts to be administered in maintenance doses can be determined by a medical professional, with the goal that the compound level is at least maintained. Maintenance doses can be administered, for example, for about 2 hours every 12 hours beginning at hour 24 and continuing at, for example, hours 36, 48, 60, 72, 84, 96, 108, and 120. Of course, maintenance doses can be stopped at any point during this time frame, as determined to be appropriate by a medical professional.

The infusion methods described above can be carried out using catheters (e.g., peripheral venous, central venous, or pulmonary artery catheters) and related products (e.g., infusion pumps and tubing) that are widely available in the art. One criterion that is important to consider in selecting a catheter and/or tubing to use in these methods is the impact of the material of these products (or coatings on these products) on the size of the drug.

Additional catheter-related products that can be used in the methods of the invention can be identified by determining whether the material of the products alters size of the compound, under conditions consistent with those that are used in drug administration. In addition, in the event that a patient already has a catheter in place that does not maintain optimal drug size, a catheter insert that is made of a compatible material (e.g., a polyamide polymer) or that includes a compatible coating can be used so that the drug solution does not contact the surface of the incompatible catheter. Such an insert, having an outside diameter that is small enough to enable it to be easily inserted into the existing catheter, while maintaining an inside diameter that is large enough to accommodate solution flow of the compound, is placed within the existing catheter and connected to tubing or a syringe through which the drug is delivered.

Appropriate frequency of administration can be determined by one of skill in the art and can be administered once to several times per day. The compositions of the invention may also be administered once each day or once every other day. In the case of acute administration, treatment is typically carried out for periods of hours or days, while chronic treatment can be carried out for weeks, months, or even years.

Both chronic and acute administration can employ standard hepatic portal administration formulations, which can be made from the formulations described elsewhere herein. Administration by this route offers several advantages, for example, rapid onset of action by administering the glycine tripeptide molecule, or a pharmaceutically acceptable salt thereof to the desired site of action, at higher local concentrations. For a therapeutic moiety to exert its desired effect, it needs to be in physical contact with its physiological target such as a receptor present on liver cells. Site-specific drug delivery ensures that such interactions take place only in the desired anatomical location of the liver; therefore, it must fulfil the following criteria: (i) it must be able to cross the anatomical barriers such as those of stomach and intestine, (ii) should be recognized selectively by the receptor present on liver cell such as asialoglycoprotein, (iii) exogenously delivered ligand for targeting should compete with the endogenously produced ligand, (iv) fabricated delivery system must be nontoxic, biocompatible, biodegradable, and physico-chemically stable in the liver cells either in vivo or in vitro, (v) it should have uniform sinusoid capillary distribution, (vi) should have controllable and predictable rate of drug release so that only therapeutic amount of the glycine tripeptide molecule, or a pharmaceutically acceptable salt thereof is released to the liver cells, (vii) drug release should not affect the drug distribution, (viii) it should show minimal drug leakage during its passage through stomach, intestine, and other parts of the body, (ix) carrier used for encapsulating the glycine tripeptide molecules must be eliminated from the body without imparting any sign of toxicity and no carrier should induce modulation of diseased state, and (x) lastly, preparation of the glycine tripeptide molecule, or a pharmaceutically acceptable salt thereof delivery system should be easy or reasonably simple, reproducible, and cost-effective.

The glycine tripeptide molecule, or a pharmaceutically acceptable salt thereof can be administered as part of a drug delivery system directed to liver tissue delivery. Different methods for coupling of a glycine-containing tripeptide molecule to a liver specific targeting moiety, can include: (a) coupling of targeting moieties on preformed nanocarriers, (b) coupling of targeting moieties by the post-insertion method, (c) coupling of targeting moieties by the Avidin/Biotin complex, (d) coupling of targeting moieties before nanocarriers formulation. Exemplary targeting moieties and carries useful in the delivery of the compositions of the present invention to specific liver cells are described in Nidhi Mishra, et al., (2013) "Efficient Hepatic Delivery of Drugs: Novel Strategies and Their Significance", BioMed Research International, BioMed Research International, Volume 2013, the disclosure of which is incorporated herein by reference in its entirety.

While certain glycine tripeptide molecules and their pharmaceutically acceptable salts, compositions and methods described herein have been described with specificity in accordance with certain embodiments, the following examples serve only to illustrate the compounds described herein and are not intended to limit the same. Each of the references recited in the present application is incorporated herein by reference in its entirety.

### EXAMPLES

### METHODS

### Animal procedures

All animal procedures were approved by the Institutional Animal Care & Use Committee of the University of Michigan (PRO00008239) and performed in accordance with the institutional guidelines. Eight-week-old male apolipoprotein E-deficient (apoE-/-) mice (B6.129P2-Apoetm1Unc/J, Stock No.: 002052) were obtained from Jackson Laboratories. Mice were fed Western diet (WD, 42% of calories from fat and 0.2% cholesterol by weight, Envigo TD.88137). After one week of WD feeding, blood was carefully collected from the facial vein to determine baseline plasma levels of total cholesterol (TC). Then, mice were maintained on WD feeding and randomly divided into 5 experimental groups and administered the following treatments via oral gavage, 6 times per week for a period of 12 weeks: 1) WD + H₂O (received water as vehicle control); 2) WD +Gly (0.67 mg/g glycine); 3) WD +Leu (0.33 mg/g leucine); 4) WD +DT-109 (1 mg/g DT-109 = Gly-Gly-Leu); 5) WD +DT-110 (1 mg/g DT-110 = Gly-Gly-dLeu). All mice were maintained on a 12-hour light/dark cycle and had ad libitum access to food and water. Body weight and food intake were measured every one and three weeks, respectively

### OGTT and non-fasting blood glucose

At week 10, oral glucose tolerance tests (OGTT) were performed following an overnight fast by administering 2 mg/g of glucose via oral gavage. To evaluate the acute effects of the treatments, glycine (0.67 mg/g), leucine (0.33 mg/g), DT-109 or DT-110 (1 mg/g) were added to the glucose solution and administrated via oral gavage. To evaluate the chronic effects of the treatments, mice were administered with glucose alone. Baseline glucose levels were measured after collecting blood from the tip of the tail using glucometer and test strips (NDC: 0193-7308-50, Contour Next). Then, mice were administered with glucose with (acute) or without (chronic) treatments and blood glucose levels were measured every 30 min for up to 120 min. To assess the effects of the treatments on non-fasting glucose levels, blood was collected from the tip of the tail before routine administration of treatments via oral gavage (pre-gavage) and 30 min afterwards (post-gavage). Glucose levels were determined using glucometer and test strips (NDC: 0193-7308-50, Contour Next).

### Plasma lipids, adipokines and cytokines

Plasma levels of TC, triglycerides (TG), low-density lipoprotein cholesterol (LDL) and high-density lipoprotein cholesterol (HDL) were measured with a Cobas Mira chemistry analyzer (Roche Diagnostics) at the Chemistry Laboratory of the Michigan Diabetes Research Center (MDRC) using manufacturer-provided assay reagents and protocols. Plasma levels of glycine tripeptide molecule, resistin, interleukin-6 (IL-6) and monocyte chemoattractant protein-1 (MCP1) were measured with a Luminex 200 chemistry analyzer (Luminex Corporation) at the Chemistry Laboratory of the MDRC using manufacturer-provided assay reagents and protocols (Millipore Multiplex, MMHMAG-44K).

### Histological analyses

Histology processing was performed by the In Vivo Animal Core (IVAC) Histology Laboratory within the Unit for Laboratory Animal Medicine (ULAM) of the University of Michigan. Formalin-fixed tissues were processed through graded alcohols and cleared with xylene followed by infiltration with molten paraffin using an automated VIP5 or VIP6 tissue processor (TissueTek, Sakura-Americas). Following paraffin embedding using a Histostar Embedding Station (ThermoFisher Scientific), tissues were sectioned on a M 355S rotary microtome (ThermoFisher Scientific) at 4 µm thickness and mounted on glass slides. Following deparaffinization and hydration with xylene and graded alcohols, slides were stained for hematoxylin and eosin (H&E) using Harris hematoxylin (ThermoFisher Scientific, Cat# 842), differentiated with Clarifier (ThermoFisher Scientific, Cat#7401), blued with bluing reagent (ThermoFisher Scientific, Cat#7301), stained with eosin Y, alcoholic (ThermoFisher Scientific, Cat# 832), then dehydrated and cleared through graded alcohols and xylene and coverslipped with Micromount (Leica cat# 3801731) using a Leica CV5030 automatic coverslipper. Immunohistochemical staining was performed on an IntelliPATH FLX automated immunohistochemical stainer (Biocare Medical, Cat# IPS0001US) and included blocking for endogenous peroxidases and non-specific binding, detection using a horseradish peroxidase biotin-free polymer based commercial detection system, disclosure with diaminobenzidine chromogen, and nuclear counterstaining with hematoxylin. Specific to F4/80, (Bio-Rad ABD Serotec, Cat# MCA497), the rat monoclonal primary antibody (clone CI:A3-1) was diluted to 1:400 in DaVinci Diluent (Biocare Medical, Cat# PD900) and incubated for 60 min followed by detection using Rat-on-Mouse HRP-Polymer, (Biocare Medical, Cat# RT517) 2-step probe-polymer incubation for 10 and 30 min respectively.

### RNA Isolation, reverse transcription and quantitative polymerase chain reaction (qPCR)

Total RNA from liver or adipose tissue samples was extracted using QIAGEN's RNeasy kits (QIAGEN). RNA was reverse-transcribed into cDNA with SuperScript III and random primers (Invitrogen). Specific transcript levels were assessed by a real-time PCR system (Bio-Rad) using iQ SYBR Green Supermix (Bio-Rad) and the ΔΔCt threshold cycle method of normalization. Gene expression levels were normalized to glyceraldehyde 3-phosphate dehydrogenase (GAPDH). Primer pairs used for qPCR were obtained from Integrated DNA Technologies and are listed below:

| Gene | Forward primer | SEQ ID NO | Reverse primer | SEQ ID NO |
|---|---|---|---|---|
| GLUT1 | ACCTGCAGGAGATGAAAGAAGAG | 3 | CTCGAAGATGCTCGTTGAGTAGT | 4 |
| GLUT2 | ATCCTACTTGGCCTATCTGCTGT | 5 | GGTGACATCCTCAGTTCCTCTTA | 6 |
| GLUT4 | ACTAGCTGAGCTGAAGGATGAGA | 7 | ACTCGAAGATGCTGGTTGAATAG | 8 |
| G6pc | CCTCCGGAAGTATTGTCTCATC | 9 | GTAGTGTCAAGGTGGACCCATT | 10 |
| PCK1 | ATATGGTGGGAACTCACTACTCG | 11 | AAGTTAGTCTTCCCACAGGCACT | 12 |
| FBP1 | TGCCATCAATGAGTATCTCCAG | 13 | ACATAAGCTATGGGGTTGCACT | 14 |
| AMPKα1 | GTCAAAGCCGACCCAATGATA | 15 | CGTACACGCAAATAATAGGGGTT | 16 |
| PPARγ | GGAAGACCACTCGCATTCCTT | 17 | GTAATCAGCAACCATTGGGTCA | 18 |
| PPARα | AACATCGAGTGTCGAATATGTGG | 19 | CCGAATAGTTCGCCGAAAGAA | 20 |
| PGC1α | ATCACGTTCAAGGTCACCCTAC | 21 | TTCTGCTTCTGCCTCTCTCTCT | 22 |
| PGC1β | GTACAGCTCATTCGCTACATGC | 23 | GGCCAGAAGTTCCCTTAGGATA | 24 |
| CD36 | TGCTGGAGCTGTTATTGGTG | 25 | TGGGTTTTGCACATCAAAGA | 26 |
| PNPLA2 | TCCGTGGCTGTCTACTAAAGA | 27 | TGGGATATGATGACGTTCTCTCC | 28 |
| PNPLA3 | CCATTAAAGAAACCGGTAGCAG | 29 | TGCTAGGGAGAGAAAGAAGGAA | 30 |
| CPT1a | AGATCAATCGGACCCTAGACAC | 31 | CAGCGAGTAGCGCATAGTCA | 32 |
| CPT2 | CCTGCTCGCTCAGGATAAACA | 33 | GTGTCTTCAGAAACCGCACTG | 34 |
| CACT | CAACCACCAAGTTTGTCTGGA | 35 | CCCTCTCTCATAAGAGTCTTCCG | 36 |
| ACADs | AGTGGTGCAGGCTTGGATTAC | 37 | ACTGAGGGCAAAACAGCCG | 38 |
| ACADm | AGGGTTTAGTTTTGAGTTGACGG | 39 | CCCCGCTTTTGTCATATTCCG | 40 |
| ACADI | TGCCCTATATTGCGAATTACGG | 41 | CTATGGCACCGATACACTTGC | 42 |
| ACOX1 | CCGCCACCTTCAATCCAGAG | 43 | CAAGTTCTCGATTTCTCGACGG | 44 |
| UCP2 | ATGGTTGGTTTCAAGGCCACA | 45 | TTGGCGGTATCCAGAGGGAA | 46 |
| LXRα | TGCCATCAGCATCTTCTCTG | 47 | GGCTCACCAGCTTCATTAGC | 48 |
| LXRβ | CGCTACAACCACGAGACAGA | 49 | TGTTGATGGCGATAAGCAAG | 50 |
| SREBP2 | TGGGAGAGTTCCCTGATTTG | 51 | GATAATGGGACCTGGCTGAA | 52 |
| HMGCS | TTTGATGCAGCTGTTTGAGG | 53 | CCACCTGTAGGTCTGGCATT | 54 |
| LDLR | TCCTGGAGATGTGATGGACA | 55 | GAGCCATCTAGGCAATCTCG | 56 |
| CYP7a1 | CTAAAAGCCCAGCTACAGGAAA | 57 | GCTCTTTGAAAGGTGGTACAGG | 58 |
| ABCG5 | CGTCCAGAACAACACGCTAA | 59 | GCAGCATCTGCCACTTATGA | 60 |
| ABCG8 | AAGACGGGCTGTACACTGCT | 61 | AGGAGGACATGTGGAAGGTG | 62 |
| TNFα | CTGTGAAGGGAATGGGTGTT | 63 | GGTCACTGTCCCAGCATCTT | 64 |
| IL-6 | TAGTCCTTCCTACCCCAATTTCC | 65 | TTGGTCCTTAGCCACTCCTTC | 66 |
| MCP1 | TTAAAAACCTGGATCGGAACCAA | 67 | GCATTAGCTTCAGATTTACGGGT | 68 |
| IL-10 | CCTGGGTGAGAAGCTGAAGA | 69 | ACTCTTCACCTGCTCCACTG | 70 |
| GAPDH | CTGCGACTTCAACAGCAACT | 71 | GAGTTGGGATAGGGCCTCTC | 72 |

### Hepatic lipid extraction

Livers were rapidly removed from the euthanized mice and kept at -80°C. Frozen liver samples (approximately 100 mg) were homogenized in PBS and centrifuged (14,000 RPM, 20 min). The supernatants were collected and analyzed for protein content by the Bio-Rad Bradford assay. To assess liver lipid composition, lipids were extracted from the supernatants using hexane (≥99%, 32293, Sigma-Aldrich) and isopropanol (≥99.5%, A426-4, Fisher Scientific) at a 3:2 ratio (v:v), and the hexane phase was left for evaporation for 48 h. The amount of liver TG or TC was determined spectrophotometrically using commercially available kits (Wako Chemicals). Data were normalized to protein levels and presented as µg TG or TC / mg protein.

### Hepatic protein extraction and Western blotting

Tissue extracts were prepared using radioimmunoprecipitation assay lysis buffer (RIPA buffer, Thermo Scientific) supplemented with a protease inhibitor cocktail (Roche Applied Science). Proteins were resolved in 10% sodium dodecyl sulfate polyacrylamide gel electrophoresis (SDS-PAGE) and transferred to nitrocellulose membranes (Bio-Rad). The membranes were blocked for 1 hour at room temperature in tris-buffered saline-Tween 20 (TBST) containing 5% fat-free milk and incubated with primary antibody at 4°C overnight. The following primary antibodies were used: rabbit polyclonal anti-ABCG8 (Santa Cruz Biotechnology, sc-30111, working dilution 1:1000) and rabbit polyclonal anti-β-Actin (Cell signaling, 4967S, working dilution 1:3000). After TBST washing, membranes were incubated with secondary antibodies (LI-COR Biotechnology, donkey anti-rabbit IRDye 926-32213, 926-68073, working dilution 1:10000) for 1 hour at room temperature. After TBST washing, bands were visualized and quantified using an Odyssey Infrared Imaging System (LI-COR Biosciences, version 2.1).

### Analysis of atherosclerotic lesions

After collection of blood, the mice were perfused with 20 mL of saline solution through the heart, followed by 20 mL of 37% formalin. The mice were fixed with formalin and the whole aortic tree was dissected under a surgical microscope. Next, the aortic trees were stained with Oil Red O solution (0.2% Oil Red O (w/v) in 3.5:1 of methanol:1N NaOH) for 50 min, followed by 70% ethanol for 30 min. Aortic trees were then kept in DDW. The aortic trees were then cleared of adhering adipose and connective tissues, and longitudinally opened with Vannas scissors to expose the atherosclerotic lesions. Images of whole aortic trees were obtained using a digital camera, and the percentage of the plaque area stained by Oil Red O with respect to the total luminal surface area was quantified with ImageJ analysis software (http://imagej.nih.gov/ij/).

### Statistical analysis

SPSS 24.0 software (SPSS Inc. IBM) was used for data analysis. Data are presented as box and whiskers plots or as mean ± SEM. The number of animals used for each study is specified for each FIGFIG. legend. One-way analysis of variance (ANOVA) followed by Tukey post hoc test was used for data analysis. Differences were considered statistically significant at p<0.05.

### RESULTS

### Experiment 1 Effects on body weight and adiposity

The experimental design is depicted in FIG. 1A. After one week of Western diet (WD) feeding to apoE-/- mice, blood was collected from the facial vein (FV) to determine baseline plasma levels of TC. Then, mice were maintained on WD feeding and randomly divided into 5 experimental groups administrated the following treatments via oral gavage, 6 times per week for a period of 12 weeks: 1) WD + H₂O; 2) WD +Gly (0.67 mg/g glycine); 3) WD +Leu (0.33 mg/g leucine); 4) WD +DT-109 (1 mg/g DT-109); 5) WD +DT-110 (1 mg/g DT-110). No significant differences in food intake were noted throughout the study (FIG. 1B). Endpoint measurements revealed significantly lower body weight in mice administrated with glycine (by 12%, p<0.05, FIG. 1C), whereas the total gain in body weight from baseline to endpoint was significantly lower in mice treated with glycine or DT-109 (by 51%, p<0.01 or 28%, p<0.05, respectively, FIG. 1D). Gross appearance of the peritoneal cavities at endpoint revealed yellowish coloration of the liver and enlarged visceral fat pads in control mice and in mice administrated with leucine, which were attenuated by treatments with glycine, DT-109 or DT-110 (FIG. 1E). Accordingly, plasma levels of glycine-containing tripeptide molecule were significantly lower in mice administrated with glycine, DT-109 or DT-110 (by 53%, 62% or 56%, p<0.05, FIG. 1F). Taking together, these results indicate protective effects of glycine, DT-109 and to a lesser degree DT-110 against WD-induced adiposity.

### Experiment 2 Effects on glucose homeostasis

At week 10 of WD feeding, OGTT was performed following an overnight fast. To determine the acute effects of the treatments on blood glucose levels, glycine (0.67 mg/g), leucine (0.33 mg/g), DT-109 or DT-110 (1 mg/g) were added to the glucose solution (2 mg/g) and administrated via oral gavage. As shown in FIG. 2A, no significant differences in blood glucose levels were noted between the groups at baseline. After 30 min from administration of glucose with or without treatments, blood glucose levels were markedly decreased by all treatments with most significant effects for glycine or DT-109. At later time points (60-120 min), blood glucose levels were significantly decreased in mice treated with glycine, DT-109 and DT-110, but not with leucine. To evaluate the chronic effects of the treatments, OGTT was repeated at week 11 of WD feeding, this time without adding treatments to the glucose solution. No significant differences were noted between the groups after glucose loading without treatments (FIG. 2B). To assess the effects of the treatments on non-fasting glucose level, blood was collected before the routine administration of treatments via oral gavage (pre-gavage) and 30 min afterwards (post-gavage). Compared to control mice, pre-gavage glucose levels were significantly lower in all groups. However, whereas oral gavage with water or leucine resulted in trends towards increased blood glucose, oral gavage with glycine, DT-109 or DT-110 significantly decreased blood glucose levels (by 10%, p<0.05, 21%, p<0.01 or 13%, p<0.05, respectively, FIG. 2C). Finally, endpoint glucose levels (following a 6 h fast), were significantly lower in mice treated with glycine or DT-109 (by 41%, p<0.01 or 30%, %, p<0.05, respectively, FIG. 2D). Analysis of hepatic gene expression revealed that genes regulating glucose uptake (GLUT1, GLUT2 and GLUT4) were upregulated by glycine treatment with no significant change in the expression of genes regulating gluconeogenesis (G6pc, PCK1 and FBP1). GLUT1 expression was also markedly induced in livers from mice treated with DT-110 (FIG. 2E). Taken together, these results indicate marked postprandial glucose-lowering properties for glycine, DT-109 and DT-110.

### Experiment 3 Effects on hepatic lipid metabolism

H&E staining of liver samples revealed marked microvesicular and macrovesicular steatosis in control mice as well as in mice administrated with leucine. Significantly, treatment with glycine, DT-109 or with DT-110 abolished WD-induced hepatic steatosis (FIG. 3A). Accordingly, extraction of hepatic lipids followed by quantification of TG and TC contents, revealed that treatment with glycine, DT-109 or with DT-110 significantly decreased hepatic TG (by 74%, 73% or 68%, p<0.01, respectively, FIG. 3B) and TC (76%, 71%, p<0.01, or 63%, p<0.01, respectively, FIG. 3C), with no significant effects for leucine administration.

Experiment 4 Expression of genes regulating TG, fatty acid and cholesterol accumulation in the liver was evaluated next to explain the observed effects on hepatic steatosis. Interestingly, treatment with glycine, DT-109 or DT-110, but not with leucine, significantly induced the expression of master regulators of hepatic lipid oxidation - AMPKα1 and PPARα (FIG. 4A). Accordingly, key target genes regulating TG hydrolysis (PNPLA2), mitochondrial β-oxidation (CPT1a, CACT, ACADI) as well as the mitochondrial anion carrier UCP2, were significantly upregulated in livers from mice treated with glycine, DT-109 or DT-110 (FIG. 4A, FIG. 4B). As for genes regulating cholesterol homeostasis in the liver, treatment with glycine, DT-109 or DT-110 significantly increased the expression of ABCG5 and ABCG8, key regulators of cholesterol excretion into bile. A significant upregulation of LDLR was found in livers from mice treated with DT-110, with similar trends for glycine and DT-109 treatments (FIG. 4C). Overexpression of ABCG8 in livers of mice treated with glycine, DT-109 or DT-110 was confirmed by Western blot (FIG. 4D, and FIG. 4E). Overall, these results highlight potent protective effects of glycine, DT-109 and DT-110 against WD-induced hepatic steatosis in relation with overexpression of genes regulating lipid oxidation and extraction of cholesterol into bile.

### Experiment 5 Effects on plasma lipid profile and atherosclerosis

Before randomization into experimental groups, blood was collected from mice after one week of WD feeding to determine baseline plasma TC levels. Plasma TC were measured again at endpoint, after 12 weeks of WD feeding with treatments or water control. As shown in FIG. 5A, the time-dependent increase in plasma TC was significantly attenuated by treatment with glycine or DT-109, with a similar trend for DT-110 treatment, but not for leucine treatment. Accordingly, endpoint plasma levels of TC and LDL were significantly reduced in mice treated with glycine or DT-109 (TC: by 19%, p<0.05 or 25%, p<0.01, respectively; LDL: by 30%, p<0.01 or 36%, p<0.01, respectively), with a similar trend for DT-110 treatment (FIG. 5B, FIG. 5C). Interestingly, plasma HDL levels were significantly lower in mice treated with glycine (by 35%, p<0.05), but were preserved by DT-109 treatment (FIG. 5D). No significant changes in plasma TG levels were noted (FIG. 5E). Finally, analysis of *en face* aortic lesions by Oil Red O staining revealed a significant reduction in total atherosclerotic plaques in mice treated with glycine, DT-109 or DT-110 (by 48%, p<0.01, 62%, p<0.001 or 49%, p<0.01, respectively, FIG. 5F, FIG. 5G), but not in mice treated with leucine.

### Experiment 6 Effects on systemic, hepatic and adipose tissue inflammation

Since inflammation plays a key role in the pathogenesis of both NAFLD and atherosclerosis, plasma levels of proinflammatory cytokines and adipokines were evaluated next. Whereas no significant changes were noted between the groups for plasma levels of IL-6 and resistin (FIG. 6A, FIG. 6B), MCP1 levels were significantly reduced in mice treated with glycine, DT-109 or DT-110 (FIG. 6C). Analysis of gene expression in epididymal adipose tissue (EAT) and in subcutaneous adipose tissue (SAT) revealed a significant downregulation of TNFα in EAT by all treatments with the most significant effects for DT-110 (FIG 6D). In accordance with lower MCP1 plasma levels, MCP1 expression both in EAR and in SAT was significantly suppressed by treatment with glycine, DT-109 or DT-110, but not with leucine (FIG. 6D, FIG. 6E). Finally, although glycine suppressed MCP1 expression in the liver (with a similar trend for DT-109, FIG. 7A), no significant changes were noted in tissue F4/80 determined by immunohistochemistry (FIG. 7B, FIG. 7C). Overall, these results suggest some anti-inflammatory effects for glycine, DT-109 and DT-110 related mainly to suppression of MCP1 in adipose tissues and in the circulation.

### EXAMPLE x Glycine-Based Treatment for NAFLD

### A. Introduction

NAFLD, the most common chronic liver disease, affects 25% of the population worldwide. NAFLD encompasses a spectrum of liver pathologies ranging from hepatic steatosis (HS), NASH, characterized by hepatocyte damage and lobular inflammation in association with fibrosis progression, and cirrhosis that may lead to liver failure or hepatocellular carcinoma. Cardiometabolic comorbidities, including obesity, type 2 diabetes (T2D), metabolic syndrome (MetS) and dyslipidemia are common in NAFLD patients. Apart from liver-specific mortality, cardiovascular disease is a leading cause of death in NAFLD patients, particularly with NASH. Despite the global burden of NAFLD and substantial efforts in drug development, no therapy has been approved so far.

Recent advances, particularly in metabolomics and the gut microbiota, has improved our understanding of NAFLD pathogenesis, suggesting new therapeutic targets. Whereas abnormal lipid and carbohydrate metabolism are known to be implicated in NAFLD, recent metabolomics-based studies indicate that dysregulated metabolism of specific amino acids (AA) plays a role in NAFLD pathogenesis. Particularly, while most circulating AA are increased in NAFLD patients, glycine levels are decreased. Circulating glycine levels are negatively correlated with the level of HS, hepatocyte ballooning and lobular inflammation. Plasma glycine together with known biomarkers (e.g. aspartate-aminotransferase (AST) and PNPLA3 genotype) was recently included in a model to predict NASH. Lower plasma glycine is associated with higher prevalence of obesity, T2D, MetS, coronary heart disease and myocardial infarction, while higher plasma glycine is associated with a favorable lipid profile (summarized in Table S1).

**Table S1. Previous clinical evidences associating lower circulating glycine with NAFLD and cardiometabolic diseases**

| **Cardiometabolic disease** | **N human subjects** | **Circulating glycine outcome (reference in the main text)** |
|---|---|---|
| NAFLD | Training dataset: 1535 Healthy vs. 465 NAFLD | Glycine was decreased while the majority of AA were increased in NAFLD patients. |
| | Validation dataset: 1661 Healthy vs. 499 NAFLD | |
| NAFLD | 86 (varying degrees of hepatic steatosis) | Glycine was negatively correlated with the level of hepatic steatosis. |
| NAFLD | 20 Healthy vs. 44 NAFLD | Glycine decreased in NAFLD patients and negatively correlated with hepatocyte ballooning and lobular inflammation. |
| Obesity | 67 Lean vs. 74 Obese | Glycine was decreased while various AA increased in obese patients. |
| T2D | Meta-analysis including 8,000 subjects (1940 with T2D) | Glycine inversely associated with prediabetes and T2D. |
| MetS (obesity and dyslipidemia) | 472 (population study) | Glycine was negatively correlated with waist circumference, plasma TG, and MetS while positively correlated with HDL-C. |
| AMI (obesity, T2D and dyslipidemia) | 4109 (with suspected stable angina pectoris) | Glycine was inversely associated with risk of AMI and positively associated with lower prevalence of obesity, T2D and a favorable lipid profile (higher HDL-C and apoA1, lower TG). |
| CHD | 11,147 (2053 with CHD) | Glycine was associated with lower risk of CHD and MI. |

As a non-essential AA, glycine is synthesized from several precursors mainly in the liver. These reactions are catalyzed by key enzymes driving glycine formation from serine (serine hydroxymethyltransferases, SHMTs), threonine (threonine dehydrogenase, TDH), choline via sarcosine (choline dehydrogenase, CHDH; sarcosine dehydrogenase, SARDH) and from alanine to glyoxylate (AGXTs). Glycine is utilized in multiple pathways to generate essential molecules including nucleic acids, heme and glutathione. In NAFLD or T2D, glutathione synthesis is diminished due to limited glycine availability and is restored following dietary supplementation. Beyond this antioxidant role, glycine has dual benefits in lipid and glucose metabolism. Glycine ingestion reduces blood glucose, partly through stimulating insulin secretion from pancreatic β-cells. In genetically obese/diabetic KK-A^{y} mice, dietary glycine improved glucose tolerance, lowered circulating TG and alanine-aminotransferase (ALT), while attenuating HS and inflammatory infiltration. In sucrose-fed rats, glycine reduced intra-abdominal fat, plasma TG and increased FAO markers in hepatic mitochondria. Nevertheless, a comprehensive investigation of glycine role in NAFLD, applying models that fully mimic the human disease and accurate dosing, has not been pursued.

Considering the burden of NAFLD, lack of available therapies, and consistent reports associating lower circulating glycine to NAFLD severity, there is a strong rationale to better understand glycine metabolism in NAFLD, which could lead to novel therapeutics. Herein, we identified suppression of glycine biosynthetic genes, predominantly AGXT1, in human and murine NAFLD. Applying genetic (AGXT1^{-/-}) and dietary approaches to limit glycine availability, we found exacerbated hyperlipidemia and steatohepatitis. Searching for glycine-based compounds with dual lipid/glucose-lowering properties, we identified DT-109 that potently protected mice from diet-induced NASH by modulating hepatic FAO.

### B. Abbreviations:

AA, amino acids; ACAA, acetyl-CoA acyltransferase; ACAD, acyl-CoA dehydrogenase; ACOT, acyl-CoA thioesterase; ACOX, acyl-CoA oxidase; ACSL, acyl-CoA synthetase long chain; ACSM, acyl-CoA synthetase medium-chain; AGXT, alanine-glyoxylate aminotransferase; ALP, alkaline phosphatase; ALT, alanine aminotransferase; ApoE, apolipoprotein E; AST, aspartate aminotransferase; CACT, carnitine/acylcarnitine translocase; Cas9, CRISPR-associated protein 9; CCL, C-C motif chemokine ligand; CCR, C-C motif chemokine receptor; CD, chow diet; CHD, coronary heart disease; CLAMS, comprehensive laboratory animal monitoring system; CPT, carnitine palmitoyltransferase; CRISPR, clustered regularly interspaced short palindromic repeats; DAG, diacylglycerols; DAO, D-amino acid oxidase; DEG, differentially expressed genes; ECI, enoyl-CoA delta isomerase; FA, fatty acids; FAO, fatty acid β-oxidation; HADH, hydroxyacyl-Coenzyme A dehydrogenase; HS, hepatic steatosis; LDA, linear discriminant analysis; LEfSe, linear discriminant analysis effect size; MCP-1, monocyte chemoattractant protein-1; MetS, metabolic syndrome; NAFLD, nonalcoholic fatty liver disease; NASH, nonalcoholic steatohepatitis; NF-κB, nuclear factor kappa-light-chain-enhancer of activated B cells; NMR, nuclear magnetic resonance; OGTT, oral glucose tolerance test; ORO, Oil Red O; PGC1α, PPARG-coactivator-1α (PPARGC1A); PNPLA, patatin-like phospholipase domain-containing protein; PPAR, peroxisome proliferator-activated receptor; RER, respiratory exchange ratio; SARDH, sarcosine dehydrogenase; SERPINE, serine peptidase inhibitor clade E; SHMT, serine hydroxymethyltransferase; T2D, type 2 diabetes; TC, total cholesterol; TDH, threonine dehydrogenase; TG, triglycerides; TGFBR, transforming growth factor beta receptor; TGFβ, transforming growth factor-β; TIMP, tissue inhibitor of metalloproteinase; TLR, toll-like receptor; TNF, tumor necrosis factor; TNFRSF, TNF receptor superfamily; WD, Western diet.

### C. Transcript Profiling:

RNA-sequencing data have been deposited in NCBI's SRA or GEO (accession numbers: PRJNA556537 or GSE126204). Metagenomics sequencing data have been deposited in NCBI's SRA (accession number: PRJNA544728).

### D. Background & Aims:

The prevalence of nonalcoholic fatty liver disease (NAFLD) including steatohepatitis (NASH) is increasing worldwide with no pharmacological therapy approved. Lower circulating glycine is consistently reported in NAFLD patients, but a causative role of glycine and its therapeutic potential remain unclear. We applied genetic and dietary strategies to study glycine metabolism in NAFLD and evaluated glycine-based treatments.

### E. METHODS:

We performed transcriptomics in livers from humans and mice with NAFLD, uncovering suppression of glycine biosynthetic genes, predominantly alanine-glyoxylate aminotransferase-1 (AGXT1). We generated AGXT1^{-/-} mice using CRISPR/Cas9 and developed glycine-modified diets. We searched for glycine-based compounds with glucose/lipid-lowering properties and tested therapeutic use in murine models of hyperlipidemia/NAFLD. Plasma lipids, liver enzymes and cytokines were analyzed. Livers were studied using histology, lipid quantification, RNA-sequencing, qPCR and immunoblotting. NMR-based body composition and energy metabolism were assessed using indirect calorimetry. Gut microbiota was studied using 16S metagenomic sequencing.

### F. RESULTS:

Genetic (AGXT1^{-/-}) and dietary approaches to limit glycine availability exacerbated diet-induced hyperlipidemia and steatohepatitis with suppressed mitochondrial/peroxisomal fatty acid β-oxidation (FAO) and enhanced inflammation as underlying pathways. We identified a glycine-based tripeptide (gly-gly-L-leu/DT-109) with potent glucose/lipid-lowering effects. In mice with established NASH, DT-109 improved body composition, lowered circulating lipids, liver enzymes and steatohepatitis by stimulating FAO pathways. DT-109 reduced lobular/systemic inflammation and fibrosis by suppressing NF-κB and TGFβ/SMAD pathways. The bacterial genera Clostridium sensu stricto positively correlated with NAFLD severity and was decreased by DT-109, while Alistipes showed inverse correlations and was increased by DT-109.

### G. METHODS

### (i) Animal Procedures

Animal procedures were approved (PRO00008239) by the Institutional Animal Care & Use Committee of the University of Michigan (U-M) and performed in accordance with the institutional guidelines. Seven weeks-old C57BL/6J or apoE-/- mice (B6.129P2-Apoetm1Unc/J, Stock:002052) were from Jackson Laboratories. AGXT1-/- mice on C57BL/6J background were generated using CRISPR/Cas9, with guide-RNA targeting exon 1 of AGXT1: 5'-GGGTCCGGGGCCCTCCAACC-3'. Eight-week old male C57BL/6J, apoE-/- or AGXT1-/- mice were used throughout, and fed ad libitum: standard chow diet (CD, LabDiet 5L0D, 13% of calories from fat); high-fat, high-cholesterol Western-diet (WD, Envigo TD.88137, 42% fat); AA-defined WD with or without glycine (developed with Envigo-Teklad Custom Diets, Table S2): WDAA+Gly (TD.170525) or WDAA-Gly (TD.170526); high-fat, high-cholesterol, high-fructose NASH-diet (Research Diets D17010103, 40% fat), that we confirmed to potently induce NASH in mice. When indicated, mice were orally gavaged with glycine (Sigma-Aldrich G5417), leucine (Sigma-Aldrich L8912), or DT-109 (CSBio) at 0.125-1 mg/g body weight/day or H₂O as control.

**Table S2. Composition of the amino acid-defined WD with or without glycine (WD_{AA} +/-Gly)**

| **Selected nutrient information** | **CD** | **WD_{AA} +Gly** | **WD_{AA} -Gly** |
|---|---|---|---|
| Protein (% of kcal) | 28.2 | 22.4 | 22.5 |
| Carbohydrates (% of kcal) | 59.1 | 35.4 | 35.2 |
| Fat (% of kcal) | 12.6 | 42.2 | 42.3 |
| Calorie density (kcal/g) | 3.3 | 4.5 | 4.5 |
| Sucrose (g/kg) | 150.0 | 345.5 | 345.5 |
| Maltodextrin (g/kg) | 100.0 | 20.0 | 20.2 |
| Anhydrous milkfat (g/kg) | 37.2 | 210.0 | 210.0 |
| Cholesterol (g/kg) | 0 | 1.5 | 1.5 |
| L-Alanine (g/kg) | 14.4 | 14.4 | 14.4 |
| L-Arginine (g/kg) | 15.7 | 15.7 | 15.7 |
| L-Asparagine (g/kg) | 5.3 | 5.3 | 8.1 |
| L-Aspartate (g/kg) | 28.1 | 28.1 | 33.8 |
| L-Cysteine (g/kg) | 3.9 | 3.9 | 3.9 |
| L-Glutamate (g/kg) | 47.4 | 47.4 | 47.7 |
| L-Glutamine (g/kg) | 28.7 | 28.7 | 31.8 |
| **Glycine (g/kg)** | **12.8** | **12.8** | **0** |
| L-Histidine (g/kg) | 8.4 | 8.4 | 8.4 |
| L-Isoleucine (g/kg) | 10.6 | 10.6 | 10.6 |
| L-Leucine (g/kg) | 18.9 | 18.9 | 18.9 |
| L-Lysine (g/kg) | 18.5 | 18.5 | 18.5 |
| L-Methionine (g/kg) | 5.9 | 5.9 | 5.9 |
| L-Phenylalanine (g/kg) | 11.1 | 11.1 | 11.1 |
| L-Proline (g/kg) | 14.7 | 14.7 | 19.6 |
| L-Serine (g/kg) | 11.8 | 11.8 | 11.8 |
| L-Threonine (g/kg) | 9.7 | 9.7 | 9.7 |
| L-Tryptophan (g/kg) | 2.8 | 2.8 | 2.8 |
| L-Tyrosine (g/kg) | 7.7 | 7.7 | 7.7 |
| L-Valine (g/kg) | 11.6 | 11.6 | 11.6 |
| Taurine (g/kg) | 0.3 | 0.3 | 0.3 |
| Cellulose (g/kg) | 50.0 | 50.0 | 50.0 |
| Mineral mix (79055) (g/kg) | 13.4 | 13.4 | 13.4 |
| Calcium phosphate (g/kg) | 21.8 | 21.8 | 21.8 |
| Calcium carbonate (g/kg) | 8.3 | 8.3 | 8.3 |
| Vitamin Mix (40060) | 10.0 | 10.0 | 10.0 |

### (ii) Human Data

Differentially expressed genes (DEG) driving glycine biosynthesis were analyzed using two public liver microarray data-sets: 1) GSE83452, from 104 NASH patients and 44 heathy controls, 2) GSE61260, from 24 NASH patients and 24 healthy obese controls. We used a linear regression model with age, sex, and BMI as covariates to identify significant glycine biosynthetic genes that are associated with NASH. To increase statistical power, we further performed a meta-analysis of the two studies with a fixed effect model. Our previously published microarray data (GSE26106, n=206 liver transplantation donors), was used to test correlations between gene expression levels and hepatic fat content.

### (iii) Histology and Immunohistochemistry

All histological procedures were performed by U-M In Vivo Animal Core, Histology Laboratory. Technicians were blinded to experimental groups. H&E or Sirius Red staining were used to score NAFLD activity (NAS) or fibrosis.

### (iv) Identification of Glycine-based Compounds

Compounds structurally similar to glycine were chosen to evaluate structural, conformational, electronic and isosteric modifications to the glycine scaffold. Searches were conducted via SciFinder^{©} to determine commercial availability. Water-soluble compounds with oral LD₅₀>1 mg/g were defined as suitable for oral administration to mice.

### (v) RNA-Sequencing

Library preparation and sequencing were performed by the U-M DNA Sequencing Core on a NovaSeq 6000 Sequencing System (Illumina). RNA-sequencing and pathway analyses were performed as described.

### (vi) Comprehensive Laboratory Animal Monitoring System (CLAMS)

Body composition was assessed using nuclear magnetic resonance (NMR)-based analyzer (Minispec LF90II; Bruker Optics) at the U-M Animal Phenotyping Core. Oxygen consumption (VO₂), carbon dioxide production (VCO₂) and motor activity were measured using CLAMS (Columbus Instruments). Respiratory exchange ratio (RER) is VCO₂/VO₂.

### (vii) Fecal Microbiome Analysis

Fecal DNA extraction, amplification using primers specific to the V4 region of the 16S rRNA, characterization of the gut microbiota using linear discriminant analysis (LDA) effect size (LEfSe) method and analysis of correlations with disease parameters were performed as previously described.

### (viii) Statistical Analysis

Statistical analyses were performed using GraphPad Prism 7.0. Unless indicated otherwise, values are presented as mean±SD showing all points. All data were tested for normality and equal variance. If passed, Student t test was used to compare two groups or one-way ANOVA followed by Bonferroni post-hoc test for comparisons among >2 groups. Otherwise, nonparametric tests (Mann-Whitney U or Kruskal-Wallis) were used. P value <0.05 was considered statistically significant.

### (ix) Generation of AGXT1-/- mice using CRISPR/Cas9

AGXT1-/- mice in the C57BL/6J background were generated using CRISPR/Cas9. The guide RNA target site on exon 1 of the AGXT1 gene was 5'-GGGTCCGGGGCCCTCCAACC-3'. We performed genotyping using the following primers: forward: 5'-ACACCTCCACTGTCCTGTCC-3', reverse: 5'-GGTCAGATCTGCCTGCTACC-3'. Sanger sequencing using the following primer: 5'-GCAGAGCTAGCTGGGAAATG-3' confirmed A deletion 3 bases from the protospacer adjacent motif (PAM, FIG. 8A). The frame-shift mutation after AA 53 of the ORF, introduced a premature stop-codon, and the absence of AGXT1 was confirmed by Western blot (FIG. 8B). No CRISPR off-target effects were detected as assessed using CRISPOR.

### (x) Human Data

Differentially expressed glycine biosynthetic genes in NASH patients were analyzed using two public data sets. The first study consists of liver microarray data obtained from 104 NASH patients and 44 normal controls (GSE83452). Linear regression was used to test the association between gene expression levels and NASH. Age and sex were adjusted as covariates. The second data set includes liver microarray data collected from 24 NASH patients and 24 heathy obese controls (GSE61260). Similarly, we used the linear regression model with age, sex, and BMI as covariates to identify significant glycine biosynthetic genes that are associated with NASH. To increase the statistical power and compare the results, we further performed a meta-analysis of the two studies with a fixed effect model using the metafor R package. Genes with Benjamini-Hochberg adjusted p value < 0.05 and Cochran's Q heterogeneity test p value > 0.05 were considered as significant.

The correlations between gene expression levels and hepatic fat content were tested using our previously published microarray data (GSE26106) collected from liver transplantation donors (n=206). The tissue dissection, sample exclusion, and microarray data generation were performed as previously described. The hepatic fat contents of the donor livers were quantified using organic solvents (hexane/isopropanle 3:2), as described previously. The extracted total fat content was normalized to the total protein concentration and transformed to log10 scale for the subsequent analysis. Pearson correlation was used to determine the significance of the hepatic fat correlated with expression of glycine biosynthetic genes.

### (xi) Histology, Immunohistochemistry and Scoring of NAFLD Activity and Fibrosis

Histological procedures were performed by technicians blinded to the experimental groups at the In Vivo Animal Core (IVAC) Histology Laboratory, University of Michigan. Formalin-fixed tissues were processed through graded alcohols and cleared with xylene followed by infiltration with molten paraffin using an automated VIP5 or VIP6 tissue processor (TissueTek, Sakura-Americas). Using a Histostar Embedding Station (ThermoFisher Scientific), tissues were then sectioned on a M355S rotary microtome (ThermoFisher Scientific) at 4 µm thickness and mounted on glass slides. Slides were stained for hematoxylin and eosin (H&E, ThermoFisher Scientific). For Sirius Red staining, slides were treated with 0.2 phosphomolybdic acid for 3 min and transferred to 0.1% Sirius Red saturated in picric acid (Rowley Biochemical Inc.) for 90 min, then transferred to 0.01N hydrochloric acid for 3 min.

H&E staining was used to score NAFLD activity. Steatosis was scored from 0-3 (0: <5% steatosis; 1: 5-33%; 2: 34-66%; 3: >67%). Hepatocyte ballooning was scored from 0-2 (0: normal hepatocytes, 1: normal-sized with pale cytoplasm, 2: pale and enlarged hepatocytes, at least 2-fold). Lobular inflammation was scored from 0-2 based on foci of inflammation counted at 20X (0: none, 1: <2 foci; 2: ≥2 foci). NAFLD activity score (NAS) was calculated as the sum of steatosis, hepatocyte ballooning and lobular inflammation scores. Sirius Red staining was used to score hepatic fibrosis from 0-4 (0: no fibrosis; 1: perisinusoidal or portal fibrosis; 2: perisinusoidal and portal fibrosis; 3: bridging fibrosis; 4: cirrhosis).

Frozen section processing was used for Oil Red O (ORO) staining. Formalin-fixed liver samples were cryoprotected in 20% sucrose at 4oC overnight, blotted, then liquid nitrogen-snap frozen in O.C.T. Compound (Tissue-Tek, Cat #4583) and stored at -80oC until ready for cryosectioning. Prior to sectioning, frozen blocks were brought up to about -20oC, then sectioned at 5 µm on a Cryotome SME (Thermo-Shandon, Cat# 77200227). Slides were stored at -80oC until stained. Prior to staining, liver slides were thawed to room temperature for 30 min. Slides were post-fixed in 10% Neutral Buffered Formalin for 20 min, rinsed in DDW, followed by rinsing in 60% Isopropanol before being placed in working ORO-isopropanol stain (Rowley Biochemical Inc., H-503-1B) for 5 min. Slides were then rinsed in 60% Isopropanol followed by three changes of DDW. Then, slides were nuclear counterstained in Harris Hematoxylin and mounted in Aqua-Mount (Lerner Laboratories, Cat# 13800) aqueous mounting media.

Immunohistochemical staining was performed on a IntelliPATH FLX automated immunohistochemical stainer (Biocare Medical) with blocking for endogenous peroxidases and nonspecific binding, followed by detection using a horseradish peroxidase biotin-free polymer based commercial detection system, disclosure with diaminobenzidine chromogen, and nuclear counterstaining with hematoxylin. Specific to F4/80, (Bio-Rad ABD Serotec, Cat# MCA497), the rat monoclonal primary antibody (clone Cl:A3-1) was diluted to 1:400 in DaVinci Diluent (Biocare Medical, Cat# PD900) and incubated for 60 min followed by detection using Rat-on-Mouse HRP-Polymer, (Biocare Medical, Cat# RT517) 2-step probe-polymer incubation for 10 and 30 min respectively.

### (xii) Plasma Analyses

Complete plasma lipid profile (TC, TG, LDL and HDL) was measured with a Cobas Mira chemistry analyzer (Roche Diagnostics) at the Chemistry Laboratory of the Michigan Diabetes Research Center (MDRC) using manufacturer-provided reagents and protocols or at our lab, using commercially available kits (Wako Chemicals 999-02601 and 994-02891). Plasma glycine-containing tripeptide molecule and resistin were measured on a Luminex 200 platform (Luminex) at the MDRC using a Multiplex Assay (MMHMAG-44K, Millipore). Plasma MCP-1 was measured with the mouse CCL2/JE/MCP-1 Quantikine ELISA Kit (R&D Systems). Clinical chemistry assays for ALT, AST and ALP were performed by the University of Michigan IVAC on a Liasys 330 chemistry analyzer (AMS Diagnostics) using manufacturer-provided reagents and protocols. Plasma oxalate was measured using the Oxalate Assay kit (Abcam, ab196990). Plasma glucose was measured using glucometer and test strips (NDC: 0193-7308-50, Contour Next). Plasma AA analysis was performed by University of Michigan Metabolomics Core. Twenty µL of plasma were derivatized and prepared for GC-MS analysis according to the instructions of the EZ Faast Amino Acids Analysis kit (Phenomenex). Briefly, samples were subjected to column clean-up and transferred to a GC autosampler vial. Then, samples were derivatized, dried under a gentle nitrogen stream at room temperature, and re-suspended for GC analysis on an Agilent 69890N GC-5975 MS detector with the following parameters: 1 µL sample was injected with a 1:15 split ratio on an ZB-AAA 10m column (Phenomenex) with He gas flow rate of 1.1 mL/min. The GC oven initial temperature was 110°C and was increased by 30°C per minute to 320°C. The inlet temperature was 250°C and the MS-source and quad temperatures were 230° and 150°C respectively. Data were processed using MassHunter Quantitative analysis version B.07.00. Metabolites were normalized to the nearest isotope labeled internal standard and quantified using 2 replicated injections of 5 standards to create a linear calibration curve with accuracy better than 80% for each standard. Peak areas were used for differential analysis between groups.

### (xiii) Quantitative Real-time PCR Analysis

Total RNA from mouse liver samples was extracted using QIAGEN's RNeasy kits (QIAGEN). RNA was reverse-transcribed into cDNA with SuperScript III and random primers (Invitrogen). Specific transcript levels were assessed by a real-time PCR system (Bio-Rad) using iQ SYBR Green Supermix (Bio-Rad) and the ΔΔCt threshold cycle method of normalization. Gene expression levels were normalized to glyceraldehyde 3-phosphate dehydrogenase (GAPDH). Primer pairs used for qPCR were obtained from Integrated DNA Technologies and are listed in Table S3.

**Table S3. Primers used for qPCR analyses**

| **Gene** | **Forward primer** | **SEQ ID NO** | **Revers primer** | **SEQ ID NO** |
|---|---|---|---|---|
| **Mus musculus** | | | | |
| Alanine-glyoxylate aminotransferase (AGXT1) | AAGGCATCCAGTATGTG TTCCA | 73 | TTCCGGTTAGAAAGGA GTCCC | 74 |
| Alanine-glyoxylate aminotransferase 2 (AGXT2) | TCACCTGAGAAATACCA GTCCC | 75 | CAAAGAGCCACTCCAT GTGTC | 76 |
| Serine hydroxymethyltransferase 1 (SHMT1) | GGATGATAATGGGGCG TATCTCA | 77 | GTCTTGTGGGTTGTAG TGGTC | 78 |
| Serine hydroxymethyltransferase 2 (SHMT2) | ATGCCCTATAAGCTCAA TCCCC | 79 | TCTCATGCGTGCATAG TCAATG | 80 |
| Choline dehydrogenase (CHDH) | TGAGCTGGGTGCCAAT ATGTA | 81 | CGAAGCCCTCCTGTTG GAA | 82 |
| Sarcosine dehydrogenase (SARDH) | GAGAGCGACTGACCTC TGG | 83 | CCGTGTGTGAGCCAAA AGC | 84 |
| Threonine dehydrogenase (TDH) | CTGGCTGTTTCACTACA GTGC | 85 | GGAGAGGTAGGTCCA AAGGC | 86 |
| Peroxisome proliferator activated receptor alpha (PPARα) | AACATCGAGTGTCGAAT ATGTGG | 87 | CCGAATAGTTCGCCGA AAGAA | 88 |
| Acyl-Coenzyme A oxidase 1 (ACOX1) | CCGCCACCTTCAATCCA GAG | 89 | CAAGTTCTCGATTTCT CGACGG | 90 |
| Hydroxyacyl-Coenzyme A dehydrogenase alpha (HADHA) | ACCTCGGTGTAAAGCA CAAAGT | 91 | GAGGTTTTGTCAGTGG TGATGA | 92 |
| Acetyl-Coenzyme A acyltransferase 2 (ACAA2) | AGTCCCCCTACTGTGTC AGAAA | 93 | CCATCTCCTCATTGAA GTAGCC | 94 |
| Enoyl-Coenzyme A delta isomerase 1 (ECI1) | TGCTGTGACTACAGGG TTATGG | 95 | GATCCTCAGGTACCAC CTCATC | 96 |
| Enoyl-Coenzyme A delta isomerase 2 (ECI2) | ACTACTGCAGTGGGAAT GACCT | 97 | ATAGTCCCAGAAGGGT GACAGA | 98 |
| Acyl-CoA Synthetase Long Chain 1 (ACSL1) | GAGCAATGATCACTCAC CAAAA | 99 | TCTTAGCTCCATGACA CAGCAT | 100 |
| Acyl-CoA thioesterase 3 (ACOT3) | GCTCAGTCACCCTCAG GTAA | 101 | AAGTTTCCGCCGATGT TGGA | 102 |
| Acyl-CoA thioesterase 4 (ACOT4) | ACATCCAAAGGTAAAAG GCCCA | 103 | TCCACTGAATGCAGAG CCATT | 104 |
| Nudix-type motif 19 (NUDT19) | GCACCACCACAGTTCTA TGAAA | 105 | TAAGGACTTTGCCTTC CTTCAC | 106 |
| Acyl-CoA synthetase medium-chain 5 (ACSM5) | TGACAGCGAAGGATCT CAAGTA | 107 | GAGTTCTCGGAAATTG ATCCAG | 108 |
| Nuclear factor of kappa light polypeptide gene enhancer in B cells 1, p105 (NFBK1) | GGTATGGCTACTCGAA CTACGG | 109 | TTTCCTTCTCAGGGAG AGTCAG | 110 |
| Nuclear factor of kappa light polypeptide gene enhancer in B cells 2, p49/p100 (NFKB2) | GAGGTTCGGTTCTATGA GGATG | 111 | CTCTGCACTTCCTCCT TGTCTT | 112 |
| Avian reticuloendotheliosis viral (v-rel) oncogene related B (RELB) | ATCCTCTCTGAGCCTGT CTACG | 113 | CACATCAGCTTGAGAG AAGTCG | 114 |
| Tumor necrosis factor (TNFα) | CTGTGAAGGGAATGGG TGTT | 115 | GGTCACTGTCCCAGCA TCTT | 116 |
| Tumor necrosis factor receptor superfamily 9 (TNFRSF9) | GTTTTGCTCCTCTACCC ACAAC | 117 | CTTAAGCACAGACCTT CCGTCT | 118 |
| C-C motif chemokine ligand 2 (CCL2) | TTAAAAACCTGGATCGG AACCAA | 119 | GCATTAGCTTCAGATT TACGGGT | 120 |
| C-C motif chemokine ligand 5 (CCL5) | ATATGGCTCGGACACC ACTC | 121 | CCACTTCTTCTCTGGG TTGG | 122 |
| C-C motif chemokine receptor 2 (CCR2) | GATGATGGTGAGCCTT GTCATA | 123 | AGTGAGCCCAGAATGG TAATGT | 124 |
| C-C motif chemokine receptor 5 (CCR5) | ACCCTGTCATCTATGCC TTTGT | 125 | GTGGATCGGGTATAGA CTGAGC | 126 |
| Toll-like receptor 2 (TLR2) | CGGCTGCAAGAGCTCT ATATTT | 127 | TGGCGTCTCCATAGTA AAGGAT | 128 |
| Toll-like receptor 4 (TLR4) | CAGCACTCTTGATTGCA GTTTC | 129 | CATTCACCAAGAACTG CTTCTG | 130 |
| CD86 Antigen (CD86) | TCTTGCTGATCTCAGAT GCTGT | 131 | CGTACAGAACCAACTT TTGCTG | 132 |
| Transforming growth factor, beta 1 (TGFB1) | TGCGCTTGCAGAGATTA AAA | 133 | CTGCCGTACAACTCCA GTGA | 134 |
| Transforming growth factor, beta 2 (TGFB2) | CTAATGTTGTTGCCCTC CTACAG | 135 | GCACAGAAGTTAGCAT TGTACCC | 136 |
| Transforming growth factor, beta receptor 1 (TGFBR1) | GGACCATTGTGTTACAA GAAAGC | 137 | CATGGCGTAACATTAC AGTCTGA | 138 |
| Transforming growth factor, beta receptor 2 (TGFBR2) | TCCTAGTGAAGAACGAC TTGACC | 139 | TACCAGAGCCATGGAG TAGACAT | 140 |
| Collagen, type I, alpha 1 (COL1A1) | TGAACGTGACCAAAAAC CAA | 141 | GCAGAAAAGGCAGCAT TAGG | 142 |
| Collagen, type I, alpha 2 (COL1A2) | AGGCAGGTCTGGGCTT TATT | 143 | CGTATCCACAAAGCTG AGCA | 144 |
| Collagen, type III, alpha 1 (COL3A1) | CTGTGAATCATGTCCAA CTGGT | 145 | GATCCAGGATGTCCAG AAGAAC | 146 |
| Collagen, type IV, alpha 1 (COL4A1) | CAGGCATAGTCAGACA ACAGATG | 147 | TGGACAGCCAGTAAGA GTAGTCG | 148 |
| Collagen, type IV, alpha 2 (COL4A2) | CCCATCTGACATCACAC TTGTT | 149 | CCTCTGCTTCCTTTCT GTCCTA | 150 |
| Tissue inhibitor of metalloproteinase 1 (TIMP1) | ATTCAAGGCTGTGGGA AATG | 151 | CTCAGAGTACGCCAGG GAAC | 152 |
| Tissue inhibitor of metalloproteinase 2 (TIMP2) | AGAAGAAGAGCCTGAA CCACAG | 153 | GGTCCTCGATGTCAAG AAACTC | 154 |
| Serine (or cysteine) peptidase inhibitor, clade E, member 1 (SERPINE1) | GTAGCACAGGCACTGC AAAA | 155 | ATCACTTGGCCCATGA AGAG | 156 |
| Peroxisome proliferative activated receptor, gamma, coactivator 1 alpha (PGC1α) | ATCACGTTCAAGGTCAC CCTAC | 157 | TTCTGCTTCTGCCTCT CTCTCT | 158 |
| Acyl-Coenzyme A dehydrogenase, medium chain (ACADM) | CTGTGATTCTTGCTGGA AATGA | 159 | GCCGTTGATAACATAC TCGTCA | 160 |
| Acyl-Coenzyme A dehydrogenase, long chain (ACADL) | CTATATTGCGAATTACG GCACA | 161 | ACACCTTGCTTCCATT GAGAAT | 162 |
| Acyl-Coenzyme A dehydrogenase, very long chain (ACADVL) | GCAGATGAGTGCATCC AAATAA | 163 | TGAGTTCCTTTCCTTTG TCCAT | 164 |
| Hydroxyacyl-Coenzyme A dehydrogenase (HADH) | AACACGTCTTCTTTGCA GATCA | 165 | AATGAGGTATGGCACC AAGAGT | 166 |
| Cytochrome P450, family 4, subfamily a, polypeptide 14 (CYP4A14) | TATGTCCTCTGATGGAC GTTTG | 167 | CTGTTCCTATCCTCCA TTCTGG | 168 |
| Mitochondrial carnitine/acylcarnitine translocase (CACT) | CAACCACCAAGTTTGTC TGGA | 169 | CCCTCTCTCATAAGAG TCTTCCG | 170 |
| Carnitine palmitoyltransferase 1a (CPT1a) | AGATCAATCGGACCCTA GACAC | 171 | CAGCGAGTAGCGCATA GTCA | 172 |
| Glyceraldehyde-3-phosphate dehydrogenase (GAPDH) | CTGCGACTTCAACAGCA ACT | 173 | GAGTTGGGATAGGGC CTCTC | 174 |

| **Homo sapiens** | | | | |
|---|---|---|---|---|
| Alanine-glyoxylate aminotransferase (AGXT1) | AGAGACATCGTCAGCTA CGTCA | 175 | CAGGTCACAGCTTCTT CTTGG | 176 |
| Glyceraldehyde-3-phosphate dehydrogenase (GAPDH) | ACAACTTTGGTATCGTG GAAGG | 177 | GCCATCACGCCACAGT TTC | 178 |

### (xiv) RNA-Sequencing and Data Analysis

Total RNA from mouse liver samples was extracted as described above. Library preparation and sequencing were performed by the University of Michigan DNA Sequencing Core. RNA was assessed for quality using the TapeStation (Agilent, Santa Clara, CA). All samples had RNA integrity numbers (RINs) > 8.5. Samples were prepped using the NEBNext Ultra II Directional RNA Library Prep Kit for Illumina (NEB, E7760L) with Poly(A) mRNA Magnetic Isolation Module (NEB, E7490L) and NEBNext Multiplex Oligos for Illumina Unique dual (NEB, E6440L), where 10 ng - 1 µg of total RNA were subjected to mRNA polyA purification. The mRNA was then fragmented and copied into first strand cDNA using reverse transcriptase and dUTP mix. Samples underwent end repair and dA-Tailing step followed by ligation of NEBNext adapters. The products were purified and enriched by PCR to create the final cDNA library. Final libraries were checked for quality and quantity by TapeStation (Agilent) and qPCR using Kapa's library quantification kit for Illumina Sequencing platforms (Kapa Biosystems, KK4835). Libraries were paired-end sequenced on a NovaSeq 6000 Sequencing System (Illumina).

The quality of the raw Fastq files was checked through FastQC v0.11.8 (https://www.bioinformatics.babraham.ac.uk/projects/fastqc/). Trimmomatic v.0.35 was used to trim the low-quality reads with the parameters: SLIDINGWINDOW:4:20 MINLEN:25.12 The resulted high-quality reads were then mapped to the mouse reference genome (GRCm38.90) using HISAT2 v.2.1.0.13 Gene level quantification was performed using HTSeq-counts v0.6.0 based on the GRCm38.90 genome annotations.14 The R package DESeq2 was then used to identify significant differentially expressed genes.15 We considered genes with adjusted P value less than 0.05 and absolute fold change larger than 2 as significant differentially expressed genes (DEGs). The up- and down-regulated DEGs were then analyzed respectively for significantly enriched KEGG pathways using the clusterProfiler package.16 The significance of the enrichment was determined by right-tailed Fisher's exact test followed by Benjamini-Hochberg multiple testing adjustment.

### (xv) Hepatic Lipid Analysis

Livers were rapidly removed from the euthanized mice, snap-frozen with liquid nitrogen, and kept at -80°C. Frozen liver samples (100 mg) were homogenized in PBS and centrifuged (14,000 RPM, 20 min). The supernatants were collected and analyzed for protein content using Bio-Rad Bradford assay. To assess liver lipid composition, lipids were extracted from the supernatants using hexane (≥99%, Sigma-Aldrich 32293) and isopropanol (≥99.5%, Fisher Scientific A426-4) at a 3:2 ratio (v:v), and the hexane phase was left for evaporation for 48 h. The amount of liver TG or TC was determined spectrophotometrically using commercially available kits (Wako Chemicals 999-02601 and 994-02891). Liver diacylglycerols (DAG) were determined using ELISA Kit (Aviva Systems Biology, OKEH02607), according to the manufacturer's instructions. Hepatic TG, TC and DAG data were normalized to protein levels.

### (xvi) Western Blot Analysis

Liver samples were lysed in radioimmunoprecipitation assay lysis buffer (RIPA buffer, Thermo Scientific) supplemented with a protease/phosphatase inhibitor cocktail (Roche Applied Science). Proteins were resolved in 10% sodium dodecyl sulfate polyacrylamide gel electrophoresis (SDS-PAGE) and transferred to nitrocellulose membranes (Bio-Rad). The membranes were blocked for 1 h at room temperature in tris-buffered saline-Tween 20 (TBST) containing 5% fat-free milk and incubated with primary antibody at 4°C overnight. The following primary antibodies were used: AGXT1 (Santa Cruz Biotechnology sc-517388, 1:500), HADHA (Proteintech 10758-1-AP, 1:1000), ACAA2 (ABclonal A15778, 1:500), phospho-SMAD2 (Ser465/467, Cell Signaling, #3108S; 1:1000), SMAD2 (Cell Signaling Technology, #5339S; 1:1000), GAPDH (Santa Cruz Biotechnology sc-365062; 1:2000), or β-Actin (Cell Signaling Technology #3700). The membranes were washed with TBST before incubated with IRDye-conjugated secondary antibodies (LI-COR Biosciences, 1:10000) at room temperature for 1 h. After TBST washing, bands were visualized and quantified using an Odyssey Infrared Imaging System (LI-COR Biosciences, version 2.1).

### (xvii) Identification of Glycine-based Compounds

Compounds structurally similar to glycine were chosen with the objective of evaluating structural, conformational, electronic and isosteric modifications to the glycine scaffold (FIG. 9A). Searches were conducted via SciFinder^{©} to determine commercial availability. N-methylglycine (FIG. 9B), N,N-dimethylglycine (FIG. 9C), and N,N,N-trimethylglycine (FIG. 9D) explored various degrees of methylation on the glycine amine moiety and glycolic acid (FIG. 9E) evaluated an amine to alcohol substitution. Modifications to the acid region Wx

ere explored by glycinamide (FIG. 9F), 2-amino-N-methylacetamide (FIG. 9G), and ethanolamine (FIG. 9H), while 2-oxopiperazine (FIG. 9I) and morpholin-2-one (FIG. 9J) explored conformationally restricted analogues. (1H-tetrazol-5-yl) methanamine (FIG. 9K) explored the isosteric replacement of the acid.

### (xviii) Oral Glucose Tolerance Tests (OGTT)

OGTT were performed after 12 h fasting. Blood samples were taken from the tail tip at 0, 15, 30, 60 and 120 min after oral gavage of glucose (2 mg/g body weight) with or without leucine, glycine, DT-109 or other glycine-based compounds at the indicated dosages (0.17-1 mg/g body weight). Blood glucose concentrations were measured using a blood glucometer and test strips (Contour Next).

### (xix) Body Composition and Comprehensive Laboratory Animal Monitoring System (CLAMS)

All measurements were performed by the University of Michigan Animal Phenotyping Core. Body fat, lean body mass, and free fluids were measured using a nuclear magnetic resonance (NMR)-based analyzer (Minispec LF90II; Bruker Optics). The analyzer is daily checked using a reference sample as recommended by the manufacture. The mice were placed individually into the measuring tube with minimum restrain. Oxygen consumption (VO2), carbon dioxide production (VCO2) and motor activity were measured using CLAMS (Columbus Instruments), an integrated open-circuit calorimeter equipped with an optical beam activity monitoring device. Mice were weighed before the measurements and individually placed into the sealed chambers (7.9" x 4" x 5") with free access to food and water. The study was carried out in an experimentation room set at 20-23°C with 12-12 h dark-light cycles (6:00PM~6:00AM). Measurements were carried out continuously for 48 h. During this time, animals were provided with food and water through the feeding and drinking devices located inside the chamber. VO2 and VCO2 in each chamber were sampled sequentially for 5s in 10 min intervals and the motor activity was recorded every second in X and Z dimensions. The air flow rate through the chambers was adjusted to keep the oxygen differential around 0.3% at resting conditions. Respiratory exchange ratio (RER) was calculated as VCO2/VO2. Total energy expenditure, glucose oxidation and fat oxidation were calculated based on the values of VO2, VCO2, and the protein breakdown.

### (xx) Fecal Microbiome Analysis

Total genomic DNA of the gut microbiota was extracted from fecal samples using the QIAamp DNA Stool Mini Kit (51540, Qiagen) according to the manufacturer's instructions. DNA was prepared for community analysis as previously described.17 Briefly, barcoded dual-index primers specific to the V4 region of the 16S rRNA gene were used to amplify the DNA.18 PCR reactions were composed of 5 µL of 4 µM equimolar primer set, 0.15 µL of AccuPrime Taq DNA High Fidelity Polymerase, 2 µL of 10 x AccuPrime PCR Buffer II (Thermo Fisher Scientific 12346094), 11.85 µL of PCR-grade water, and 1 µL of DNA template. The PCR conditions were 2 min at 95°C, followed by 30 cycles of 95°C for 20 s, 55°C for 15 s, and 72°C for 5 min, followed by 72°C for 10 min. Each PCR reaction was normalized using the SequalPrep Normalization Plate Kit (Thermo Fisher Scientific A1051001). The normalized reactions were pooled and quantified using the Kapa Biosystems Library qPCR MasterMix (ROX Low) Quantification kit for Illumina platforms (KK4873). The Agilent Bioanalyzer was used to confirm the size of the amplicon library (~399 bp) using a high-sensitive DNA analysis kit (5067-4626). Pooled amplicon library was then sequenced on the Illumina MiSeq NANO platform (Microbial Systems Molecular Biology Laboratory, University of Michigan) according to standard protocols.

DNA sequencing data was processed using Mothur according to SOP and focused primarily on α - and β - diversity based analyses. The sequences were trimmed to remove primers and barcodes, quality filtered, and chimera checked as previously described. A total of 3565 sequences in each sample were used for further statistical analysis. The trimmed DNA sequences were clustered using the average neighbor approach to form operational taxonomic units (OTUs) at 97% sequence similarity cutoff (3% sequence divergence). Phylogenetic trees were constructed using the Clearcut program. An OTU-based approach was used for the beta diversity measurements. A heatmap of the relative abundance of each OTU across all samples was generated using log2 scaling of the relative abundance values of the top 107 OTUs (> 1%). Molecular AMOVA statistical analysis was performed to determine significance of structural similarity among communities across sampling groups. The UniFrac analysis was used to estimate weighted (WUnF) and unweighted (UWUnF) UniFrac metrics. Constrained ordination RDA (redundancy analysis) was calculated, and the significance of environmental variables was checked by forward selection analysis. The characterization of microorganismal features differentiating the gut microbiota was performed using the linear discriminant analysis (LDA) effect size (LEfSe) method (http://huttenhower.sph.harvard.edu/lefse/) for biomarker discovery, which emphasizes both statistical significance and biological relevance. The principal component analysis (PCA) was plotted accordingly using Phyloseq package in R. The correlations between changes in the altered bacterial genera and NAFLD-related parameters in the liver or plasma were calculated using nonparametric Spearman's test.

### (xxi) In vitro Studies

The HepG2 human hepatoma cell line was obtained from the American Type Culture Collection (ATCC) and cultured at 37°C and 5% CO2 in Dulbecco's Modified Eagle Medium (DMEM, Gibco) supplemented with 10% fetal bovine serum (FBS, Sigma-Aldrich) and 1% Penicillin-Streptomycin (Pen-Strep, Gibco). In some experiments, the cells were loaded with palmitic acid (PA, 200 µM, Sigma-Aldrich P0500) in DMEM without FBS, but supplemented with 0.1% BSA. siRNA targeting AGXT1 (siAGXT1: GCAAGGAUAUGUACCAGAUtt, siRNA ID s1190) and non-targeting siRNA control (siCTL, siRNA ID AM4611) were obtained from Ambion. HepG2 cells were transfected with 20 nM of siAGXT1 or siCTL using Lipofectamine RNAiMAX (Invitrogen) in Opti-MEM reduced-serum medium (Gibco) in accordance with the manufacturer's protocol. RNA isolation, protein or lipid extraction were conducted 48 h post transfection. Total RNA was purified from HepG2 cells using the QIAGEN's RNeasy kit (QIAGEN). qPCR analysis was performed as described above using human primer pairs listed in Table S3. Cells were lysed using RIPA buffer (Thermo Scientific) supplemented with a protease/phosphatase inhibitor cocktail (Roche Applied Science). AGXT1 protein abundance was assessed using Western blot, as described above. Extraction of cellular lipids from the cells was done using hexane:isopropanol at a 3:2 ratio and the hexane phase was left to evaporate for 48 h. The remaining cells in the plates were disrupted in 0.1 M NaOH for 24 h and an aliquot was taken for measurement of cellular protein using the Bradford protein assay (Bio-Rad). The content of cellular TG or TC was determined spectrophotometrically using commercially available kits (Wako Chemicals). Cellular TG and TC data were normalized to protein levels and presented as µg TG or TC / mg protein.

### H. Results

### (i) Impaired Glycine Biosynthesis in Murine and Human NAFLD

To test whether altered glycine metabolism contributes to NAFLD development, we first studied C57BL/6J with WD-induced HS. After 12 weeks of WD feeding, hypercholesterolemia (FIG. 10A) and HS verified by H&E and Oil Red O (ORO) staining and quantification of hepatic TG and total cholesterol (TC) were evident (FIG. 10B-D). Targeted metabolomics revealed that among all AA, plasma glycine was most significantly reduced while its precursors serine, threonine and alanine were markedly increased (FIG. 10E), indicating impaired glycine biosynthesis. Accordingly, we next studied the expression of genes driving glycine formation. We found that key glycine biosynthetic genes were downregulated in mice with HS, among which, AGXT1 was most significantly suppressed (FIG. 10F). Additionally, AGXT1 was markedly downregulated in HepG2 cells with palmitic acid (PA)-induced TG accumulation (FIG. 10G,H).

To test whether a similar pattern is evident in more severe NAFLD, we performed RNA-sequencing of livers from mice with advanced NASH and fibrosis induced by 24 weeks of NASH-diet feeding (FIG. 10I). Pathway analysis revealed alternations in known pathways implicated in NASH, including upregulation of chemokine, NF-κB, toll-like receptor (TLR) and transforming growth factor-β (TGFβ) signaling, and downregulation of FA degradation and peroxisome proliferator-activated receptor (PPAR) signaling (FIG. 10J). Interestingly, pathways regulating AA biosynthesis, including metabolism of glycine, serine, threonine and glyoxylate were significantly downregulated in NASH, including AGXT1 suppression (*P*=0.0009, FIG. 10K). Using an independent cohort of mice with diet-induced NASH, AGXT1 suppression was confirmed by qPCR (FIG. 10L).

To test whether glycine biosynthetic genes are similarly suppressed in human NAFLD, we performed a meta-analysis based on transcriptomics of livers from NASH patients. We found that AGXT1 (beta=-0.141, *P*=0.0041) and AGXT2 (beta=-0.134, *P*=0.0135) were significantly downregulated in NASH, whereas D-amino acid oxidase (DAO) which catalyzes glycine degradation to glyoxylate, was markedly upregulated (beta=0.216, *P*=0.0025, FIG. 10M). Among 206 samples obtained from liver transplantation donors, we found that AGXT1 expression was inversely correlated with hepatic fat content (*r*=-0.199, *P*=0.0044, FIG. 10N). Interestingly, a similar inverse correlation was found between the expression of PPARα (master regulator of hepatic FAO) and hepatic fat content (*r*=-0.154, *P*=0.0275), whereas expression of C-C motif chemokine ligand 5 (CCL5) and TGFβ, key players in steatohepatitis and fibrosis, was positively correlated with hepatic fat (*r=*0.185, *P*=0.0078 and *r*=0.284, *P*<0.0001, respectively, not shown). These results are in line with recent reports of lower circulating glycine in NAFLD, in relation to suppression of glycine biosynthetic genes, and suggest a role for AGXT1 in NAFLD.

### (ii) Loss of AGXT1 Exacerbates Diet-induced Hyperlipidemia and NASH

To study a potential role of AGXT1, a liver-specific gene localized to the peroxisome or mitochondria of hepatocytes, in cellular lipid accumulation, we knocked-down AGXT1 in HepG2 cells, which enhanced PA-induced TG accumulation (FIG. 8C-E). To study the effects of loss of AGXT1 *in vivo,* we generated AGXT1^{-/-} mice using CRISPR/Cas9 (FIG. 8A, B). Under CD feeding, AGXT1^{-/-} liver histology was comparable to that of AGXT1^{+/+}, as previously indicated, and we additionally found that plasma liver enzymes were also comparable (FIG. 8F-K). However, after 12 weeks on NASH-diet, AGXT1⁻¹⁻ mice had increased plasma TG, TC, AST and ALT (FIG. 8L-O), while the ratio of glycine to oxalate significantly decreased (FIG. 8P). Gross appearance of the peritoneal cavities revealed larger livers with enhanced yellowish coloration in AGXT1^{-/-} mice (FIG. 11A). While no significant differences were noted in body weight, liver weight was significantly increased (FIG. 11B, FIG. 11C,D). H&E and ORO staining combined with quantification of hepatic lipids revealed increased HS in AGXT1^{-/-} mice (FIG. ,11A,E,F), and Sirius Red staining uncovered increased fibrosis (FIG. 11A). Further histological analysis confirmed higher NAS and fibrosis score in AGXT1-/- mice (FIG. 11G,H, FIG. 11I).

To understand the underlying mechanisms of accelerated diet-induced NASH in AGXT1^{-/-} mice, we performed RNA-sequencing of livers from AGXT1^{+/+} and AGXT1^{-/-} mice followed by qPCR validations. Pathway analysis revealed suppression of energy metabolism and FAO pathways in AGXT1^{-/-} mice, while pro-inflammatory pathways were upregulated (FIG. 11J). Genes regulating peroxisomal (acyl-CoA thioesterase-3, ACOT3, acyl-CoA synthetase medium-chain-5, ACSM5, acyl-CoA synthetase long chain-1, ACSL1,) and mitochondrial FAO (hydroxyacyl-CoA dehydrogenase-alpha, HADHA, and acetyl-CoA acyltransferase-2, ACAA2) were significantly downregulated in AGXT1^{-/-} mice (FIG. 11K-M, albeit marginally for PPARα, *P*=0.0557), whereas genes encoding for regulators of pro-inflammatory signaling (NFKB1/2, RELB, CCR2/5, and TLR2/4), cytokines (TNFα and CCL2), fibrogenesis (TGFB1/2 and TGFBR2) and extracellular matrix (ECM) remodeling (COL1A2, COL4A2 and TIMP1) were significantly upregulated (FIG. 11N,O). Thus, AGXT1, a liver-specific glycine biosynthetic gene, is suppressed in NAFLD and its deficiency accelerates diet-induced NASH.

### (iii) Glycine Deficiency Exacerbates Diet-induced Hyperlipidemia and HS

Lower plasma glycine levels are associated with NAFLD and cardiometabolic diseases, while higher levels are associated with a favorable lipid profile. To investigate a role for dietary glycine in dyslipidemia, we developed AA-modified WD with or without glycine (WD_{AA}+Gly or WD_{AA}-Gly, Table S2) and fed hyperlipidemic apoE^{-/-} mice CD, WD_{AA}+Gly or WD_{AA} -Gly for 10 weeks. A significant decrease in plasma glycine was found in mice fed WD_{AA} -Gly (FIG. 12A). NMR-based body composition and CLAMS analyses indicated increased body weight and fat in mice fed WD_{AA} -Gly, but not in mice fed WD_{AA} +Gly (FIG. 12B-D), without significant changes in food intake, activity or energy metabolism (FIG. 12E-H). Accordingly, plasma glycine-containing tripeptide molecule increased in mice fed WD_{AA} A-Gly (FIG. 12I) and increased adipocyte hypertrophy was noted in epididymal and subcutaneous adipose tissues (EAT and SAT, FIG. 12J). Compared to WD_{AA} +Gly, mice fed WD_{AA} - Gly had increased plasma TC and TG (FIG. 13A-D). Increased plasma glucose was noted in mice fed WD_{AA} -Gly, but not in mice fed WD_{AA} +Gly (FIG. 13E). Histology and lipid quantification revealed increased HS following WD_{AA} -Gly feeding (FIG. 13F-H). Linear regression analyses indicated significant inverse correlations between individual levels of plasma glycine and plasma TC, glucose or liver TG (FIG. 13I-K). Thus, dietary glycine deficiency exacerbates hyperlipidemia, hyperglycemia and HS in hyperlipidemic mice.

### (iv) DT-109: A Glycine-based Tripeptide with Dual Glucose/Lipid-lowering Properties

Considering the findings above, we reasoned that glycine-based compounds may have a therapeutic potential through a dual glucose/lipid-lowering effect. Thus, we searched for compounds with structural similarities to glycine, applying various chemical modifications at the amine or carboxyl groups. Ten potential compounds were identified (FIG. 9A-K), of which four were suitable for oral administration. Chronic glycine supplementation (1 mg/g/day) was shown to reduce circulating TG in apoE^{-/-} mice, restore glucose tolerance and accelerate fat loss in obese C57BL/6 mice. In humans, ingestion of glycine together with glucose attenuated the increase in plasma glucose by >50%. To test glucose-lowering effects, we performed oral glucose tolerance tests (OGTT) in C57BL/6J mice administered glycine or glycine-based compounds (0.5 mg/g). None of the tested compounds attenuated the increase in blood glucose more efficiently than glycine (FIG. 14A-D). Another AA reported to lower glucose in humans and mice is leucine. Previous studies from our lab revealed profound glucose-lowering effects for DT-109, a tripeptide of glycine combined with leucine (gly-gly-L-leu). We tested DT-109 or its D-isomer (gly-gly-D-leu, DT-110) using OGTT. Whereas the glucose-lowering effects of DT-110 were similar to glycine, DT-109 was the only compound that lowered glucose more efficiently than glycine (FIG. 14E,F), particularly when compared to equivalent levels of its individual AA (FIG. 14G).

To test lipid-lowering effects, we fed hyperlipidemic apoE^{-/-} mice WD with oral administration of DT-109 (1 mg/g/day), equivalent levels of leucine, glycine or H₂O (FIG. 15A). After 10 weeks, DT-109 had the most potent glucose-lowering effects both in OGTT and in non-fasting glucose (FIG. 15B,C). No significant differences in food intake or body weight were observed (FIG. 15D,E). Lipid profile analyses showed that mice treated with DT-109 had the lowest TC and LDL, without the reduction in HDL noted in glycine-treated mice (FIG. 15F-I), and significant differences in plasma TG (FIG. 15J). Glycine or DT-109 reduced glycine-containing tripeptide molecule and adipocyte hypertrophy in EAT and SAT (FIG. 15K,L). Histology and lipid quantification revealed reduced HS in livers from mice treated with glycine or DT-109, but not with leucine (FIG. 16A-C). qPCR analyses revealed upregulation of key genes regulating FAO in livers from mice treated with glycine or DT-109 (PPARα, PNPLA2, carnitine/acylcarnitine translocase, CACT, carnitine palmitoyltransferase 1a, CPT1a, and acyl-CoA dehydrogenase-long chain, ACADL) and downregulation of CCL2 without significant changes in TNFα (FIG. 16D). Thus, DT-109 has dual glucose/lipid-lowering properties and prevents WD-induced HS in hyperlipidemic mice.

### (v) DT-109 Improves Body Composition and Protects against Diet-induced NASH

To further explore the therapeutic potential of DT-109 against NASH, we devised an experimental approach to model advanced NAFLD (FIG. 17A). C57BL/6J mice fed NASH-diet for 12 weeks had increased plasma glucose, TC, AST and ALT compared to CD feeding (FIG. 17B). A subset of the mice was sacrificed, confirming increased liver weight following NASH-diet feeding (FIG. 17C). H&E and ORO histology revealed HS, hepatocyte ballooning and infiltration of inflammatory cells, while Sirius Red staining confirmed early fibrosis (FIG. 17D). After confirming NASH (FIG. 17D-F), the rest of the mice were randomized to receive DT-109 at 0.125 or 0.5 mg/g/day, equivalent levels of leucine, glycine or H₂O via oral gavage for 12 additional weeks under NASH-diet. Mice fed CD and administered H₂O served as control.

At week 18, OGTT and non-fasting glucose measurements confirmed a most potent glucose-lowering effect for 0.5 mg/g/day DT-109 (FIG. 17G,H). Body composition analysis revealed increased body weight in all mice fed NASH-diet (FIG. 17I), although mice treated with 0.5 mg/g/day DT-109 showed reduced body fat with preserved lean mass compared to the H₂O controls (FIG. 17J,K), and decreased adipocyte hypertrophy in EAT and SAT (FIG. 17L), without significant differences in food intake (FIG. 17M). The metabolic response to diet-induced obesity involves a shift towards higher fat versus lower carbohydrate utilization, reflecting a reduction in RER. CLAMS analysis revealed decreased RER in all groups fed NASH-diet, but no significant differences were noted in mice treated with 0.5 mg/g/day DT-109 (FIG. 17N-P). No significant differences in energy expenditure or activity were noted (FIG. 17Q,R).

At endpoint, the increase in plasma AST, ALT and alkaline phosphatase (ALP) observed in mice fed NASH-diet was attenuated by glycine or DT-109 treatment (FIG. 18A-C). Plasma TG were decreased in mice treated with glycine or DT-109 (FIG. 18D), and TC was decreased by 0.5 mg/g/day DT-109 (FIG 18E). Accordingly, NASH-diet-induced hepatomegaly and yellowish coloration were markedly attenuated by treatment with glycine or DT-109, but not with leucine (FIG. 18F,G, FIG. 18H), with NAS significantly decreased by 0.5 mg/g/day DT-109 (FIG. 18I, FIG. 18J). Linear regression analyses indicated highly significant positive correlations between individual levels of AST, ALT or ALP and NAS (FIG. 18K-M).

### (vi) DT-109 Reverses NASH-diet-induced Transcriptome Alterations: Key Role for FAO

To explore the mechanisms by which glycine-based treatment protects against diet-induced NASH, we performed RNA-sequencing of livers collected at endpoint. Principal component analysis (PCA) revealed that the gene expression profile from mice on NASH-diet treated with leucine clustered with H₂O controls, with intermediate patterns for glycine and 0.125 mg/g/day, while 0.5 mg/g/day DT-109 clustered closer to CD mice (FIG. 19A). Volcano plot analysis confirmed major DEG alterations in mice fed NASH-diet and treated with H₂O or leucine compared to CD (3606 or 3145 DEG, respectively) that markedly reduced by treatment with glycine or DT-109 at 0.125 or 0.5 mg/g/day (1300, 1093 or 642 DEG, respectively, FIG. 19B). Analysis of the top 50 DEGs, further underscores the similarity of DT-109 (0.5 mg/g/day) to the CD group (FIG. 19C). Pathway analysis comparing NASH-diet H₂O control to CD confirmed suppression of pathways regulating glycine biosynthesis and glyoxylate metabolism, with downregulation of AGXT1, SHMT1 and SARDH, along with energy metabolism and FAO pathways. In contrast, known NASH-related pro-inflammatory/fibrotic pathways were upregulated (FIG. 19D,E). Pathway analysis comparing NASH-diet H₂O control to 0.5 mg/g/day DT-109 showed a similar pattern as CD, indicating that DT-109 reversed NASH-diet-induced alterations in underlying pathways (FIG. 19F). Analysis of 50 genes implicated in major aspects of NASH pathogenesis (FIG. 19G), revealed that key genes regulating FAO (PPARα, PPARG-coactivator-1α (PPARGC1A/PGC1α), acyl-CoA oxidase-1 (ACOX1), CPT2, ACADS/M/L, HADHA/B, and ACOT3/4) were overrepresented in CD mice and suppressed in NASH mice treated with H₂O or leucine. This was reversed by treatment with glycine or DT-109, particularly 0.5 mg/g/day, as confirmed by qPCR and Western blot analyses (FIG. 19H,I, FIG. 19J). Accordingly, ORO and lipid quantification confirmed marked HS in livers from NASH mice treated with H₂O or leucine which was significantly attenuated by glycine or DT-109 (FIG. 19K-L, FIG. 19M). Particularly, diacylglycerols (DAG), which are known to promote liver injury and NASH, were significantly reduced by 0.5 mg/g/day DT-109 (FIG. 19N). Thus, glycine-based treatment corrects impaired FAO, reduces NASH-diet-induced HS and lipotoxic lipids.

### (vii) DT-109 Reduces NASH-diet-induced Hepatic Inflammation and Fibrosis

RNA-sequencing analysis revealed suppression of key inflammatory pathways/genes by glycine-based treatment (FIG. 19F,G), suggesting anti-inflammatory roles. Indeed, immunostaining for F4/80, a well-established marker for hepatic macrophages, was significantly increased in livers from mice treated with H₂O or leucine under NASH-diet, but attenuated by glycine or DT-109 (FIG. 20A,B). In plasma, monocyte chemoattractant protein-1 (MCP-1/CCL2) and resistin, known inflammatory markers in NASH patients, were lower in mice treated with glycine or DT-109 (FIG. 20C,D). Accordingly, RNA-sequencing revealed that genes encoding for pro-inflammatory signaling regulators (NFKB1/2, RELB, CCR1/2/5, TLR1/2/4, and TNFRSF1A/9/12) and cytokines (TNFα and CCL2/5) were upregulated in mice fed NASH-diet and treated with H₂O or leucine and attenuated by glycine or DT-109 (FIG. 19G). This was confirmed by qPCR analyses in which NFKB2, RELB and TNFα were significantly downregulated by either glycine or DT-109, while CCL2, CCR2 and CCR5 were downregulated only by 0.5 mg/g/day DT-109 (FIG. 20E).

RNA-sequencing also demonstrated that pathways/genes related to TGFβ signaling (TGFB1/2 and TGFBR1/2) and ECM remodeling (COL1A1/1A2/3A1/4A1/4A2, TIMP1/2 and SERPINE1) were upregulated by NASH-diet and attenuated by glycine or DT-109 (FIG. 19F,G). Indeed, histological analysis based on Sirius Red and fibrosis scoring revealed protective effects of glycine or DT-109, but not leucine, against NASH-diet-induced hepatic fibrosis (FIG. 20A,F,G). Linear regression analyses demonstrated highly significant positive correlations between individual levels of AST, ALT or ALP and fibrosis scores, indicating that glycine or DT-109 attenuate NASH-diet-induced liver damage (FIG. 20H,I,J). To test whether glycine-based treatment attenuates TGFβ-mediated hepatic fibrosis, we next analyzed SMAD signaling and found reduced SMAD2 Ser465/467 phosphorylation, mainly by DT-109 (FIG. 20K). qPCR analyses confirmed that TGFβ-related genes were markedly upregulated in livers from NASH mice administered H₂O or leucine which was attenuated by DT-109 (FIG. 20L). Thus, consistent with reduced HS and lipotoxicity,2 glycine-based treatment lowers NASH-diet-induced steatohepatitis and fibrosis.

### I. Discussion

Whereas abnormal lipid and glucose metabolism are known features of NAFLD, perturbations in AA metabolism was suggested to be implicated in NASH. Particularly, lower circulating glycine has been consistently reported in NAFLD patients, but the causes for reduced glycine and its therapeutic potential remain unclear. Herein, using genetic and dietary approaches to limit glycine availability, we provide evidence for a causative role for glycine in NAFLD development. In a search for potential glycine-based therapies for NAFLD, we identified DT-109 as having dual glucose/lipid-lowering effects and potently protected mice from diet-induced NASH.

The results presented herein, indicate that lower glycine levels found in NAFLD patients, are associated with suppression of hepatic glycine biosynthetic genes. Particularly, both in murine and human NASH, we found a significant suppression of AGXT1, which catalyzes the conversion of glyoxylate to glycine, and showed that AGXT1 expression inversely correlated with hepatic fat content in humans. While others reported that AGXT1 is suppressed in NASH patients or murine models, we report for the first time a causative role for AGXT1 in NAFLD. Mutations in AGXT1 are responsible for primary hyperoxaluria type 1 caused by impaired conversion of glyoxylate to glycine and excessive hepatic oxalate production leading to renal failure. Interestingly, proteomics of livers from AGXT1^{-/-}mice indicated significant alterations in glucose and lipid metabolic pathways, but AGXT1 role in NASH had not been evaluated before. Using CRISPR/Cas9, we generated AGXT1⁻¹⁻ mice that presented exacerbated hyperlipidemia and NASH already after 12 weeks on NASH-diet. We identified suppression of FAO pathways, which in turn promote steatohepatitis and fibrosis, in AGXT1^{-/-} mice.

We further applied dietary approaches to limit glycine availability, and compared lipid profile and HS in hyperlipidemic mice fed WD with or without glycine. The enhanced adiposity, hyperlipidemia and HS observed in mice fed glycine-deficient WD are in line with previous reports in which dietary glycine accelerated fat loss, improved glucose tolerance, reduced plasma lipids or HS in various rodent models. Interestingly, reduced HS and plasma liver enzymes were found in NAFLD patients following supplementation with the glycine precursor, serine, albeit with a small sample size and a short treatment period. In a thorough investigation using a model of advanced NAFLD featuring coexistence of steatohepatitis and fibrosis, we report for the first time protective effects of glycine treatment, with a relatively low dose in mice of 0.33 mg/g/day.

During our search for glycine-based compounds, none of the identified compounds reduced plasma glucose more efficiently than glycine. Therefore, we tested combinations of glycine with leucine, another AA reported to lower glucose in humans, and reduce HS in mice. Particularly, applying various T2D models, our lab uncovered potent glucose-lowering effects of the tripeptide gly-gly-L-leu that exceeded those of free glycine, leucine or their dipeptide combinations. For the first time, we show that DT-109 also improves lipid profile, HS and NASH using genetic and dietary models. While no significant effects were observed in mice treated with equivalent leucine levels, metabolic benefits were evident following glycine treatment. Nevertheless, some outcomes, including robust glucose-lowering, preservation of HDL levels in hyperlipidemic mice, prevention of NASH-diet-induced alternations in body composition, reduction of liver DAG and a significant decrease in NAS, were evident only with the higher doses of DT-109. It should be noted that profound benefits were also noted with a lower dose of 0.125 mg/g/day DT-109.

Lipid overload is central to NASH pathogenesis. When free fatty acids are supplied to the liver in excess and/or their disposal via FAO is impaired, they are used as substrates for lipotoxic species that induce oxidant stress and pro-inflammatory/fibrogenic pathways, promoting steatohepatitis and fibrosis. Applying unbiased transcriptomics, we identified key FAO pathways suppressed in livers from NASH mice which was reversed by DT-109, with subsequent reduction in HS and lipotoxic DAG. This indicates that glycine-based treatment normalizes impaired hepatic FAO and lowers HS and lipotoxicity, which in turn can attenuate NASH progression. Indeed, using our model, featuring steatohepatitis and fibrosis, we found that glycine or DT-109 attenuated NASH-diet-induced hepatic/systemic inflammation and fibrosis as evident by histological, transcriptomics and plasma analyses. In line, previous studies reported anti-inflammatory and hepatoprotective effects of glycine in mice with endotoxemia. In T2D patients, glycine treatment (5 g/day) for 3 months reduced hemoglobin-A1c and plasma TNFR1.

In summary, identification of impaired glycine metabolism in NAFLD led to the inventors to identify glycine-based treatment that proved effective in experimental NAFLD by modulating hepatic FAO.
The present disclosure will now be further defined in the following paragraphs:
1. A method for treating at least one of hyperlipidemia, fatty liver, steatohepatitis, non-alcoholic fatty liver disease, non-alcoholic steatohepatitis, obesity, hyperglycemia, metabolic syndrome, cardiovascular disease, and atherosclerosis in a mammalian subject comprising administering to a subject in need thereof, a therapeutical effecitve amount of glycine a glycine-containing tripeptide molecule, or a pharmaceutically acceptable salt thereof.
2. The method of paragraph 1, wherein the glycine-containing tripeptide molecule significantly decreases the hepatic triglyceride levels.
3. The method of paragraph 1, wherein the glycine-containing tripeptide molecule significantly decreases the hepatic total cholesterol level.
4. The method of any one of paragraphs 1-3, wherein the glycine-containing tripeptide molecule is Gly-Gly-Leu, Gly-Gly-dLeu, or a pharmaceutically acceptable salt thereof.
5. A method to enhance hepatic lipid oxidation or utilization, to lower the triglyceride level in the blood of a subject, or lower the cholesterol level in the blood of a hypercholesterolemic subject in need thereof, comprising administering to a subject, a glycine-containing tripeptide molecule, or a pharmaceutically acceptable salt thereof, wherein hepatic lipid oxidation is increased, and triglyceride level, hypercholesterolemia or any combination thereof, is ameliorated as a result of treatment.
6. The method of paragraphs 5, wherein the glycine-containing tripeptide molecule is Gly-Gly-Leu, Gly-Gly-dLeu, or a pharmaceutically acceptable salt thereof.
7. The method of paragraph 5 or paragraph 6, wherein the glycine-containing tripeptide molecule Gly-Gly-Leu, Gly-Gly-dLeu, or a pharmaceutically acceptable salt thereof, significantly induces the expression of regulators of hepatic lipid oxidation, AMPKα1 or PPARα.
8. The method of paragraph 5 or paragraph 6, wherein the glycine-containing tripeptide molecule Gly-Gly-Leu, Gly-Gly-dLeu, or a pharmaceutically acceptable salt thereof, reduces hepatic triglyceride levels by significantly upregulating CPT1a, CACT, or ACADI (mitochondrial β-oxidation) or PNPLA2.
9. The method of paragraph 5 or paragraph 6, wherein the glycine-containing tripeptide molecule Gly-Gly-Leu, Gly-Gly-dLeu, or a pharmaceutically acceptable salt thereof, reduces hepatic triglyceride levels by significantly upregulating the mitochondrial anion carrier UCP2.
10. The method of paragraph 5 or paragraph 6, wherein the glycine-containing tripeptide molecule Gly-Gly-Leu, Gly-Gly-dLeu, or a pharmaceutically acceptable salt thereof, regulates cholesterol homeostasis in the liver by significantly increasing the expression of ABCG5 and ABCG8.
11. A method of treating the subject's plasma lipid profile, comprising administering to a subject in need thereof, a glycine-containing tripeptide molecule, or a pharmaceutically acceptable salt thereof, to lower the subject's plasma triglyceride, plasma LDL level, to prevent further progression of the atherosclerotic lesions, or to regress existing atherosclerotic lesions in the arteries of the subject, to reduce the occurrence of MACE, prevention, delaying or reducing the severity of a primary cardiovascular event.
12. The method of paragraph 11, wherein the glycine-containing tripeptide molecule is Gly-Gly-Leu, Gly-Gly-dLeu, or a pharmaceutically acceptable salt thereof.
13. The method of paragraph 11 or 12, wherein the Gly-Gly-Leu, Gly-Gly-dLeu, or a pharmaceutically acceptable salt thereof, reduces atherosclerotic lesions.
14. The method of paragraph 11 or 12, wherein the Gly-Gly-Leu, Gly-Gly-dLeu, or a pharmaceutically acceptable salt thereof, lowers plasma total cholesterol, plasma LDL cholesterol, nonHDL-cholesterol, VLDL-cholesterol or a combination thereof.
15. A method of treating inflammation in adipose tissues and reducing the level of an inflammation marker in the circulation, comprising administering to a subject in need thereof, a glycine-containing tripeptide molecule, or a pharmaceutically acceptable salt thereof, wherein the administration of the glycine-containing tripeptide molecule or a pharmaceutically acceptable salt thereof results in reduced inflammation in the adipose tissues and an inflammation marker in the circulation of the subject.
16. The method of paragraph 15, wherein the inflammation marker in the circulation is reduced by lowering the level of plasma MCP1 by administration of Gly-Gly-Leu, Gly-Gly-dLeu, or a pharmaceutically acceptable salt thereof to the subject.
17. The method of paragraph 15, wherein the inflammation in the adipose tissue is in the epididymal adipose tissue (EAT) or the subcutaneous adipose tissue (SAT) and the level of MCP1 mRNA is decreased.
18. A method of treating a subject for lowering plasma levels of leptin, comprising administering to a subject in need thereof, a glycine-containing tripeptide molecule, or a pharmaceutically acceptable salt thereof, wherein the administration of the glycine-containing tripeptide molecule reduces the level of plasma leptin.
19. The method of paragraph 18, wherein the treatment is with Gly-Gly-Leu, Gly-Gly-dLeu, or a pharmaceutically acceptable salt thereof.
20. A method of treating a subject, comprising administering to a subject in need thereof, Gly-Gly-Leu, Gly-Gly-dLeu, or a pharmaceutically acceptable salt thereof, wherein the subject has a liver disease.
21. The method of paragraph 20, wherein the liver disease is nonalcoholic fatty liver disease (NAFLD) or nonalcoholic steatohepatitis (NASH), or alcoholic hepatic steatosis.
22. A method of stabilization or reduction of the NAFLD activity score (NAS) in a subject, comprising, administering to the subject Gly-Gly-Leu, Gly-Gly-dLeu, or a pharmaceutically acceptable salt thereof.
23. The method of paragraph 22, wherein the method comprises slowing the progression of, stabilizing, or reducing the steatosis component of NAS.
24. The method of any one of paragraphs 22-23, wherein the method comprises slowing the progression of, stabilizing, or reducing the lobular inflammation component of NAS.
25. The method of any one of paragraphs 22-24, wherein the method comprises slowing the progression of, stabilizing, or reducing the hepatocyte ballooning component of NAS.
26. The method according to any one of paragraphs 22-25, wherein NAS is different by no less than 1.5 points after 6 months of treatment with Gly-Gly-Leu, Gly-Gly-dLeu, or a pharmaceutically acceptable salt thereof.
27. A method of reducing hepatic fibrosis in a subject in need thereof, comprising administering to the subject Gly-Gly-Leu, Gly-Gly-dLeu, or a pharmaceutically acceptable salt thereof.
28. A method of treating atherosclerosis, the method comprising administering to a subject in need thereof, a therapeutically effective amount of a glycine-containing tripeptide molecule or a pharmaceutically acceptable salt thereof.
29. The method of paragraph 28, wherein the glycine-containing tripeptide molecule molecule is Gly-Gly-Leu, Gly-Gly-dLeu, or a pharmaceutically acceptable salt thereof.
30. A method of treating a complication of atherosclerosis by administering to the subject with the complication, a glycine-containing tripeptide molecule to treat the complication selected from the group consisting of myocardial infarction, arteriosclerosis, coronary artery disease, carotid artery disease, peripheral artery disease, atherothrombotic stroke, aneurisms, or chronic kidney disease.
31. The method of paragraph 30, wherein the glycine-containing tripeptide molecule is Gly-Gly-Leu, Gly-Gly-dLeu, or a pharmaceutically acceptable salt thereof.
32. The method of any one of paragraphs 1 to 31, wherein the method further comprises administering a second therapeutic agent to the subject in need thereof comprising a cholesterol absorption inhibitor, a PCSK9 inhibitor, a PPAR-alpha agonist, an ACE inhibitor, a calcium channel blocker, an ARBs, a diuretic, renin, GLP-1 or a synthetic variant thereof, insulin, or a synthetic variant thereof, metformin, a sulfonyll urea compound, a thiazolidinedione (TZD), a SGLT2 inhibitor, a DPP-IV inhibitor, an inhibitor of HMGCoA reductase, an inhibitor of proprotein convertase subtilisin/kexin type 9 (PCSK9), ezetimibe, gemfibrozil, fenofibrate, clofibrate, bezafibrate, pemafibrate, gemcabene (CI-1027), benpodoic acid (ETC-1002), an ACC inhibitor, an ApoC-III inhibitor, an ACL-inhibitor, prescription fish oil, a CETP inhibitor an anti-fibrotic agent, and combinations thereof.
33. A kit for treating a subject with NAFLD or with NASH comprising a selected tripeptide, optionally a statin, and instructions for use.
34. The kit according to paragraph 30, the kit comprises DT-109 (Gly-Gly-Leu) and/or (DT-110 (Gly-Gly-dLeu), optionally a statin, and instructions for use.
35. The kit according to paragraph 32 or paragraph 33, wherein the kit further optionally comprises a cholesterol absorption inhibitor, a PCSK9 inhibitor, a PPAR-alpha agonist, an ACE inhibitor, a calcium channel blocker, an ARBs, a diuretic, renin, GLP-1 or a synthetic variant thereof, insulin, or a synthetic variant thereof, metformin, a sulfonyll urea compound, a thiazolidinedione (TZD), a SGLT2 inhibitor, a DPP-IV inhibitor, an inhibitor of HMGCoA reductase, an inhibitor of proprotein convertase subtilisin/kexin type 9 (PCSK9), ezetimibe, gemfibrozil, fenofibrate, clofibrate, bezafibrate, pemafibrate, gemcabene (CI-1027), benpodoic acid (ETC-1002), an ACC inhibitor, an ApoC-III inhibitor, an ACL-inhibitor, prescription fish oil, a CETP inhibitor an anti-fibrotic agent, and combinations thereof.
36. The kit according to paragraph 32 or paragraph 33, wherein the kit optionally comprises ezetimibe.

## Claims

1. A glycine-containing tripeptide molecule Gly-Gly-Leu or Gly-Gly-dLeu, or a pharmaceutically acceptable salt thereof for use in treating atherosclerosis in a subject,
wherein the use reduces atherosclerotic plaque burden in the aorta,
wherein the use reduces plasma LDL cholesterol levels,
wherein the use reduces hepatic triglyceride content and hepatic total cholesterol levels in the subject.

2. The glycine-containing tripeptide molecule for use of claim 1, wherein the subject has a complication of atherosclerosis, wherein the complication is myocardial infarction, arteriosclerosis, coronary artery disease, carotid artery disease, peripheral artery disease, atherothrombotic stroke, aneurysms or chronic kidney disease.

3. The glycine-containing tripeptide molecule for use of claim 1, wherein the use further comprises administering a second therapeutic agent to the subject in need thereof, wherein the second therapeutic agent comprises a cholesterol absorption inhibitor, a PPAR-alpha agonist, a PPAR gamma agonist, a PPAR delta agonist, an ACE inhibitor, a calcium channel blocker, an ARBs, a diuretic, renin, GLP-1 or a synthetic variant thereof, insulin, or a synthetic variant thereof, metformin, a sulfonyll urea compound, a thiazolidinedione (TZD), a SGLT2 inhibitor, a DPP-IV inhibitor, an inhibitor of HMGCoA reductase, an inhibitor of proprotein convertase subtilisin/kexin type 9 (PCSK9), ezetimibe, gemfibrozil, fenofibrate, clofibrate, bezafibrate, pemafibrate, gemcabene (CI-1027), bempedoic acid (ETC-1002), an ACC inhibitor, an ApoC-III inhibitor, an ACL-inhibitor, prescription fish oil, a CETP inhibitor, or an anti-fibrotic agent, and combinations thereof.

4. The glycine-containing tripeptide molecule for use of claim 1, wherein the use reduces plasma MCP-1.
